(19) Europäisches Patentamt
European Patent Office
Office européen des brevets

(11) EP 4 039 250 A1

(12) **EUROPEAN PATENT APPLICATION**

(43) Date of publication:
**10.08.2022 Bulletin 2022/32**

(21) Application number: **21203289.0**

(22) Date of filing: **27.12.2017**

(51) International Patent Classification (IPC):
***A61K 9/107*** *(2006.01)* ***A61K 47/10*** *(2017.01)*
***A61K 47/14*** *(2017.01)* ***A61K 47/44*** *(2017.01)*

(52) Cooperative Patent Classification (CPC):
**A61K 9/107; A61K 47/10; A61K 47/14; A61K 47/44**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2016 JP 2016255469**
**09.06.2017 JP 2017114074**

(62) Document number(s) of the earlier application(s) in accordance with Art. 76 EPC:
**17886597.8 / 3 563 833**

(71) Applicant: **CHUGAI SEIYAKU KABUSHIKI KAISHA**
**Tokyo 115-8543 (JP)**

(72) Inventors:
- **TAMPO, Yoshihiro**
  **Gotemba-shi, 412-8513 (JP)**
- **TAKANO, Ryusuke**
  **Kita-Ku, 115-8543 (JP)**
- **HISADA, Nozomi**
  **Chuo-ku, 103-8324 (JP)**
- **SAKAI, Kenichi**
  **Kita-Ku, 115-8543 (JP)**
- **OZEKI, Kazuhisa**
  **Gotemba-shi, 412-8513 (JP)**
- **SAKURAI, Yuji**
  **Gotemba-shi, 412-8513 (JP)**
- **HIGASHIDA, Atsuko**
  **Gotemba-shi, 412-8513 (JP)**

(74) Representative: **Vossius & Partner**
**Patentanwälte Rechtsanwälte mbB**
**Siebertstraße 3**
**81675 München (DE)**

Remarks:
This application was filed on 18-10-2021 as a divisional application to the application mentioned under INID code 62.

(54) **SELF-EMULSIFYING DRUG FORMULATION FOR IMPROVING MEMBRANE PERMEABILITY OF COMPOUND**

(57) An objective of the present invention is to provide lymphatically-transported self-emulsifying formulations for enhancing membrane permeability of poorly membrane-permeable compounds in oral administration of the compounds. It was discovered that by applying lymphatically-transported self-emulsifying formulations containing a surfactant containing oleic acid as a moiety or oily component to poorly membrane-permeable compounds containing a cyclic peptide and having features (i) and (ii) below, the membrane permeability and absorbability of the compounds can be improved: (i) logD (pH 7.4) value of the compounds is 3.2 or greater; and (ii) Caco-2 Papp (cm/sec) value of the compounds is 1.8E-6 or less.

EP 4 039 250 A1

## Description

### Technical Field

The present invention relates to lymphatically-transported self-emulsifying formulations for improving membrane permeability of poorly membrane-permeable compounds (compounds with low membrane permeability).

### Background Art

Generally, compounds with a molecular weight of 500 or higher have low membrane permeability when administered orally and are considered to have problems with oral absorbability (NPLs 1 and 2). In recent years, development of drug discovery techniques using middle molecular-weight compounds (for example, a molecular weight of 500 to 2000) is receiving attention, which techniques enable development of drugs towards tough targets represented by inhibition of protein-protein interaction, agonists, and molecular chaperones (NPL 3). Furthermore, there are reports on cases where even compounds that do not fall within the Lipinski's rule of 5 (most with a molecular weight of over 500), in particular natural products, can be used for oral agents or can inhibit intracellular targets (NPL 4). Middle molecular-weight compounds are highly valuable molecular species in that they have possibilities of achieving things which are not achievable with small molecules for the compounds' ability to access tough targets and which are not achievable with antibodies for the compounds' ability to transfer into cells (allowing development of intracellularly-targeted drugs, and drugs for oral administration).

Peptide pharmaceuticals are highly valuable chemical species, and 40 or more types have already been on the market (NPL 5). Peptides are middle molecular-weight compounds and have been generally regarded as having poor membrane permeability and low metabolic stability; and, cyclosporine A is one of the few representative examples of peptides that can be administered orally. Cyclosporine A is an 11-residue peptide produced by microorganisms which can be administered orally and inhibits an intracellular target (cyclophilin). The distinguishing feature of cyclosporine A as a peptide includes that it comprises a non-natural amino acid called "*N*-methyl amino acid" as its structural component. Stemming from this, in recent years, there have been a number of reports on studies that introduce *N*-methyl amino acids into peptides to increase the drug-likeness of the peptides, and further apply them to drug discovery (NPLs 6, 7, and 8). In particular, it is now known that introduction of *N*-methyl amino acid leads to the decrease in hydrogen-bond donor hydrogens, acquisition of protease resistance, and fixing of conformation, thereby contributing to membrane permeability and metabolic stability (NPLs 6 and 9, and PTL 1).

As a formulation used in pharmaceuticals, a self-emulsifying formulation (Self-Emulsifying Drug Delivery System; hereinafter referred to as "SEDDS") composed of oil, surfactants, and such, is known. Conventionally, self-emulsifying formulations have been used mainly to improve the solubility of water insoluble compounds (PTL 2). Water insoluble compounds show decreased absorbability and varied absorbability due to individual differences when administered orally. Therefore, in developing oral agents of water insoluble compounds, improving absorbability and reducing variation in absorbability become important problems. SEDDS has been known as one of the formulation methods for solving such problems with absorbability. SEDDS is a formulation prepared by mixing the above-mentioned constituents to homogeneity under a water-free condition, and then dissolving or dispersing. After administration, SEDDS is dispersed and dissolved in water within the digestive tract to form emulsions, which improves the solubility of the water insoluble drugs, and improves variation in absorbability due to individual differences.

Other than improving the solubility of water insoluble drugs, the purposes of using SEDDS include examples where it is applied to formulations targeting compounds having poor metabolic stability. Generally, when a compound is orally administered, the major part of it is transported into blood; therefore, only a small fraction of the compound is transported into the lymph vessels. Drugs that are transported to the lymph vessels do not pass through the portal vein. Therefore, drugs that are transported to the lymph vessels can avoid the first-pass effect by the liver. Examples of application of SEDDS have been reported, where the objective is to avoid the first-pass effect by increasing lymphatic absorbability of compounds with poor metabolic stability using SEDDS (NPLs 10 and 11, and PTLs 3 and 4).

There is a report suggesting that lymphatic absorbability of halofantrine (molecular weight of approximately 500), which is a lipid-soluble drug, correlates with the carbon chain length of the fatty acid used as the administration base material (NPL 12), and it was shown that when the carbon chains of simultaneously administered fatty acids are short (for example, short chain fatty acids or medium chain fatty acids), the amount of lymphatic transport of halofantrine decreases. Therefore, use of medium chain fatty acids and short chain fatty acids in lymphatically-transported self-emulsifying formulations has been considered difficult.

[Citation List]

[Patent Literature]

[PTL 1] WO 2013/100132
[PTL 2] WO 2006/112541
[PTL 3] WO 2002/102354
[PTL 4] WO 2012/033478

[Non Paten Literature]

[NPL 1] Donovan, M.D. et al., (1990) Absorption of polyethylene glycols 600 through 2000: The molecular weight dependence of gastrointestinal and nasal absorption. Pharm. Res. 7, 863-868
[NPL 2] CA Lipinski, Adv. Drug Del. Rev. 1997, 23, 3 (Lipinski, rule of 5)
[NPL 3] Satyanarayanajois, S. D., Hill, R. A. Medicinal chemistry for 2020, Future Med. Chem. 2011,3, 1765
[NPL 4] Ganesan, A., The impact of natural products upon modern drug discovery. Curr. Opin. Chem. Bio. 2008, 12, 306.
[NPL 5] 4: Gracia, S. R., Gaus, K., Sewald, N. Synthesis of chemically modified bioactive peptides: recent advances, challenges and developments for medicinal chemistry. Future Med. Chem. 2009, 1, 1289
[NPL 6] R. S. Lokey et al., Nat. Chem. Biol. 2011, 7(11), 810-817.
[NPL 7] J. W. Szostak et al., J. Am. Chem. Soc. 2008, 130, 6131-6136.
[NPL 8] T. Kawakami et al., Chemistry & Biology, 2008, Vol. 15, 32-42.
[NPL 9] H. Kessler et al., J. Am. Chem. Soc. 2012, 134, 12125-12133
[NPL 10] Drug Metab Dispos. 2006 May; 34(5): 729-33.
[NPL 11] Pharm Res. 2009 Jun; 26(6): 1486-95.
[NPL 12] Journal of Pharmaceutical Sciences, Vol.89, 1073-1084 (2000)

## Summary of the Invention

[Problems to be Solved by the Invention]

The present invention was achieved in view of the above circumstances. An objective of the present invention is to provide lymphatically-transported self-emulsifying formulations for enhancing membrane permeability of poorly membrane-permeable compounds in oral administration.

[Means for Solving the Problems]

To solve the above-mentioned problems, the present inventors undertook investigation for improving the membrane permeability and absorbability of various poorly membrane-permeable middle molecular-weight compounds. As a result, the present inventors discovered that by applying lymphatically transported self-emulsifying formulations which comprise a surfactant comprising oleate ester or oleylether as a moiety and an oily component to poorly membrane-permeable compounds containing a cyclic peptide having features (i) and (ii) below, the membrane permeability and absorbability of the compounds can be improved:

(i) logD (pH 7.4) value of the drug is 3.2 or greater; and
(ii) Caco-2 Papp (cm/sec) value of the drug is 1.8E-6 or less.

The present invention is based on such findings, and specifically provides [1] to [16] below:

[1] a self-emulsifying formulation, which comprises a surfactant comprising oleate ester or oleylether as a moiety, an oily component, and a poorly membrane-permeable drug;
[2] the self-emulsifying formulation of [1], which is for enhancing membrane permeability of the poorly membrane-permeable drug;
[3] the self-emulsifying formulation of [1] or [2], which is lymphatically transported;

[4] the self-emulsifying formulation of any one of [1] to [3], wherein the drug has features (i) and (ii) below:

(i) logD (pH 7.4) value of the drug is 3.2 or greater; and
(ii) Caco-2 Papp (cm/sec) value of the drug is 1.8E-6 or less;

[5] the self-emulsifying formulation of any one of [1] to [4], wherein the drug is a peptide compound comprising a cyclic portion, wherein the compound has features (i) and/or (ii) below:

(i) the compound comprises the cyclic portion whose total number of natural amino acid and amino acid analog residues is 5 to 12, and the compound's total number of natural amino acid and amino acid analog residues is 9 to 13;
(ii) the compound comprises at least two *N*-substituted amino acids and comprises at least one amino acid that is not *N*-substituted;

[6] the self-emulsifying formulation of any one of [1] to [5], which further comprises one or more types of hydrophilic surfactants;
[7] the self-emulsifying formulation of any one of [1] to [6], which further comprises oleic acid;
[8] the self-emulsifying formulation of any one of [1] to [7], wherein the surfactant comprising oleate ester or oleylether as a moiety is one or more types of surfactants selected from the group consisting of glyceryl monooleate, decaglyceryl monooleate, polyglyceryl-3 oleate, polyglyceryl-3 dioleate, polyethylene glycol (10) monooleate, polyethylene glycol (15) monooleate, polyethylene glycol (20) monooleate, polyethylene glycol (30) monooleate, polyethylene glycol (35) monooleate, apricot kernel oil polyoxyethylene-6 ester, sorbitan monooleate, sorbitan trioleate, polyethylene glycol (10) oleylether, polyethylene glycol (15) oleylether, polyethylene glycol (20) oleylether, and polyethylene glycol (50) oleylether;
[9] the self-emulsifying formulation of any one of [1] to [8], wherein the oily component is one or more types of oily components selected from the group consisting of olive oil, almond oil, coconut oil, cacao butter, macadamia nut oil, avocado oil, safflower oil, soybean oil, linseed oil, rapeseed oil, castor oil, palm oil, high-oleic sunflower oil, high-oleic safflower oil, sunflower oil, cotton seed oil, corn oil, sesame oil, peanut oil, apricot kernel oil, candlenut oil, grapeseed oil, pistachio seed oil, sunflower oil, hazelnut oil, jojoba oil, meadowfoam oil, rosehip oil, Tricaproin, Tricaprylin, Tricaprin, Tripalmitolein, Triolein, Trilinolein, Trilinolenin, Trieicosenoin, and Trierucin;
[10] the self-emulsifying formulation of any one of [1] to [8], wherein the oily component is an oily component comprising oleic acid as the main oil type component;
[11] the self-emulsifying formulation of [10], wherein the oily component comprising oleic acid as the main oil type component is one or more types of oily component selected from the group consisting of camellia oil, sunflower oil, avocado oil, avocado oil, safflower oil, almond oil, olive oil, rapeseed oil, and cashew oil;
[12] the self-emulsifying formulation of any one of [6] to [11], wherein the hydrophilic surfactant is polyoxyethylene hydroxystearate, polyoxyethylene hydroxyoleate, polyoxyl 40 hardened castor oil, or polyoxyl 35 castor oil;
[13] the self-emulsifying formulation of any one of [1] to [12], wherein the oily component is 8 vol% to 40 vol% based on the whole formulation (excluding the volume of the drug);
[14] a self-emulsifying formulation, which comprises glyceryl monooleate at 9 vol% to 19 vol%, polyoxyethylene hydroxystearate at 51 vol% to 61 vol%, and olive oil at 17.5 vol% to 27.5 vol% based on the whole formulation (excluding the volume of a drug), and a poorly membrane-permeable drug;
[15] a self-emulsifying formulation, which comprises a surfactant comprising oleate ester or oleylether as a moiety, an oily component, and a poorly membrane-permeable drug having features (i) to (iii) below:

(i) logD (pH 7.4) value of the drug is 3.2 or greater;
(ii) Caco-2 Papp (cm/sec) value of the drug is 1.8E-6 or less; and
(iii) molecular weight of the drug is 500 or greater; and

[16] the self-emulsifying formulation of [15], wherein the drug is a peptide compound comprising a cyclic portion, wherein the compound has features (i) and/or (ii) below:

(i) the compound comprises the cyclic portion whose total number of natural amino acid and amino acid analog residues is 5 to 12, and the compound's total number of natural amino acid and amino acid analog residues is 9 to 13; and
(ii) the compound comprises at least two *N*-substituted amino acids, and comprises at least one amino acid that is not *N*-substituted.

Furthermore, the following inventions are provided:

[2-1] a lymphatically transported self-emulsifying formulation, which comprises a surfactant comprising oleate ester or oleylether as a moiety, an oily component, and a poorly membrane-permeable drug, wherein the formulation increases membrane permeability of the drug;
[2-2] a lymphatically transported self-emulsifying formulation, which comprises a surfactant comprising oleate ester or oleylether as a moiety, an oily component, and a poorly membrane-permeable drug, wherein the formulation enhances membrane permeability of the drug;
[2-3] a lymphatically transported self-emulsifying formulation, which comprises a surfactant, an oily component, and a poorly membrane-permeable drug, wherein the oleate content is 25 vol% to 75 vol% based on the whole formulation (excluding the volume of the drug);
[2-4] a lymphatically transported self-emulsifying formulation, which comprises a surfactant comprising oleate ester or oleylether as a moiety, an oily component, and a poorly membrane-permeable drug, wherein the oleic acid content is 25 vol% to 75 vol% based on the whole formulation (excluding the volume of the drug);
[2-5] a lymphatically transported self-emulsifying formulation, which comprises a surfactant, an oily component which comprises oleic acid as a main oil type component, and a poorly membrane-permeable drug, wherein the oleic acid content is 25 vol% to 75 vol% based on the whole formulation (excluding the volume of the drug);
[2-6] a self-emulsifying formulation, which comprises a surfactant comprising oleate ester or oleylether as a moiety, an oily component, and a poorly membrane-permeable drug;
[2-7] a self-emulsifying formulation, which comprises a surfactant, an oily component which comprises oleic acid as the main oil type component, and a poorly membrane-permeable drug;
[2-8] the self-emulsifying formulation of any one of [2-3] to [2-7], which is for enhancing membrane permeability of the poorly membrane-permeable drug;
[2-9] a self-emulsifying formulation for providing escape for a poorly membrane-permeable drug from exocytosis by a transporter expressed in a small intestinal epithelial cell, wherein the formulation comprises a surfactant comprising oleate ester or oleylether as a moiety, an oily component, and the poorly membrane-permeable drug;
[2-10] a self-emulsifying formulation for providing escape for a poorly membrane-permeable drug from exocytosis by a transporter expressed in a small intestinal epithelial cell, wherein the formulation comprises a surfactant, an oily component which comprises oleic acid as the main oil type component, and the poorly membrane-permeable drug;
[2-11] a lymphatically transported self-emulsifying formulation for enhancing simple diffusion (passive diffusion) of a poorly membrane-permeable drug in a small intestinal epithelial cell membrane, wherein the formulation comprises a surfactant comprising oleate ester or oleylether as a moiety, an oily component, and the poorly membrane-permeable drug;
[2-12] a lymphatically transported self-emulsifying formulation for enhancing simple diffusion (passive diffusion) of a poorly membrane-permeable drug in a small intestinal epithelial cell membrane, wherein the formulation comprises a surfactant, an oily component which comprises oleic acid as the main oil type component, and the poorly membrane-permeable drug;
[2-13] a lymphatically transported self-emulsifying formulation for avoiding a first-pass effect on a poorly membrane-permeable drug, wherein the formulation comprises a surfactant comprising oleate ester or oleylether as a moiety, an oily component, and the poorly membrane-permeable drug;
[2-14] a lymphatically transported self-emulsifying formulation for avoiding a first-pass effect on a poorly membrane-permeable drug, wherein the formulation comprises a surfactant, an oily component which comprises oleic acid as the main oil type component, and the poorly membrane-permeable drug;
[2-15] a lymphatically transported self-emulsifying formulation, which comprises a surfactant comprising oleate ester or oleylether as a moiety, an oily component, and a poorly membrane-permeable drug, wherein when time taken to reach maximum blood concentration (Tmax) is assayed for the drug in the plasma of a mammalian subject after the administration, the Tmax is lower than the Tmax for a non-self-emulsifying formulation for the same drug administered at the same dose;
[2-16] a lymphatically transported self-emulsifying formulation, which comprises a surfactant, an oily component which comprises oleic acid as the main oil type component, and a poorly membrane-permeable drug, wherein when time taken to reach maximum blood concentration (Tmax) of the drug is assayed in the plasma of a mammalian subject after administration, the Tmax is lower than the Tmax for a non-self-emulsifying formulation of the same drug administered at the same dose;
[2-17] a lymphatically transported self-emulsifying formulation, which comprises a surfactant comprising oleate ester or oleylether as a moiety, an oily component, and a poorly membrane-permeable drug, wherein when time taken to reach maximum blood concentration (Tmax) of the drug is assayed in the plasma of a mammalian subject after administration, the Tmax is 90% or less of the Tmax for a non-self-emulsifying formulation of the same drug administered at the same dose;

[2-18] a lymphatically transported self-emulsifying formulation, which comprises a surfactant, an oily component which comprises oleic acid as the main oil type component, and a poorly membrane-permeable drug, wherein when time taken to reach maximum blood concentration (Tmax) of the drug is assayed in the plasma of a mammalian subject after administration, the Tmax is 90% or less of the Tmax for a non-self-emulsifying formulation of the same drug administered at the same dose;

[2-19] a lymphatically transported self-emulsifying formulation, which comprises a surfactant comprising oleate ester or oleylether as a moiety, an oily component, and a poorly membrane-permeable drug, wherein when amount of lymphatic transport of the drug is assayed in the plasma of a mammalian subject after administration, the amount of lymphatic transport is higher than the amount of lymphatic transport for a non-self-emulsifying formulation of the same drug administered at the same dose;

[2-20] a lymphatically transported self-emulsifying formulation, which comprises a surfactant, an oily component which comprises oleic acid as the main oil type component, and a poorly membrane-permeable drug, wherein when amount of lymphatic transport of the drug is assayed in the plasma of a mammalian subject after administration, the amount of lymphatic transport is higher than the amount of lymphatic transport for a non-self-emulsifying formulation of the same drug administered at the same dose;

[2-21] a lymphatically transported self-emulsifying formulation, which comprises a surfactant comprising oleate ester or oleylether as a moiety, an oily component, and a poorly membrane-permeable drug, wherein when bioavailability of the drug is assayed in the plasma of a mammalian subject after administration, the bioavailability is higher than the bioavailability for a non-self-emulsifying formulation of the same drug administered at the same dose;

[2-22] a lymphatically transported self-emulsifying formulation, which comprises a surfactant, an oily component which comprises oleic acid as the main oil type component, and a poorly membrane-permeable drug, wherein when bioavailability of the drug is assayed in the plasma of a mammalian subject after administration, the bioavailability is higher than the bioavailability for a non-self-emulsifying formulation of the same drug administered at the same dose;

[2-23] a lymphatically transported self-emulsifying formulation, which comprises a surfactant comprising oleate ester or oleylether as a moiety, an oily component, and a poorly membrane-permeable drug, wherein when membrane permeability of the drug is assayed *in vitro* using cultured cells, the membrane permeability is higher than the membrane permeability for a non-self-emulsifying formulation of the same drug;

[2-24] a lymphatically transported self-emulsifying formulation, which comprises a surfactant, an oily component which comprises oleic acid as the main oil type component, and a poorly membrane-permeable drug, wherein when membrane permeability of the drug is assayed *in vitro* using cultured cells, the membrane permeability is higher than the membrane permeability for a non-self-emulsifying formulation of the same drug;

[2-25] the self-emulsifying formulation of any one of [2-1] to [2-24], wherein the drug has features (i) and (ii) below:

(i) logD (pH 7.4) value of the drug is 3.2 or greater; and
(ii) Caco-2 Papp (cm/sec) value of the drug is 1.8E-6 or less;

[2-26] the self-emulsifying formulation of any one of [2-1] to [2-25], wherein the drug is a peptide compound comprising a cyclic portion, wherein the compound has features (i) and/or (ii) below:

(i) the compound comprises the cyclic portion whose total number of natural amino acid and amino acid analog residues is 5 to 12, and the compound's total number of natural amino acid and amino acid analog residues is 9 to 13;
(ii) the compound comprises at least two *N*-substituted amino acids and comprises at least one amino acid that is not *N*-substituted;

[2-27] the self-emulsifying formulation of any one of [2-1] to [2-26], which further comprises one or more types of hydrophilic surfactants;

[2-28] the self-emulsifying formulation of any one of [2-1] to [2-27], which further comprises oleic acid;

[2-29] the self-emulsifying formulation of any one of [2-1] to [2-28], wherein the surfactant comprising oleate ester or oleylether as a moiety is one or more types of surfactants selected from the group consisting of glyceryl monooleate, decaglyceryl monooleate, polyglyceryl-3 oleate, polyglyceryl-3 dioleate, polyethylene glycol (10) monooleate, polyethylene glycol (15) monooleate, polyethylene glycol (20) monooleate, polyethylene glycol (30) monooleate, polyethylene glycol (35) monooleate, apricot kernel oil polyoxyethylene-6 ester, sorbitan monooleate, sorbitan trioleate, polyethylene glycol (10) oleylether, polyethylene glycol (15) oleylether, polyethylene glycol (20) oleylether, and polyethylene glycol (50) oleylether;

[2-30] the self-emulsifying formulation of any one of [2-1] to [2-29], wherein the oily component is one or more types

of oily components selected from the group consisting of olive oil, almond oil, coconut oil, cacao butter, macadamia nut oil, avocado oil, safflower oil, soybean oil, linseed oil, rapeseed oil, castor oil, palm oil, high-oleic sunflower oil, high-oleic safflower oil, sunflower oil, cotton seed oil, corn oil, sesame oil, peanut oil, apricot kernel oil, candlenut oil, grapeseed oil, pistachio seed oil, sunflower oil, hazelnut oil, jojoba oil, meadowfoam oil, rosehip oil, Tricaproin, Tricaprylin, Tricaprin, Tripalmitolein, Triolein, Trilinolein, Trilinolenin, Trieicosenoin, and Trierucin;
[2-31] the self-emulsifying formulation of any one of [2-1] to [2-30], wherein the oily component is an oily component comprising oleic acid as the main oil type component;
[2-32] the self-emulsifying formulation of [2-31], wherein the oily component comprising oleic acid as the main oil type component is one or more types of oily component selected from the group consisting of camellia oil, sunflower oil, avocado oil, avocado oil, safflower oil, almond oil, olive oil, rapeseed oil, and cashew oil;
[2-33] the self-emulsifying formulation of any one of [2-27] to [2-32], wherein the hydrophilic surfactant is one or more types of hydrophilic surfactants selected from the group consisting of polyoxyethylene hydroxystearate and polyoxyl 35 castor oil; and
[2-34] the self-emulsifying formulation of any one of [2-1] to [2-33], wherein the oily component is 8 vol% to 40 vol% based on the whole formulation (excluding the volume of the drug).

Furthermore, the following inventions are provided:

[3-1] a method of measuring membrane permeability of a test substance using Caco-2 cells, which comprises the step of pre-incubating Caco-2 cells in the mixed presence with the test substance;
[3-2] the method of [3-1], which comprises the step of pre-incubating Caco-2 cells in the mixed presence with the test substance for two hours or more;
[3-3] the method of [3-1] or [3-2], which comprises the step of pre-incubating Caco-2 cells in the mixed presence with the test substance for four hours or more;
[3-4] the method of any one of [3-1] to [3-3], which comprises the step of pre-incubating Caco-2 cells in the mixed presence with the test substance for six hours or more;
[3-5] the method of any one of [3-1] to [3-4], which comprises the step of pre-incubating Caco-2 cells in the mixed presence with the test substance for eight hours or more;
[3-6] the method of any one of [3-1] to [3-5], which comprises the step of pre-incubating Caco-2 cells in the mixed presence with the test substance for twelve hours or more;
[3-7] the method of any one of [3-1] to [3-6], which comprises the step of pre-incubating Caco-2 cells in the mixed presence with the test substance for 24 hours or more;
[3-8] the method of any one of [3-1] to [3-7], wherein pre-incubation time in the pre-incubating step is 48 hours or less;
[3-9] a pre-incubation method for measuring membrane permeability, which comprises the step of incubating Caco-2 cells in the mixed presence with a test substance for four hours or more;
[3-10] the method of [3-9], which comprises the step of incubating Caco-2 cells in the mixed presence with the test substance for 24 hours or more;
[3-11] the method of any one of [3-1] to [3-8], wherein the pre-incubation is performed by contacting the Caco-2 cells with a medium;
[3-12] the method of [3-11], wherein the medium is a cell culture medium;
[3-13] the method of [3-11], wherein the medium is DMEM;
[3-14] the method of any one of [3-1] to [3-13], wherein the test substance is a substance with ClogP of 3 or greater;
[3-15] the method of any one of [3-1] to [3-14], wherein the test substance is a peptide compound;
[3-16] the method of any one of [3-1] to [3-15], wherein the test substance is a cyclic peptide compound;
[3-17] the method of any one of [3-1] to [3-16], which further comprises the step of measuring membrane permeability of the test substance by placing a solution containing the test substance so that it contacts with one side of a Caco-2 cell layer, placing a solution not containing the test substance so that it contacts with the other side of the Caco-2 cell layer, and after a predetermined period of time, measuring the amount of the test substance in the solution on said one side and/or the amount of the test substance in the solution on said other side;
[3-18] a method of screening for a test substance, which comprises the steps of:

(a) measuring membrane permeability of a plurality of test substances by the method of any one of [3-1] to [3-17]; and
(b) selecting a desired test substance from the plurality of test substances based on the membrane permeability data obtained in (a);

[3-19] the method of [3-18], wherein the step (b) includes selecting a test substance having a membrane permeability coefficient ($P_{app}$) of $1.0 \times 10^{-6}$ cm/second or greater;

[3-20] a method of producing a peptide compound, which comprises the steps of:

(i) measuring membrane permeability of a plurality of peptide compounds by the method of any one of [3-1] to [3-17];
(ii) selecting a desired peptide compound from the plurality of peptide compounds based on the membrane permeability data obtained in (i); and
(iii) producing a peptide compound based on the amino acid sequence of the peptide compound selected in (ii); and

[3-21] a peptide compound produced by the method of [3-20].

[Effects of the Invention]

In the present invention, lymphatically transported self-emulsifying formulations which comprise a surfactant containing oleate ester or oleylether as a moiety and/or an oily component which comprises oleic acid as the main oil type component are applied to poorly membrane-permeable compounds, and there are thus provided self-emulsifying formulations in which membrane permeability and absorbability of the compounds are improved compared to those in conventional prescribed formulations which have been used as a lymphatically transported formulation. Furthermore, there provided are self-emulsifying formulations that achieve drug absorption profile with higher maximum blood concentration (Cmax) and shorter time taken to reach maximum blood concentration (Tmax) as compared to those of solution administration formulations.

Brief Description of the Drawings

Fig. 1 is a graph showing the change in blood concentration of SEDDS (1).
Fig. 2 is a graph showing the change in blood concentrations of SEDDS (2) to (4).
Fig. 3 is a graph showing the change in blood concentrations of SEDDS (5) to (9).
Fig. 4 is a graph showing the change in blood concentrations of SEDDS (10) to (14).
Fig. 5 is a graph showing the change in blood concentrations of SEDDS (15) to (19).
Fig. 6 is a graph showing the change in blood concentrations of SEDDS (20) to (24).
Fig. 7 is a graph showing the change in blood concentrations of SEDDS (25) to (29).
Fig. 8 is a graph showing the change in blood concentrations of SEDDS (30) and (31).
Fig. 9 is a graph showing the change in blood concentration of SEDDS (35).
Fig. 10 is a graph showing the change in blood concentration of SEDDS (36).
Fig. 11 is a graph showing the change in blood concentration of SEDDS (37).
Fig. 12 is a graph showing the change in blood concentration of SEDDS (38).
Fig. 13 is a graph showing the change in blood concentration of SEDDS (39).
Fig. 14 is a graph showing the change in blood concentration of SEDDS (1) in mice whose lymphatic absorption was inhibited.
Fig. 15 is a graph showing the change in blood concentration of SEDDS (1) in mice whose P-gp and BCRP transporters were knocked out.
Fig. 16 is a diagram showing the preferred physical property range (logD (pH 7.4), Caco-2 Papp (cm/sec)) of compounds whose drug membrane permeability can be enhanced by lymphatically transported self-emulsifying formulations of the present invention.
Fig. 17 is a diagram showing an outline for an embodiment of the method of measuring membrane permeability.
Fig. 18 is a conceptual diagram showing the presupposed and actual cell membrane permeability measurements using Caco-2 cells.
Fig. 19 is a graph showing the change in TEER when Caco-2 cells were incubated up to three hours according to a conventional method.
Fig. 20 is a graph showing the change in TEER when Caco-2 cells were incubated up to 24 hours according to an improved method.
Fig. 21 is a graph showing the correlation between $P_{app}$ and Fa measured by a conventional method.
Fig. 22 is a graph showing the correlation between $P_{app}$ and Fa measured by an improved method. Compared to the conventional method, the improved method showed higher correlation with Fa.

Mode for Carrying Out the Invention

- Poorly membrane-permeable drugs (Drugs with low membrane permeability)

The term "poorly membrane-permeable drug (drug with low membrane permeability)" of the present invention means a compound (drug) that is hardly absorbed from the digestive tract when administered orally. The "poorly membrane-permeable drug (drug with low membrane permeability)" in the present invention is not particularly limited as long as it has such features, and examples include middle molecular-weight compounds (for example, molecular weight of 500 to 6000 or so) including natural products, sugar chains, peptides, and nucleic acid medicines, and preferred examples include cyclic peptides.

Whether compounds (drugs) are hardly absorbed from the digestive tract or not can be determined, for example, by checking the membrane permeability of a compound of interest using the following known methods. The methods for checking membrane permeability include, for example, rat intestine methods, cultured cell (Caco-2, MDCK, HT-29, LLC-PK1, *etc.*) monolayer methods, immobilized artificial membrane chromatography methods, methods using distribution coefficients, ribosome membrane method, or parallel artificial membrane permeation assay (PAMPA) methods. Specifically, when using the PAMPA method, for example, membrane permeability can be checked according to the method described in the literature of Holger Fischer *et al.* (NPL: H. Fischer et al., Permeation of permanently positive charged molecules through artificial membranes-influence of physic-chemical properties. Eur J. Pharm. Sci. 2007, 31, 32-42).

When the compound formulated as self-emulsifying formulations in the present invention is a peptide, selection of the amino acid residues constituting the peptide is not particularly limited. When the peptide includes a chemically modified non-natural amino acid (amino acid analog), the compound is preferably a compound wherein the CLogP (a computed distribution coefficient which can be calculated, for example, by using Daylight Version 4.9 (Daylight Chemical Information Systems, Inc.)) of the formed molecule is 3 or greater, 4 or greater, 5 or greater 6 or greater, 7 or greater, or 8 or greater, and is 20 or less, 19 or less, 18 or less, 17 or less, 16 or less, or 15 or less when the peptide is regarded as the shape (main chain structure) of the molecule in which all of the chemical modifications are completed.

As a non-limiting embodiment, for evaluation of membrane permeability of drugs targeted for the self-emulsifying formulations in the present invention, checks are more preferably done by the later-described Caco-2 measurements. More specifically, membrane permeability can be checked according to the method described in Example 1-2-1.

As a non-limiting embodiment, for the "poorly membrane-permeable drug (drug with low membrane permeability)" of the present invention, the value obtained by the above-mentioned Caco-2 measurement (Caco-2 Papp (cm/sec)) is preferably 1.0E-5 or less, 9.0E-6 or less, 8.0E-6 or less, 7.0E-6 or less, 6.0E-6 or less, 5.0E-6 or less, 4.0E-6 or less, or 3.0E-6 or less, and more preferably 2.0E-6 or less, 1.8E-6 or less, 1.6E-6 or less, 1.4E-6 or less, 1.2E-6 or less, 1.0E-6 or less, 9.8E-7 or less, 9.6E-7 or less, 9.4E-7 or less, 9.2E-7 or less, 9.0E-7 or less, 8.8E-7 or less, 8.6E-7 or less, 8.4E-7 or less, 8.2E-7 or less, 8.0E-7 or less, 7.8E-7 or less, 7.6E-7 or less, 7.4E-7 or less, 7.2E-7 or less, 7.0E-7 or less, 6.8E-7 or less, 6.6E-7 or less, 6.4E-7 or less, 6.2E-7 or less, 6.0E-7 or less, 5.8E-7 or less, 5.6E-7 or less, 5.4E-7 or less, 5.2E-7 or less, 5.0E-7 or less, 4.8E-7 or less, 4.6E-7 or less, 4.4E-7 or less, 4.2E-7 or less, 4.0E-7 or less, 3.8E-7 or less, 3.6E-7 or less, 3.4E-7 or less, 3.2E-7 or less, 3.0E-7 or less, 2.8E-7 or less, 2.6E-7 or less, 2.4E-7 or less, 2.2E-7 or less, 2.0E-7 or less, 1.8E-7 or less, 1.6E-7 or less, 1.4E-7 or less, 1.2E-7 or less, or 1.0E-7 or less. E-n (n is a natural number) means $10^{-n}$ (for example, 1.0E-5 = $1.0 \times 10^{-5}$).

As a non-limiting embodiment, evaluation of membrane permeability (Caco-2) of drugs to be targeted for the self-emulsifying formulations in the present invention can be carried out by the following method.

1) Caco-2 cells are cultured on a 96-well transwell for three weeks, then DMEM, FaSSIF (1% DMSO), and a drug are added to the Apical side and DMEM is added to the Basal side, and this is pre-incubated under the condition of 5% $CO_2$, 37°C, and 80 rpm for 20 hours to 24 hours.
2) After pre-incubation, the pre-incubation solutions on the Apical side and on the Basal side are removed by aspiration and washed, FaSSIF/HBSS buffer (pH 6.0) containing the drug is added to the Apical side, and HBSS buffer (pH 7.4) containing 4% BSA is added to the Basal side (initiation of permeability test).
3) Each well is shaken at 5% $CO_2$, 37°C, and 80 rpm, and 180 minutes after initiation, samples are collected from the Basal side, and the amount of permeated drug is determined by LC/MS.
4) The permeability coefficient of the drug is calculated from the determined amount of permeation (Caco-2 Papp (cm/sec)).

The present invention also provides such methods of evaluating membrane permeability of drugs to be targeted for the self-emulsifying formulations of the present invention.

Generally, polar functional groups that are excessively ionized *in vivo* (pH = around 7) such as an alkylamino group and an alkylguanidino group are not preferred in acquiring membrane permeability. However, compounds targeted

for the self-emulsifying formulations of the present invention may comprise these functional groups in their structures. When the compound targeted for the self-emulsifying formulations of the present invention is a peptide, a total number of amino acids included in the peptide compound is, although not particularly limited, preferably 25 or less, 20 or less, 18 or less, 17 or less, 16 or less, 15 or less, or 14 or less, and more preferably 13 or less (for example, 12, 11, 10, or 9).

In a non-limiting embodiment, the "poorly membrane-permeable drug (drug with low membrane permeability)" in the present invention can also be defined by a value obtained by a LogD (pH 7.4) measurement, in addition to a value obtained by the above-described Caco-2 measurement. LogD (pH 7.4) of a compound can be measured appropriately by those skilled in the art using a known method. More specifically, it can be checked according to the method described in Example 1-2-2.

In a non-limiting embodiment, the "poorly membrane-permeable drug (drug with low membrane permeability)" in the present invention is a drug in which a value obtainable by the above-mentioned LogD (pH 7.4) measurement is preferably 2.0 or greater, 2.1 or greater, 2.2 or greater, 2.3 or greater, 2.4 or greater, 2.5 or greater, 2.6 or greater, 2.7 or greater, 2.8 or greater, 2.9 or greater, 3.0 or greater, or 3.1 or greater, and is more preferably 3.2 or greater, 3.3 or greater, 3.4 or greater, 3.5 or greater, 3.6 or greater, 3.7 or greater, 3.8 or greater, 3.9 or greater, 4.0 or greater, 4.1 or greater, 4.2 or greater, 4.3 or greater, 4.4 or greater, 4.5 or greater, 4.6 or greater, 4.7 or greater, 4.8 or greater, 4.9 or greater, 5.0 or greater, 5.1 or greater, 5.2 or greater, 5.3 or greater, 5.4 or greater, or 5.5 or greater.

In a non-limiting embodiment, the "poorly membrane-permeable drug (drug with low membrane permeability)" in the present invention is particularly preferably a drug in which the value obtained by the above-mentioned Caco-2 measurement (Caco-2 Papp (cm/sec)) and the value obtained by the above-mentioned LogD (pH 7.4) measurement are within either one or both of the following ranges:

(i) logD (pH 7.4) value of the drug is 3.2 or greater; and
(ii) Caco-2 Papp (cm/sec) value of the drug is 1.8E-6 or less.

Preferred combination (Caco-2:LogD) of the values obtained by the above-mentioned Caco-2 measurement (Caco-2 Papp (cm/sec)) and the above-mentioned LogD (pH 7.4) measurement is 3.2 or greater: 1.8E-6 or less, 3.3 or greater:1.8E-6 or less, 3.4 or greater:1.8E-6 or less, 3.5 or greater:1.8E-6 or less, 3.6 or greater: 1.8E-6 or less, 3.7 or greater:1.8E-6 or less, 3.8 or greater: 1.8E-6 or less, 3.9 or greater:1.8E-6 or less, 4.0 or greater: 1.0E-6 or less, 4.0 or greater:9.0E-7 or less, 4.0 or greater:8.0E-7 or less, 4.0 or greater:7.0E-7 or less, 4.0 or more:6.0E-7 or less, 4.0 or greater:5.0E-7 or less, 4.0 or greater:4.0E-7 or less, 4.0 or greater:3.0E-7 or less, 4.0 or greater:2.0E-7 or less, 4.0 or greater: 1.0E-7 or less, 4.1 or greater:9.0E-7 or less, 4.2 or greater:8.0E-7 or less, 4.3 or greater:7.0E-7 or less, 4.4 or greater:6.0E-7 or less, 4.5 or greater:5.0E-7 or less, 4.6 or greater:4.0E-7 or less, 4.7 or greater:4.0E-7 or less, 4.8 or greater:4.0E-7 or less, 4.9 or greater:4.0E-7 or less, 5.0 or greater:4.0E-7 or less, 5.2 or greater:4.0E-7 or less, 5.4 or greater:3.0E-7 or less, or 5.6 or greater:2.0E-7 or less.

The compounds targeted for the self-emulsifying formulations in the present invention are not particularly limited, but their molecular weights are preferably 500 or greater, 550 or greater, 600 or greater, 650 or greater, 700 or greater, 750 or greater, 800 or greater, 850 or greater, 900 or greater, or 950 or greater, and particularly preferably 1000 or greater, 1100 or greater, 1200 or greater, 1300 or greater, 1400 or greater, 1500 or greater, 1600 or greater, 1700 or greater, or 1800 or greater. The upper limit of the molecular weight is not particularly limited, but the molecular weight is preferably 6000 or less, 5000 or less, 4000 or less, 3000 or less, 2500 or less, or 2000 or less.

In a non-limiting embodiment, the "poorly membrane-permeable drug (drug with low membrane permeability)" in the present invention is preferably a compound also having satisfactory metabolic stability. For the compounds targeted for the self-emulsifying formulations in the present invention to have satisfactory metabolic stability, a total number of amino acids included in the peptide compounds is preferably 9 or more, and more preferably 10 or more (for example, 10 or 11).

The metabolic stability of the compounds targeted for the self-emulsifying formulations in the present invention can be checked by known methods using, for example, hepatocytes, small intestinal cells, liver microsomes, small intestinal microsomes, or liver S9. Specifically, the stability of the peptide compounds can be checked, for example, by measuring the stability of the peptide compounds in the liver microsome according to the description in the literature of LL von Moltke et al. (Midazolam hydroxylation by human liver microsomes in vitro: inhibition by fluoxetine, norfluoxetine, and by azole antifungal agents. J Clin Pharmacol, 1996, 36(9), 783-791).

For example, when the intrinsic hepatic clearance (CLh int (μL/min/mg protein)) value when stability in the liver microsome is measured according to the above-described method is 150 or less, or preferably 100 or less, 90 or less, 80 or less, 70 or less, or 60 or less, or particularly preferably 50 or less, 40 or less, or 30 or less, it can be determined that metabolic stability allowing for the use as oral pharmaceuticals can be obtained. In the case of drugs metabolized by CYP3A4, to avoid its metabolism in the small intestine of humans, the intrinsic hepatic clearance value is preferably 78 or less (NPL: M. Kato et al., The intestinal first-pass metabolism of substances of CYP3A4 and P-glycoprotein-quantitative analysis based on information from the literature. Drug Metab. Pharmacokinet. 2003, 18(6), 365-372), and

to exhibit bioavailability of approximately 30% or higher in humans, the value is preferably 35 or less (assuming that FaFg is 1 and protein binding rate is 0%).

The results from metabolic stability assay on compounds (1) to (6) described in the Examples herein in human liver microsomes were 74, 48, 46, 189, 58, and 86 (CLh int (μL/min/mg protein)), respectively (WO 2013/100132).

An example of methods for evaluating hepatic metabolism from iv clearance in *in vivo* assays includes methods of calculating hepatic availability (Fh) from the ratio between the hepatic blood flow rate and hepatic clearance.

In a non-limiting embodiment, the "poorly membrane permeable drug (drug with low membrane-permeability)" in the present invention has an intrinsic hepatic drug clearance (CLh int (μL/min/mg protein)) value of 150 or less, 100 or less, 90 or less, 80 or less, 70 or less, or 60 or less, or particularly preferably 50 or less, 40 or less, or 30 or less.

In a non-limiting embodiment, the "poorly membrane permeable drug (drug with low membrane permeability)" in the present invention is a drug having an Fh value (hepatic availability) of 10% or higher, 20% or higher, 30% or higher, 40% or higher, 50% or higher, 60% or higher, 70% or higher, 80% or higher, or 90% or higher.

Furthermore, the compounds which are targeted for the self-emulsifying formulations of the present invention may be water-insoluble compounds. For example, a "water-insoluble compound" means a compound having solubility in ion-exchanged water at 20°C of preferably 10 mg/mL or lower, or 1 mg/mL or lower, or more preferably 0.1 mg/mL or lower, 0.01 mg/mL or lower, or 0.001 mg/mL or lower.

- Middle molecular-weight compounds (for example, molecular weight of 500 to 6000) containing a cyclic peptide

Compounds targeted for the self-emulsifying formulations in the present invention are, without being limited thereto, preferably a "peptide compound having a cyclic portion". The phrase "peptide compound having a cyclic portion" of the present invention is not particularly limited as long as it is a peptide compound formed by formation of an amide bond or an ester bond between natural amino acids or amino acid analogs, and having a cyclic portion. The cyclic portion is preferably formed *via* a covalent bonding such as an amide bonding, carbon-carbon bond-forming reaction, S-S bonding, thioether bonding, or triazole bonding (WO 2013/100132; WO 2012/026566; WO 2012/033154; WO 2012/074130; WO 2015/030014; Comb Chem High Throughput Screen. 2010; 13: 75-87; Nature Chem. Bio. 2009, 5, 502; Nat Chem Biol. 2009, 5, 888-90; Bioconjugate Chem., 2007, 18, 469-476; ChemBioChem, 2009, 10, 787-798; and Chemical Communications (Cambridge, United Kingdom) (2011), 47(36), 9946-9958). Compounds obtained by further chemically modifying these compounds are also included in the peptide compounds of the present invention. The peptide compounds comprising a cyclic portion of the present invention may further comprise a linear chain portion. The number of the amide bonds or the ester bonds (the number and length of natural amino acids or amino acid analogs) is not particularly limited, and when the peptide compounds comprise a linear chain portion, the total residues of the cyclic portion and the linear chain portion are preferably 30 or less. More preferably, to acquire high metabolic stability, the total number of amino acids is 9 or more. In addition to the description above, the number of natural amino acids and amino acid analogs constituting the cyclic portion is more preferably 5 residues to 12 residues, 6 residues to 12 residues, or 7 residues to 12 residues, and even more preferably 7 residues to 11 residues, or 8 residues to 11 residues. 9 residues to 11 residues (10 residues or 11 residues) are particularly preferred. The number of amino acids and amino acid analogs in the linear chain portion is preferably 0 to 8, 0 to 7, 0 to 6, 0 to 5, or 0 to 4, and more preferably 0 to 3. The total number of natural amino acids and amino acid analogs are preferably 6 residues to 20 residues, 7 residues to 19 residues, 7 residues to 18 residues, 7 residues to 17 residues, 7 residues to 16 residues, 7 residues to 15 residues, 8 residues to 14 residues, or 9 residues to 13 residues. Herein, unless particularly limited, amino acids include natural amino acids and amino acid analogs.

The types of natural amino acid residues and amino acid analog residues forming the cyclic portion of the peptide compound comprising a cyclic portion of the present invention are not particularly limited, but the cyclized portion is preferably composed of amino acid residues or amino acid analog residues having functional groups with excellent metabolic stability. The cyclization methods for the peptide compound comprising a cyclic portion of the present invention are not particularly limited as long as they are methods that can form such a cyclic portion. Examples include amide bonding formed from carboxylic acid and amine, and carbon-carbon bonding reaction using a transition metal as a catalyst such as Suzuki reaction, Heck reaction, and Sonogashira reaction. Thus, the peptide compounds of the present invention contain at least one set of functional groups capable of such bonding reaction prior to cyclization. From the viewpoint of metabolic stability in particular, it is preferred that the peptide compounds include functional groups that form an amide bond as a result of the bonding reaction.

For example, it is preferred that the formation of the cyclic portion does not comprise a bond including heteroatoms, which may be easily oxidized, and a bond that hinders metabolic stability. For example, the bond generated by the cyclization includes an amide bond formed from bonding between an activated ester and an amine, and a bond formed by a Heck reaction product from a carbon-carbon double bond and an arylhalide.

Herein, "an amino acid" constituting the peptide compounds may be "a natural amino acid" or "an amino acid analog". The "amino acid", "natural amino acid", and "amino acid analog" may be referred to as "amino acid residue",

"natural amino acid residue", and "amino acid analog residue", respectively.

"Natural amino acids" are α-amino carboxylic acids (a-amino acids), and refer to the 20 types of amino acids included in proteins. Specifically, they refer to Gly, Ala, Ser, Thr, Val, Leu, Ile, Phe, Tyr, Trp, His, Glu, Asp, Gln, Asn, Cys, Met, Lys, Arg, and Pro. "Amino acid analogs" are not particularly limited, and include β-amino acids, γ-amino acids, D-amino acids, *N*-substituted amino acids, α,α-disubstituted amino acids, hydroxycarboxylic acids, and non-natural amino acids (amino acids with side chains that are different from those of natural amino acids: for example, non-natural α-amino acids, non-natural β-amino acids, and non-natural γ-amino acids). The α-amino acids may be D-amino acids or α,α-dialkyl amino acids. Similarly to α-amino acids, any conformations are allowed for the β-amino acids and γ-amino acids. There is no particular limitation on the selection of amino acid side chain, but in addition to a hydrogen atom, it can be freely selected from, for example, an alkyl group, an alkenyl group, an alkynyl group, an aryl group, a heteroaryl group, an aralkyl group, and a cycloalkyl group. Substituents may be added to each of them, and such substituents are freely selected from any functional groups including, for example, a nitrogen atom, an oxygen atom, a sulfur atom, a boron atom, a silicon atom, and a phosphorous atom (*i.e.,* an optionally substituted alkyl group, alkenyl group, alkynyl group, aryl group, heteroaryl group, aralkyl group, cycloalkyl group, and such).

"Natural amino acids" and "amino acid analogs" constituting the peptide compounds include all isotopes corresponding to them. The isotope of the "natural amino acid" or "amino acid analog" refers to one having at least one atom replaced with an atom of the same atomic number (number of protons) and different mass number (total number of protons and neutrons). Examples of the isotopes contained in the "natural amino acid" or "amino acid analog" constituting the peptide compounds of the present invention include a hydrogen atom, a carbon atom, a nitrogen atom, an oxygen atom, a phosphorus atom, a sulfur atom, a fluorine atom and a chlorine atom, which respectively include $^{2}H$ and $^{3}H$; $^{13}C$ and $^{14}C$; $^{15}N$; $^{17}O$ and $^{18}O$; $^{31}P$ and $^{32}P$; $^{35}S$; $^{18}F$; and $^{36}Cl$.

The "peptide compound" targeted for the self-emulsifying formulations in the present invention is preferably a cyclic peptide satisfying the condition of having at least two *N*-substituted amino acids (preferably 2, 3, 4, 5, 6, 7, 8, 9, or 10, or particularly preferably 5, 6, or 7) and having at least one amino acid without *N*-substitution, alone or in combination with the above-mentioned condition for the total number of natural amino acids and amino acid analogs. Examples of "*N*-substitution" include, but are not limited to, substitution of the hydrogen atom bonded to a nitrogen atom with a methyl group, an ethyl group, a propyl group, a butyl group, or a hexyl group. Preferred *N*-substituted amino acids include amino acids in which the amino group included in the natural amino acids has been *N*-methylated.

The compounds targeted for the self-emulsifying formulations in the present invention may be, for example, non-cyclic peptides other than the above-described cyclic peptides, or compounds such as natural products other than peptides; however, it is preferred that compounds satisfy at least one or more of the above-mentioned criteria for Caco-2 Papp (cm/sec), criteria for logD (pH 7.4), criteria for metabolic stability (CLh int (μL/min/mg protein)), and criteria for a molecular weight.

- Self-emulsifying formulations

In a non-limiting embodiment, the present invention provides lymphatically transported self-emulsifying formulations, which comprise a surfactant comprising oleate ester or oleylether as a moiety, an oily component, and a poorly membrane-permeable drug.

In a non-limiting embodiment, the present invention provides lymphatically transported self-emulsifying formulations, which comprise a surfactant, an oily component which comprises oleic acid as the main oil type component, and a poorly membrane-permeable drug.

Herein, the term "self-emulsifying formulation" refers to a Self-Emulsifying Drug Delivery System (SEDDS) which is composed of a water-insoluble drug, oil, a hydrophilic surfactant, a lipophilic surfactant, an absorption promoter, an auxiliary solvent, and such (see Gursoy, N.R., et al., 2004, Biomedicine & pharmacotherapy 58, 173-182, and such). Furthermore, a self-emulsifying formulation can also be expressed as a self-emulsifying microemulsion formulation, a self-emulsifying emulsion formulation, a microemulsion formulation, or an emulsion formulation.

The self-emulsifying formulations of the present invention can be formulated by known methods. In a non-limiting embodiment, a method for evaluating the emulsion property of the formed self-emulsifying formulation in the present invention is as follows. For example, particle size, separation of emulsion which is physical stability, and such are known as indicators representing the physical properties of an emulsion. Therefore, ordinarily, properties of an emulsion can be evaluated using these indicators. Conventionally known methods can be used as the methods for evaluating such properties. For example, dynamic light scattering (hereinafter referred to as "DLS") can be used as a method for measuring the particle size. Furthermore, methods of evaluating turbidity by a turbidimeter as a parameter reflecting the particle size can be used. While not being limited thereto, the average particle size is preferably less than 1 μm, more preferably less than 500 nm, and even more preferably less than 250 nm to less than 150 nm. While not being limited thereto, the turbidity (200 μL/well, when measured at incident light of 650 nm) is preferably 0.3 to 1, and more preferably less than 0.3.

In a non-limiting embodiment, the particle size for the emulsion formed by SEDDS of the present invention can be evaluated rapidly by a turbidity measurement using an absorption spectrometer for microplates (WO 2013/100132). Specifically, the following methods may be used as a method for evaluating emulsions:

(1) a method for evaluating the particle size of the emulsion by turbidity measurements;
(2) a method for evaluating the separation stability of the emulsion by measuring the change in its turbidity before and after storage; and/or
(3) a method for evaluating the separation stability of the emulsion by measuring the change in its turbidity before and after centrifugation.

Furthermore, an evaluation by combining two or more of the evaluation methods of (1) to (3) allows determination of the most suitable pharmaceutical formulation based on the evaluation results.
The following are examples of methods for evaluating the particle size by turbidity measurements. Generally, the appearance of an aqueous SEDDS solution is known to change based on the emulsion particle size, and the conditions of the appearance can be classified broadly into three conditions, specifically:

(1) a condition having clear appearance due to formation of an emulsion having a particle size of approximately 150 nm or smaller (microemulsion, hereafter referred to as "ME"; turbidity of less than 0.3 at 200 μL/well);
(2) a condition having transparent but albescent appearance due to increase in emulsion particle size compared to ME, or formation of a mixture of such enlarged emulsion and ME (white microemulsion, hereafter referred to as "WME"; turbidity of 0.3 to 1 at 200 (μL/well); and
(3) a condition having opaque and white appearance due to formation of an emulsion having particle size in the order of micrometers (macroemulsion, hereafter referred to as "MacE"; turbidity of greater than 1 at 200 μL/well).

As described above, the appearance of an emulsion changes greatly depending on the particle size. Therefore, particle size can easily be evaluated by measuring turbidity. Furthermore, measurements with low-volume samples are possible by using absorption spectrometer for microplates (for example, SpectraMax 190, Molecular Devices), and therefore low-cost and rapid evaluation becomes possible.
Evaluation of separation stability based on change in turbidity can be performed by evaluating the separation stability of the emulsion through measurement of the change in turbidity before and after storage of the emulsion or change in turbidity before and after centrifugation of the emulsion. Whether SEDDS is separated or not can be evaluated by storing the prepared emulsion under conditions of 1°C or higher to 40°C or lower for six hours or more to 72 hours or less, and evaluating the change in turbidity before and after the storage.
Alternatively, for evaluation under more stringent conditions and within a short period of time, the prepared emulsion can be subjected to centrifugation treatment. In this case, for example, centrifugation is carried out under conditions of 1,500 rpm to 2,000 rpm, and the change in turbidity before and after centrifugation is measured. In this case, the particle size increases through aggregation of emulsion particles with each other over time, and the particles with increased size are separated by centrifugation. This enables more accurate and quicker evaluation of turbidity. Furthermore, separated forms include the following cases: (1) separation into a cream layer and a transparent layer; and (2) separation into a transparent layer and another transparent layer. Such separations can also be evaluated by turbidity. Furthermore, in some cases, drug precipitation and generation of insoluble materials accompanied with change in the combination of the prescribed components can be detected.
In a non-limiting embodiment, SEDDS formulations of the present invention include oily components and surfactants, and when necessary, hydrophilic surfactants and oleic acid, and optionally absorption promoters, auxiliary solvents, and such can be added.
In a non-limiting embodiment, SEDDS formulations of the present invention comprise oily components and surfactants. As the surfactant, hydrophilic surfactants or lipophilic surfactants, or both can be used. When necessary, oleic acid, and optionally absorption promoters, auxiliary solvents, and such can be added.
Hydrophilic-lipophilic balance (hydrophile-lipophile balance; HLB) is known as an indicator that represents the effects exhibited by surfactants. The HLB refers to evenly divided values, defining a substance without a hydrophilic group as HLB = 0 and a substance without a lipophilic group as HLB = 20. HLB can be calculated by methods known to those skilled in the art such as the Griffin method. Generally, a surfactant having the HLB value of 9 or greater can be determined to be a hydrophilic surfactant, and a surfactant having the HLB value of less than 9 can be determined to be a lipophilic surfactant. Hydrophilicity increases as the HLB value increases (nears 20), and lipophilicity increases as the HLB value decreases (nears 0).
The oily components used here are not particularly limited, and examples include olive oil, almond oil, coconut oil, cacao butter, macadamia nut oil, avocado oil, safflower oil, soybean oil, linseed oil, rapeseed oil, castor oil, palm oil, high-oleic sunflower oil, high-oleic safflower oil, sunflower oil, cotton seed oil, corn oil, sesame oil, peanut oil, apricot

kernel oil, candlenut oil, grape seed oil, pistachio seed oil, sunflower oil, hazelnut oil, jojoba oil, meadowfoam oil, rosehip oil, Tricaproin, Tricaprylin, Tricaprin, Tripalmitolein, Triolein, Trilinolein, Trilinolenin, Trieicosenoin, and Trierucin. Other than the examples given above, the oily components may be plant oils collected from plants, partially degraded products obtained by hydrolyzing the plant oils, or plant oils subjected to separation and purification. Furthermore, the oily components may be those obtained through synthesis by synthetic methods. One type of oily component or a combination of two or more types of oily components may be used.

The oily components used here are not particularly limited, and are, for example, triacylglycerol, diacylglycerol, and monoacylglycerol, and may be one of these types or a mixture of them. Fatty acids chemically bonded or added to these backbones have a C6-C22 hydrocarbon chain, the number of double bonds in the chain may be a number in the whole possible range.

While other oily components are not particularly limited, examples include oily components comprising oleic acid as the main oil type component.

Herein, the term "main oil type component" means a component having a content ratio of 50% or higher, preferably 60% or higher, and more preferably 70% or higher, or 80% or higher in the target oily component. For example, since olive oil contains approximately 11.6% palmitic acid, approximately 1.0% palmitoleic acid, approximately 3.1% stearic acid, approximately 75.0% oleic acid, and approximately 7.8% linoleic acid, the main oil type component of olive oil can be determined to be oleic acid. For example, since soybean oil contains approximately 54% linoleic acid, approximately 22% oleic acid, approximately 11% palmitic acid, approximately 9% linolenic acid, and approximately 4% stearic acid, the main oil type component of soybean oil can be determined to be linoleic acid.

Oily components comprising oleic acid as the main oil type component preferably include camellia oil, sunflower oil, avocado oil, safflower oil, almond oil, olive oil, rapeseed oil, cashew oil, and apricot kernel oil. The oily component may be one type, or two or more types may be used in combination.

Herein, the phrase "surfactant comprising oleate ester or oleylether as a moiety" refers to a surfactant comprising oleate ester or oleylether in its chemical structure. For example, glyceryl monooleate which is a type of surfactant can be determined to be a surfactant comprising oleate ester as a moiety since it has a chemical structure in which one oleic acid is ester-linked to glycerin. Furthermore, for example, ethylene oxide oleylether which is a type of surfactant can be determined to be a surfactant comprising oleylether as a moiety since it has a chemical structure in which oleyl alcohol is ether-linked to ethylene oxide.

In the case of surfactants synthesized using oily components comprising a plurality of types of fatty acids as raw materials, if oleic acid is included in the oily components, such surfactants can also be determined to be surfactants comprising oleate ester as a moiety. For example, since apricot kernel oil contains approximately 70% oleic acid, approximately 23% linoleic acid, approximately 4.3% palmitic acid, approximately 1.1% stearic acid, and approximately 0.7% palmitoleic acid, surfactants synthesized using apricot kernel oil as raw materials can be determined to be one of surfactants comprising oleate ester as a moiety. The proportion of oleic acid included in the oily component which is used as raw materials of the surfactants is preferably 20% or higher or 30% or higher, and more preferably 40% or higher or 50% or higher, and particularly preferably 60% or higher, 70% or higher, or 80% or higher.

The surfactants comprising oleate ester as a moiety used herein are not particularly limited, and include glycerin fatty acid esters such as glyceryl monooleate; polyglyceryl fatty acid esters such as decaglyceryl monooleate, polyglyceryl-3 oleate, and polyglyceryl-3 dioleate; polyoxyethylene sorbitan fatty acid esters such as sorbitan monooleate and sorbitan trioleate; polyethylene glycol fatty acid esters such as polyethylene glycol (10) monooleate, polyethylene glycol (15) monooleate, polyethylene glycol (20) monooleate, polyethylene glycol (30) monooleate, and polyethylene glycol (35) monooleate; and apricot kernel oil polyoxyethylene-6 ester. The surfactant may be one type, or two types or more may be used in combination.

While the surfactants comprising oleylether as a moiety used herein are not particularly limited, examples include polyoxyethylene alkylethers such as polyethylene glycol (10) oleylether, polyethylene glycol (15) oleylether, polyethylene glycol (20) oleylether, and polyethylene glycol (50) oleylether. The surfactant may be one type, or two types or more may be used in combination.

While the hydrophilic surfactants used herein are not particularly limited, examples include polyoxyethylene hydroxystearate, polyoxyethylene hydroxyoleate, polyoxyethylene castor oil such as polyoxyl 35 castor oil, and polyoxyethylene hardened castor oil such as polyoxyl 40 hardened castor oil. While the lipophilic surfactants are not particularly limited, examples include glycerin fatty acid esters such as glyceryl monooleate and glyceryl monolinolenate; and polyoxyethylene sorbitan fatty acid esters such as sorbitan trioleate. The surfactant may be one type, or two types or more may be used in combination.

In a non-limiting embodiment, when preparing SEDDS formulations of the present invention, absorption enhancers such as sodium salicylate, sodium deoxycholate, sodium myristate, and sodium dodecyl sulfate; auxiliary solvents such as ethanol, propylene glycol, polyethylene glycol, diethylenetriamine pentaacetic acid, diethanolamine, triethanolamine, ethylenediamine, monoethanolamine, and *N*,*N*-dimethylacetamide; and such can be combined in addition to the above-mentioned constituents. For example, when using propylene glycol or polyethylene glycol as the auxiliary

solvent, it is preferably used by diluting with a diluting solvent.

One can refer to publicly known documents and reference documents (see Japanese Pharmacopoeia 13th edition; of the Japanese Pharmaceutical Codex 1997; Japanese Pharmaceutical Excipients 1998; Japanese Specifications and Standards for Food Additives, 7th Edition; Revised edition of Japanese Standards of Cosmetic Ingredients; Japanese Cosmetic Ingredients Codex; Japanese Standards of Quasi-drug Ingredients; United States Pharmacopeia 24; British Pharmacopeia 2000; European Pharmacopeia 2000; and National Formulary 19; and such) for oily components, surfactants, and additives which can be used in the present invention.

Furthermore, emulsification adjuvants, stabilizing agents, antiseptic agents, surfactants, antioxidants, polymers, and such may be included in self-emulsifying compositions of the present invention. Examples of emulsification adjuvants include 12-carbon to 22-carbon fatty acids such as stearic acid, oleic acid, linoleic acid, palmitic acid, linolenic acid, and myristic acid, and salts thereof, and oleic acid is preferred. Examples of stabilizing agents include phosphatidic acid, ascorbic acid, glycerol, and cetanol. Examples of antiseptic agents include ethyl parahydroxybenzoate and propyl parahydroxybenzoate. Examples of antioxidants include oil-soluble antioxidants such as butyrated hydroxytoluene, butyrated hydroxyanisol, propyl gallate, propyl gallate, pharmaceutically acceptable quinones, astaxanthin, and $\alpha$-tocopherol. Examples of polymers include hypromellose, hypromellose phthalate ester, hypromellose acetate ester succinate ester, polyvinylpyrrolidone, and copovidone (vinylpyrrolidone vinylacetate).

In a non-limiting embodiment, the present invention provides self-emulsifying formulations comprising a surfactant, an oily component, and a poorly membrane-permeable drug, wherein the oily component is 8 vol% to 40 vol% based on the whole formulation (excluding the volume of the drug).

In a non-limiting embodiment, the volume of the oily component in the whole formulation (excluding the volume of the drug) is preferably 0.1 vol% or more, 0.5 vol% or more, 1.0 vol% or more, or 5.0 vol% or more, preferably 5.0 vol% to 50 vol%, 10 vol% to 50 vol%, 15 vol% to 50 vol%, or 15 vol% to 40 vol%, and particularly preferably 20 vol% to 40 vol% or 20 vol% to 35 vol%.

In a non-limiting embodiment, surfactants comprising oleate ester or oleylether as a moiety may be hydrophilic surfactants or lipophilic surfactants as long as they are surfactants comprising the moiety.

While not being limited to the following, when the above-mentioned surfactant is a lipophilic surfactant, the volume of the lipophilic surfactant in the whole formulation (excluding the volume of the drug) is preferably 0.1 vol% or more, 0.5 vol% or more, 1.0 vol% or more, or 5.0 vol% or more, and particularly preferably 5 vol% to 80 vol%, 5 vol% to 70 vol%, 5 vol% to 60 vol%, 5 vol% to 50 vol%, 5 vol% to 40 vol%, 5 vol% to 30 vol%, 5 vol% to 20 vol%, or 9 vol% to 19 vol%.

While not being limited to the following, when the above-mentioned surfactant is a hydrophilic surfactant, the volume of the hydrophilic surfactant in the whole formulation (excluding the volume of the drug) is preferably 10 vol% to 90 vol%, 20 vol% to 80 vol%, or 30 vol% to 70 vol%, and particularly preferably 40 vol% to 65 vol%, 45 vol% to 65 vol%, 50 vol% to 65 vol%, or 51 vol% to 61 vol%. The surfactant may be one type (lipophilic surfactant or lipophilic surfactant), or a lipophilic surfactant and a hydrophilic surfactant may be used in combination.

In a non-limiting embodiment, the self-emulsifying formulations of the present invention can further comprise a hydrophilic surfactant, in addition to the above-mentioned surfactant comprising oleate ester or oleylether as a moiety. The volume of the hydrophilic surfactant in the whole formulation (excluding the volume of the drug) is preferably 10 vol% to 90 vol%, 20 vol% to 80 vol%, 30 vol% to 70 vol%, or 40 vol% to 60 vol%.

In a non-limiting embodiment, the present invention provides self-emulsifying formulations comprising glyceryl monooleate at 9 vol% to 19 vol%, polyoxyethylene hydroxystearate at 51 vol% to 61 vol%, and olive oil at 17.5 vol% to 27.5 vol% based on the whole formulation (excluding the volume of the drug) and a poorly membrane permeable drug. The formulation may further comprise oleic acid, and the contained oleic acid is preferably 2.5 vol% to 12.5 vol% based on the whole formulation (excluding the volume of the drug).

In a non-limiting embodiment, the ratio of the total volume obtained by adding the volume of the lipophilic surfactant comprising oleate ester or oleylether as a moiety and the volume of the hydrophilic surfactant comprising oleate ester or oleylether as a structure, to the volume of the oily component (surfactant:oily component) in a self-emulsifying formulation in the present invention is preferably 100:1, 90:1, 80:1, 70:1, 60:1, 50:1, or 40:1, and more preferably 30:1, 20:1, 10:1, 5:1, or 3.5:1.

In a non-limiting embodiment, the ratio of the volume of the hydrophilic surfactant comprising oleate ester or oleylether as a moiety to the volume of the lipophilic surfactant comprising oleate ester or oleylether as a moiety (hydrophilic:lipophilic) in a self-emulsifying formulation in the present invention is preferably 1:1, 1.5:1, 2:1, 2.5:1, 3:1, 3.5:1, 4:1, 4.5:1, 5:1, 5.5:1, 6:1, 6.5:1, or 7:1.

While not limited to the following, those skilled in the art can appropriately add an auxiliary solvent, an emulsification adjuvant, a stabilizer, an antiseptic, a surfactant, an antioxidant, and such, in addition to the above-mentioned SEDDS formulations.

The expression "vol%" in the present invention compares the volume of additives to the volume of the whole formulation (excluding the volume of the drug).

In a non-limiting embodiment, a "lymphatically transported self-emulsifying formulation" of the present invention is not particularly limited as long as it is a self-emulsifying formulation which enables a poorly membrane-permeable drug present in the formulation to be absorbed through the lymphatic route when orally administered to a nonhuman animal or a human. For example, to check the lymphatic transport properties of a drug, methods known to those skilled in the art can be used, such as PK test by laboratory animal experiments through lymphatic cannulation and PK test on mice whose lymphatic absorption has been inhibited (European Journal of Pharmaceutical Sciences 24(4), 2005, 381-388). For example, lymphatically transported self-emulsifying formulations that increase the amount of lymphatically transported drug by at least 1.05-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, or 5-fold or more compared to a non-self-emulsifying formulation (for example, a solution formulation in which a drug is completely dissolved) are preferred, but are not limited thereto.

The "lymphatically transported self-emulsifying formulation" in the present invention can be applied either under fasting and well-fed conditions, and feeding conditions are not particularly limited.

In a non-limiting embodiment, the present invention provides lymphatically transported self-emulsifying formulations for increasing membrane permeability of a poorly membrane-permeable drug, the formulation comprising a surfactant comprising oleate ester or oleylether as a moiety, an oily component, and the poorly membrane-permeable drug.

In a non-limiting embodiment, the present invention provides lymphatically transported self-emulsifying formulations for increasing membrane permeability of a poorly membrane-permeable drug comprising a surfactant, an oily component comprising oleic acid as the main oil type component, and the poorly membrane-permeable drug.

In a non-limiting embodiment, the self-emulsifying formulation "for enhancing membrane permeability" of the present invention refers to a self-emulsifying formulation that can enhance the membrane permeability (increase membrane permeation rate and/or amount of membrane permeation of a drug) of a poorly membrane-permeable drug included in the formulation, compared to a control formulation, when orally administered to a nonhuman animal or a human.

For example, in an *in vitro* assay, a method for evaluating membrane permeability of a drug using the following known method can determine whether the membrane permeability of the drug is enhanced in the self-emulsifying formulation of the present invention than in a control formulation. For example, membrane permeability can be checked using known methods such as rat intestine methods, cultured cell (Caco-2, MDCK, HT-29, LLC-PK1, *etc.*) monolayer methods, immobilized artificial membrane chromatography, methods using distribution coefficients, ribosome membrane methods, or parallel artificial membrane permeation assay (PAMPA).

In a non-limiting embodiment, evaluation of membrane permeability of a drug in the self-emulsifying formulation of the present invention and in the control formulation can also be performed by adding to the cultured Caco-2 cells a control formulation and the self-emulsifying formulation of the present invention containing the poorly membrane-permeable drug and evaluating the Caco-2 membrane permeation of the drug included in the respective formulations.

For example, in *in vivo* assays, the determination can be carried out by evaluating the blood pharmacokinetics of a drug after oral administration of the self-emulsifying formulation of the present invention in mice whose transporters having functions of eliminating drugs present in the digestive tract such as P-gp and BCRP transporters are knocked out, and in normal mice (non-knockout mice).

Without wishing to be bound by a specific theory, it can be considered that the self-emulsifying formulations in the present invention allow the drug to escape from exocytosis through the above-mentioned transporters by enhancing simple diffusion of the poorly membrane-permeable drug included in the formulations at the cell membrane. In a non-limiting embodiment, the escape from the transporters may include a mechanism where the self-emulsifying formulations in the present invention inhibit the mechanism of exocytosis of drugs by the transporters, and the drug escapes from exocytosis. In an embodiment of the present invention, escaping of drug from exocytosis by transporters may, without meaning that the action of transporters are inhibited, solely refer to the case where the drug escapes from exocytosis by the transporters because of simple diffusion of the drug at the cell membrane being enhanced by the self-emulsifying formulations of the present invention.

The above-mentioned control drug formulation is not particularly limited as long as it is a non-self-emulsifying formulation in which the drug is completely dissolved in the formulation and for example, formulations based on DMSO/Cremophor EL are preferred.

In a non-limiting embodiment, the present invention provides self-emulsifying formulations, which comprise a surfactant comprising oleate ester or oleylether as a moiety, an oily component, and a poorly membrane-permeable drug having features (i) to (iii) below:

(i) logD (pH 7.4) value of the drug is 3.2 or greater;
(ii) Caco-2 Papp (cm/sec) value of the drug is 1.8E-6 or less; and
(iii) molecular weight of the drug is 500 or greater.

While not being limited to the following, the above-described criteria can be applied to the preferred values of LogD (pH 7.4), Caco-2 Papp (cm/sec), and a molecular weight of a drug. Furthermore, the above-described criteria can be applied also to the criterion for metabolic stability of a drug (CLh int (μL/min/mg protein), and such).

In a non-limiting embodiment, the present invention provides self-emulsifying formulations in which the aforementioned drug is a peptide compound comprising a cyclic portion, the compound having features (i) and/or (ii) below:

(i) the compound comprises a cyclic portion consisting of a total of 5 to 12 natural amino acid and amino acid analog residues and a total number of natural amino acid and amino acid analog residues is 9 to 13; and
(ii) the compound comprises at least two *N*-substituted amino acids, and comprises at least one amino acid that is not *N*-substituted.

While not being limited to the following, the above-described criteria are applied to the preferred number of amino acid residues (number of natural amino acid and amino acid analog residues)

In a non-limiting embodiment, the present invention provides lymphatically transported self-emulsifying formulations, which comprise a surfactant, an oily component, and a poorly membrane-permeable drug, wherein the total oleic acid content is 25 vol% to 75 vol% based on the whole formulation (excluding the volume of the drug).

In a non-limiting embodiment, the present invention provides lymphatically transported self-emulsifying formulations, which comprise a surfactant comprising oleate ester or oleylether as a moiety, an oily component, and a poorly membrane-permeable drug, wherein the total oleic acid content is 25 vol% to 75 vol% based on the whole formulation (excluding the volume of the drug).

In a non-limiting embodiment, the present invention provides lymphatically transported self-emulsifying formulations, which comprise a surfactant, an oily component which comprises oleic acid as the main oil type component, and a poorly membrane-permeable drug, wherein the total oleic acid content is 25 vol% to 75 vol% based on the whole formulation (excluding the volume of the drug).

Herein, while the total oleic acid content (including oleic acid itself, and esterified or etherified oleic acid) in the above-mentioned embodiment is not particularly limited, it refers to the summed content of the content of oleic acid added as oleic acid, the content of esterified and etherified oleic acid contained as a moiety in the surfactant, and the content of oleic acid included in the oily component, which are present in the whole formulation (excluding the drug). For example, olive oil contains approximately 11.6% palmitic acid, approximately 1.0% palmitoleic acid, approximately 3.1% stearic acid, approximately 75.0% oleic acid, and approximately 7.8% linoleic acid. In this case, the oleic acid content in olive oil can be determined to be 75%. For example, in the case of a self-emulsifying formulation comprising 10 vol% olive oil and 10 vol% oleic acid, the oleic acid content in the formulation can be determined to be 17.5 vol%.

In a non-limiting embodiment, the total oleic acid content is preferably 20 vol% to 80 vol% or 20 vol% to 75 vol%, or more preferably 25 vol% to 75 vol%, 25 vol% to 70 vol%, 30 vol% to 70 vol%, 30 vol% to 65 vol%, 35 vol% to 65 vol%, 35 vol% to 65 vol%, or 40 vol% to 60 vol% based on the whole formulation (excluding the volume of the drug).

In a non-limiting embodiment, the present invention provides self-emulsifying formulations for providing escape for a poorly membrane-permeable drug from exocytosis by transporters expressed in small intestinal epithelial cells, the formulation comprising a surfactant comprising oleate ester or oleylether as a moiety, an oily component, and the poorly membrane-permeable drug.

In a non-limiting embodiment, the present invention provides self-emulsifying formulations for providing escape for a poorly membrane-permeable drug from exocytosis by transporters expressed in small intestinal epithelial cells, the formulation comprising a surfactant, an oily component which comprises oleic acid as the main oil type component, and the poorly membrane-permeable drug.

In the above-mentioned embodiment, the transporters expressed in small intestinal epithelial cells mean efflux transporters present in the digestive tract. While not particularly limited as long as compounds are pumped out of the cells, examples may include transporters such as P-glycoprotein (P-gp), breast cancer resistance protein (BCRP), and multidrug resistance associated protein (MRP). Whether a drug becomes a substrate of the above-mentioned transporters and whether a drug escapes from drug exocytosis by transporters can be determined appropriately by those skilled in the art using known methods such as Caco-2 assay and methods using transporter knock-out mice. For example, by checking according to the method described in Example 6-1, it is possible to evaluate whether self-emulsifying formulations of the present invention allow escaping of a drug from exocytosis.

While not wishing to be bound by a particular theory, the phrase "providing escape for a drug from exocytosis" in the above-mentioned embodiment can include the meaning that exocytosis of the drug by the transporters is avoided as a result of enhancement of simple diffusion of the drug at a cell membrane, and also, for example, the meaning that exocytosis of the drug is avoided due to the inhibition of transporter functions. Furthermore, in an embodiment of the present invention, "providing escape for a drug from exocytosis" may, without meaning that the transporter functions are inhibited, solely refer to the case where exocytosis of the drug by the transporters is avoided through enhancement of simple diffusion of the drug at the cell membrane.

In a non-limiting embodiment, the present invention provides lymphatically transported self-emulsifying formulations for enhancing simple diffusion (passive diffusion) of a poorly membrane permeable drug in a small intestinal epithelial cell, the formulation comprising a surfactant comprising oleate ester or oleylether as a moiety, an oily component, and the poorly membrane-permeable drug.

In a non-limiting embodiment, the present invention provides lymphatically transported self-emulsifying formulations for enhancing simple diffusion (passive diffusion) of a poorly membrane permeable drug in a small intestinal epithelial cell, the formulation comprising a surfactant, an oily component which comprises oleic acid as the main oil type component, and the poorly membrane-permeable drug.

In the above-mentioned embodiment, whether simple diffusion (passive diffusion) of a drug is enhanced can be determined by using as an indicator whether the time taken to reach maximum blood concentration (Tmax) of the drug is shortened or the maximum blood concentration of the drug (Cmax) increased compared to that of a non-self-emulsifying formulation (for example, a solution formulation).

The maximum blood concentration (Cmax) indicates the maximum or peak concentration of a pharmaceutical agent observed after drug administration, and the time taken to reach maximum blood concentration (Tmax) indicates the time taken to reach maximum blood concentration (Cmax) after the drug is administered.

In a non-limiting embodiment, the present invention provides lymphatically transported self-emulsifying formulations for avoiding a first-pass effect on a poorly membrane-permeable drug, the formulation comprising a surfactant comprising oleate ester or oleylether as a moiety, an oily component, and the poorly membrane-permeable drug.

In a non-limiting embodiment, the present invention provides lymphatically transported self-emulsifying formulations for avoiding a first-pass effect on a poorly membrane-permeable drug, the formulation comprising a surfactant, an oily component which comprises oleic acid as the main oil type component, and the poorly membrane-permeable drug. Whether a first-pass effect on the drug is avoided can be evaluated by checking if the bioavailability yielded at an oral administration is enhanced compared to the hepatic extraction ratio determined from the ratio of CL to hepatic blood flow rate when the drug is administered intravenously.

In a non-limiting embodiment, the present invention provides the following formulations.

Lymphatically transported self-emulsifying formulations, which comprise a surfactant comprising oleate ester or oleylether as a moiety, an oily component, and a poorly membrane-permeable drug, wherein when time taken to reach maximum blood concentration (Tmax) is assayed for the drug in the plasma of a mammalian subject after the administration, the Tmax is lower than the Tmax for a non-self-emulsifying formulation of the same drug administered at the same dose.

Lymphatically transported self-emulsifying formulations, which comprise a surfactant, an oily component which comprises oleic acid as the main oil type component, and a poorly membrane-permeable drug, wherein when time taken to reach maximum blood concentration (Tmax) is assayed for the drug in the plasma of a mammalian subject after the administration, the Tmax is lower than the Tmax for a non-self-emulsifying formulation of the same drug administered at the same dose.

In the present invention, the mammalian subjects include rodents such as mice, rats, and hamsters, and non-rodents such as humans, dogs, monkeys, chimpanzees, rabbits, sheep, cattle, and pigs, but are not limited thereto.

Furthermore, in a non-limiting embodiment, the present invention provides the following formulations.

Lymphatically transported self-emulsifying formulations, which comprise a surfactant comprising oleate ester or oleylether as a moiety, an oily component, and a poorly membrane-permeable drug, wherein when time taken to reach maximum blood concentration (Tmax) is assayed for the drug in the plasma of a mammalian subject after the administration, the Tmax is 90% or less of the Tmax for a non-self-emulsifying formulation of the same drug administered at the same dose.

Lymphatically transported self-emulsifying formulations, which comprise a surfactant, an oily component which comprises oleic acid as the main oil type component, and a poorly membrane-permeable drug, wherein when time taken to reach maximum blood concentration (Tmax) is assayed for the drug in the plasma of a mammalian subject after the administration, the Tmax is 90% or less of the Tmax for a non-self-emulsifying formulation of the same drug administered at the same dose.

In a non-limiting embodiment, the Tmax for self-emulsifying formulations of the present invention is preferably 90% or less, 80% or less, 70% or less, 60% or less, 50% or less, 40% or less, or 30% or less of the Tmax for a non-self-emulsifying formulation of the same drug administered at the same dose.

Furthermore, in a non-limiting embodiment, the present invention provides the following formulations.

Lymphatically transported self-emulsifying formulations, which comprise a surfactant comprising oleate ester or oleylether as a moiety, an oily component, and a poorly membrane-permeable drug, wherein when amount of lymphatic transport of the drug is assayed in the plasma of a mammalian subject after the administration, the amount of lymphatic transport is higher than the amount of lymphatic transport for a non-self-emulsifying formulation of the same drug administered at the same dose.

Lymphatically transported self-emulsifying formulations, which comprise a surfactant, an oily component which

comprises oleic acid as the main oil type component, and a poorly membrane-permeable drug, wherein when amount of lymphatic transport of the drug is assayed in the plasma of a mammalian subject after the administration, the amount of lymphatic transport is higher than the amount of lymphatic transport for a non-self-emulsifying formulation of the same drug administered at the same dose.

The amount of lymphatically transported drug can be measured using methods known to those skilled in the art, such as a PK test on mice whose lymphatic absorption has been inhibited (European Journal of Pharmaceutical Sciences 24(4), 2005, 381-388). For example, lymphatically transported self-emulsifying formulations that yield increase in the amount of lymphatically transported drug by at least 1.05-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2-fold or more, 3-fold or more, 4-fold or more, or 5-fold or more compared to a non-self-emulsifying formulation (for example, a solution drug formulation in which a drug is completely dissolved) are preferred, but are not limited thereto.

Furthermore, in a non-limiting embodiment, the present invention provides the following formulations.

Lymphatically transported self-emulsifying formulations, which comprise a surfactant comprising oleate ester or oleylether as a moiety, an oily component, and a poorly membrane-permeable drug, wherein when bioavailability of the drug is assayed in the plasma of a mammalian subject after the administration, the bioavailability is higher than the bioavailability for a non-self-emulsifying formulation of the same drug administered at the same dose.

Lymphatically transported self-emulsifying formulations, which comprise a surfactant, an oily component which comprises oleic acid as the main oil type component, and a poorly membrane-permeable drug, wherein when bioavailability of the drug is assayed in the plasma of a mammalian subject after the administration, the bioavailability is higher than the bioavailability for a non-self-emulsifying formulation of the same drug administered at the same dose.

Bioavailability can be evaluated by comparing AUC (area under the blood drug concentration-time curve) after oral administration of the drug to the AUC after parenteral administration of the drug.

In a non-limiting embodiment, the present invention also provides the following formulations.

Lymphatically transported self-emulsifying formulations, which comprise a surfactant comprising oleate ester or oleylether as a moiety, an oily component, and a poorly membrane-permeable drug, wherein when subjected to *in vitro* assay for membrane permeability of the drug using cultured cells, the membrane permeability is higher than the membrane permeability for a non-self-emulsifying formulation of the same drug.

Lymphatically transported self-emulsifying formulations, which comprise a surfactant, an oily component which comprises oleic acid as the main oil type component, and a poorly membrane-permeable drug, wherein when subjected to *in vitro* assay for membrane permeability of the drug using cultured cells, the membrane permeability is higher than the membrane permeability for a non-self-emulsifying formulation of the same drug.

Cell membrane permeability of peptide compounds

Herein, "cell membrane permeability" is sometimes referred to as "membrane permeability". Methods that use human colon cancer cell line Caco-2 cells have been reported as methods for measuring the membrane permeability of drugs and such (this is also referred to as "conventional methods". Artursson, P., 2001. Adv Drug Deliv Rev 46, 27-43; Mason, A.K., 2013. Drug Metab Dispos 41, 1347-1366; Polli, J.W., 2001. J Pharmacol Exp Ther 299, 620-628; and Sun, H. 2008. Expert Opin Drug Metab Toxicol 4, 395-411). These methods are methods of measuring membrane permeability of a substance to be measured (also called a "test substance") based on the membrane permeability coefficient ($P_{app}$) calculated from the amount of test substance which transferred to the basement membrane side (Basal side) after adding the test substance to the luminal side (Apical side) of the cultured Caco-2 cell layer (see Fig. 17). The permeability of Caco-2 cells evaluated by such an experiment has been found to be correlated with oral absorptivity in humans, and oral absorptivity in humans can be predicted from this correlation. Since Caco-2 cells form a monolayer membrane when cultured under specified conditions, and permeability of a drug to such a monolayer membrane shows good correlation with human oral absorptivity, it is widely used as a method for evaluating oral absorptivity *in vitro.* However, as described below, accurately measuring membrane permeability of highly lipid-soluble drugs and such is difficult using such conventional methods.

In a membrane permeability test using Caco-2 cells, as a prerequisite in calculating the membrane permeability coefficient ($P_{app}$) using Equation 1 below, it is assumed that the intracellular distribution of the drug can be ignored, that the drug concentration on the Basal side increases linearly, that a drug once permeates does not go back into the cell (*i.e.,* a sink condition), that change in concentration on the Apical side is small, that intracellular accumulation of the drug is not taken into account, and such (Bhoopathy, S., et al., 2014. Methods Mol Biol 1113, 229-252; Knipp, G.T., et al., 1997. J Pharm Sci 86, 1105-1110; Korzekwa, K.R., et al., 2012. Drug Metab Dispos 40, 865-876; and Sun, H., et al., 2008. Drug Metab Dispos 36, 102-123).
[Equation 1]

$$P_{app} = \frac{1}{C_1(0) \times S} \times \frac{dQ}{dt} \qquad \text{(Equation 1)}$$

dQ/dt is the permeation rate of the drug (the amount of drug that appears on the Basal side per unit time), S is the surface area of whole cells, and $C_1(0)$ is the concentration of the drug added to the Apical side

However, it has been reported that there are compounds for which the prerequisites in calculating $P_{app}$ using the above-mentioned Equation 1 are not applicable. For example, Ozeki *et al.* reported that they conducted membrane permeability tests using Caco-2 cells on drugs that permeate the membrane by passive diffusion (atenolol, metoprolol, and propranolol) and on P-glycoprotein (P-gp) substrates (digoxin, cyclosporine, and verapamil), and as a result of evaluating the changes in concentrations on the Apical side, inside the cell, and on the Basal side, it was found that for metoprolol and propranolol, 120 minutes of incubation caused decrease in the concentrations on the Apical side by approximately 20% and by approximately 40%, respectively (Ozeki K., et al., 2015, Int J Pharm. 495, 963-971).

That is, a large change in concentration on the Apical side is suggested. In addition, regarding the drugs propranolol, cyclosporine, and verapamil, approximately 8% of the added drugs were distributed in the cells after completion of incubation, suggesting intracellular accumulation of the drugs. Furthermore, a lagtime (intersection between the linear approximation of the linear region and the x axis) was present in the change in the concentration of cyclosporine on the Basal side (Ozeki K., et al., 2015, Int J Pharm. 495, 963-971), and the time until the linear portion starts (three times the lagtime) was over 300 minutes in both the test where a drug was added to the Apical side and sampling was performed from the Basal side (AtoB) and the test where a drug was added to the Basal side and sampling was performed from the Apical side (BtoA); therefore, this revealed that a linear region does not exist in the 120-minute incubation time. More specifically, this suggests that drug concentration on the Basal side does not increase linearly. $P_{app}$ calculated using Equation 1 from only the Basal-side concentration after the 120-minute incubation in the AtoB test gave a five-fold underestimated value compared to $P_{app}$ calculated using an optimized value from a model that adjusts for the intracellular distribution. It is considered that the cause for this is the strong cyclosporine binding to the intracellular matrix, which leads to delayed appearance of cyclosporine on the Basal side (Ozeki K., et al., 2015, Int J Pharm. 495, 963-971). In addition, it has been reported that for a compound whose $P_{app}$ value is $2.5 \times 10^{-5}$ cm/sec, the linear region ends within the 120-minute incubation time (also referred to as "plateau"). Furthermore, since a drug that has a large ClogP binds strongly to the intracellular matrix, start of the linear region is delayed, and accordingly, accurately evaluating $P_{app}$ of a highly lipid soluble drug is suggested to be difficult using conventional methods (Fig. 18).

Accordingly, the present inventors developed methods which improve the conventional method to measure the membrane permeability of peptide compounds, particularly cyclic peptide compounds. As shown in the Examples, use of this improved method, more specifically a method for measuring membrane permeability in the present disclosure, has enabled accurate measurement of membrane permeability of peptide compounds. Note, however, that test substances that can be measured by this improved method are not limited to peptide compounds.

In conventional methods, membrane permeability is measured without conducting a pre-incubating step under conditions where the test substance is mixed. Although conventional methods sometimes do involve incubation performed for a short period of time before membrane permeability measurements for buffer conditioning, this is not incubation under conditions where the test substance is mixed, and is different from the pre-incubation in the present disclosure.

In a non-limiting embodiment, the methods for measuring membrane permeability in the present disclosure (also referred to as the "measurement method in the present disclosure") comprise the step of pre-incubating Caco-2 cells in the mixed presence with a test substance. In the measurement methods in the present disclosure, "pre-incubation" and "pre-incubate (pre-incubating)" refer to incubating Caco-2 cells in the mixed presence with a test substance, prior to the step of measuring membrane permeability. Herein, "(pre)incubating Caco-2 cells in the mixed presence with a test substance" is not particularly limited as long as the state in which Caco-2 cells and a test substance co-exist occurs during the (pre)incubation, and for example, the (pre)incubation may be carried out by mixing Caco-2 cells and a test substance in a medium, and then starting the incubation. Alternatively, it may refer to placing a solution containing the test substance so that it contacts with one side of a Caco-2 cell layer, placing a solution not containing the test substance so that it contacts with the other side of the Caco-2 cell layer, and incubating for a predetermined period of time. Herein, at the start of the pre-incubation, the test substance is added to the solution on the Apical side and the test substance is not added to the solution on the Basal side, unless particularly stated otherwise.

In a non-limiting embodiment of the measurement methods in the present disclosure, during the pre-incubation, incubation can be carried out by placing a solution containing the test substance on the Apical side of the Caco-2 cell layer, or on its opposite side. The pre-incubation time in the measurement methods in the present disclosure is not particularly limited, but examples include 2 hours or more, 4 hours or more, 6 hours or more, 8 hours or more, 12 hours or more, 18 hours or more, 20 hours or more, and 24 hours or more. The upper limit of the pre-incubation time is not limited as long as it does not affect the membrane permeability measurements, but a length of time that does not cause

damage on the Caco-2 cells is preferred, and examples include 72 hours or less, 48 hours or less, 36 hours or less, 30 hours or less, 26 hours or less, or 24 hours or less.

Herein, "pre-incubation solution" refers to a solution which is made to contact with Caco-2 cells during the pre-incubation. While the pre-incubation solution of the measurement method in the present disclosure is not particularly limited, use of a medium is preferred from the viewpoint of not causing damage on Caco-2 cells by the pre-incubation. More specifically, pre-incubation in the present disclosure can be performed by contacting Caco-2 cells with a medium. Herein, "not cause(causing) damage on Caco-2 cells" can be checked by measuring the transepithelial electrical resistance (TEER) of Caco-2 cells. Herein, if the TEER value at the time of measurement is 70% or higher, or preferably 80% or higher compared to the initial value before the pre-incubation, it is determined that Caco-2 cells are not damaged at the time of measurement. TEER can be measured by methods known to those skilled in the art.

In a non-limiting embodiment, in the pre-incubation of the measurement methods in the present disclosure, a medium may be used as the pre-incubation solution. In one embodiment, in the pre-incubation in the present disclosure, the pre-incubation solution on the Apical side may be a medium. In one embodiment, as the pre-incubation solution, the medium may be used only on the Apical side or the Basal side, and while the medium may be used on both sides, the medium is preferably used at least on the Apical side. In one embodiment, it is preferred that the test substance is included in the pre-incubation solution on the Apical side of the Caco-2 cells. Herein, the medium is not particularly limited, but media that do not cause damage on Caco-2 cells are preferred, cell culture media are more preferred, and non-essential amino acid-containing media are even more preferred. Specific examples of the media include Dulbecco's modified Eagle's medium (DMEM), MEM, and RPMI 1640 medium, and among them, DMEM is preferred. In one embodiment, other components may be added to the media, and while the components that can be added are not particularly limited, examples include organic solvents and solubilizing agents, and specific examples include fasted state simulated intestinal and stomach fluids (FaSSIF), dimethyl sulfoxide (DMSO), dimethyl acetamide (DMA), methanol, acetonitrile, surfactants, and Tween. Herein, the phrase such as "the medium is DMEM" does not exclude the case where other components are included in the medium. The medium and/or the added components on the Apical side and the Basal side may be the same or different. Preferred examples of the medium on the Apical side are a mixed medium of FaSSIF and DMEM, and preferred examples of the medium on the Basal side are DMEM. In a non-limiting embodiment, while the pH of the pre-incubation solution is not particularly limited, examples include pH 5.0 to pH 8.0. Examples of the pre-incubation temperature include 5°C to 45°C, preferably 20°C to 40°C, and more preferably 37°C.

The measurement methods in the present disclosure include the step of measuring membrane permeability. In this step, membrane permeability of a test substance can be measured by placing a solution containing the test substance so that it contacts with one side of a Caco-2 cell layer, placing a solution not containing the test substance so that it contacts with the other side of the Caco-2 cell layer, and after a predetermined period of time, measuring the amount of the test substance in the solution on said other side and/or the amount of the test substance in the solution on said one side.

The aforementioned "step of measuring membrane permeability" in the measurement methods of the present disclosure can be carried out according to conventional methods. In one embodiment, after the pre-incubation step, the pre-incubation solutions on the Apical side and the Basal side are removed by aspiration, a solution containing the test substance is added to the Apical side and a solution not containing the test substance is added to the Basal side, and membrane permeability can be measured. In the step of measuring membrane permeability, for example, membrane permeability can be measured by adding a mixed solution of FaSSIF and Hanks' Balanced Salt Solutions (HBSS) containing the test substance (containing 1% DMSO) (pH 6.0) to the Apical side and adding HBSS (4% BSA) (pH 7.4) to the Basal side, culturing under conditions of 37°C, measuring the amount of the test substance on the Basal side by LC/MS after a predetermined period of time, and calculating the membrane permeability coefficient from the amount of permeation.

The compounds (test substances) measured by the measurement method in the present disclosure are not particularly limited, but examples include highly lipid-soluble compounds for which accurate measurement of membrane permeability is difficult by conventional methods. Examples of highly lipid-soluble compounds include compounds having ClogP of 3 or higher, 4 or higher, 5 or higher, 6 or higher, or 8 or higher. Furthermore, examples of highly lipid-soluble compounds include compounds having ClogP/total aa of 0.8 or higher, 1.0 or higher, 1.1 or higher, 1.2 or higher, or 1.3 or higher. In a non-limiting embodiment, the test substances may be peptide compounds, and preferably cyclic peptide compounds.

In a non-limiting embodiment, by measuring the membrane permeability of a plurality of test substances by the measurement methods in the present disclosure, test substances having the desired membrane permeability can be selected from the plurality of test substances based on the membrane permeability data obtained from this measurement. More specifically, herein, methods of screening for test substances using the measurement methods in the present disclosure are disclosed.

In a non-limiting embodiment, test substances having membrane permeability at the level required for development as pharmaceuticals can be selected by methods of screening for test substances using the measurement

methods in the present disclosure. The criteria used in this case can be selected depending on the purpose of the screening, such as location of the target molecule in a living body or administration route, and examples of the membrane permeability coefficient ($P_{app}$) include 5.0 × $10^{-7}$ cm/sec or greater, 8.0 × $10^{-7}$ cm/sec or greater, 9.0 × $10^{-7}$ cm/sec or greater, 1.0 × $10^{-6}$ cm/sec or greater, and 3.0 × $10^{-6}$ cm/sec or greater. Herein, "$10^{-n}$" may be expressed as "E-n" (for example, 1.0 × $10^{-6}$ may be expressed as 1.0E-6 or 1.0E-06).

The measurement methods (improved methods) in the present disclosure enable appropriate evaluation of membrane permeability of highly lipid-soluble test substances which were difficult to evaluate by conventional methods. Therefore, highly lipid-soluble test substances can also be subjected to test substance screening using criteria similar to that used in conventional methods. For example, by using measurement methods in the present disclosure, $P_{app}$ of 1.0 × $10^{-6}$ cm/sec or greater can be set as a criterion for enabling development as oral drugs as in conventional methods (P. Arturssonand J. et al., 1991, Biochem Biophys Res Commun. 175, 880-885).

In a non-limiting embodiment, the test substances selected by the screening methods in the present disclosure may be peptide compounds, and are particularly preferably cyclic peptide compounds. In a non-limiting embodiment, after selecting a peptide compound having the desired features by the aforementioned screening method, a peptide compound having the desired features can be produced based on the amino acid sequence of the selected peptide compound.

The present disclosure also provides pre-incubation methods which are included in the above-mentioned measurement methods in the present disclosure. More specifically, in one aspect, the present disclosure provides pre-incubation methods for membrane permeability measurements. The description, examples, preferred range, embodiments, and such of the pre-incubation methods in the present disclosure are as described above.

The membrane permeability of peptide compounds in the present disclosure can be measured by the methods for measuring membrane permeability in the present disclosure. More specifically, membrane permeability can be measured by the following method. A mixed medium of FaSSIF and DMEM containing 1% DMSO is added as the medium on the Apical side, DMEM is added as the medium on the Basal side, and under conditions of 5% $CO_2$, 37°C, and 80 rpm, Caco-2 cells and the peptide compounds are pre-incubated for 20 hours to 24 hours. Thereafter, the media on the Apical side and the Basal side are removed by aspiration and washed, a peptide compound is added to the FaSSIF/HBSS buffer (pH 6.0) containing 1% DMSO (1% DMSO) on the Apical side, HBSS buffer (pH 7.4) containing 4% BSA is added to the Basal side, and membrane permeability measurement is initiated. Each well is shaken under conditions of 5% $CO_2$, 37°C, and 80 rpm, and approximately 180 minutes after initiation, samples on the Basal side are collected. The amount of the peptide compound in the samples is measured by LC/MS, and the membrane permeability coefficient is calculated from the amount of permeation. When calculating the membrane permeability coefficient ($P_{app}$) from the amount of permeation, the above described Equation 1 can be used.

In a non-limiting embodiment, when measuring the membrane permeability of a peptide compound in the present disclosure, a peptide compound that does not carry a nucleic acid moiety serving as a template for the peptide moiety, is preferably used as the test substance. In one embodiment, after performing panning on peptide-nucleic acid complexes, peptide-nucleic acid complexes which can bind specifically to the target molecule are selected, and peptide compounds can be synthesized based on the nucleotide sequences of their nucleic acid moiety. In a preferred example, membrane permeability measurements are performed using peptide compounds synthesized as described as the test substances. In one embodiment, derivatives of peptide compounds encoded by the aforementioned nucleotide sequences can be synthesized, and these can be used as test substance to measure membrane permeability, or derivatives can be synthesized from test substances based on membrane permeability results, and membrane permeability can be measured for those derivatives.

In a non-limiting embodiment, $P_{app}$ of the cyclic peptide compound in the present disclosure is preferably 5.0 × $10^{-7}$ cm/sec or greater or 8.0 × $10^{-7}$ cm/sec or greater, more preferably 9.0 × $10^{-7}$ cm/sec or greater, even more preferably 1.0 × $10^{-6}$ cm/sec or greater, and particularly preferably 3.0 × $10^{-6}$ cm/sec or greater.

Herein, unless otherwise stated particularly, "membrane permeability coefficient ($P_{app}$)" means a value measured using the measurement method (improved method) for membrane permeability in the present disclosure, and more specifically means a value measured using the improved method for membrane permeability test described in the Examples.

All prior art documents cited herein are incorporated by reference into this description.

## Examples

Herein below, suitable specific embodiments of the present invention will be described with reference to the Examples, but it is not to be construed as being limited thereto.

[Example 1] Synthesis and physical properties of cyclic peptides

(Example 1-1) Cyclic peptide synthesis

Cyclic peptides used in the Examples, which have the amino acid sequences shown in Table 1, were synthesized using the same technique as the method described in PTL (WO 2013/100132), and the final products were obtained as dry substances. Here, the site indicated on the rightmost column in Table 1 forms the C terminus. Here, the notation "pip" means that piperidinamide was formed by formation of an amide bond between the C-terminal carboxylic acid and piperidine. Abbreviations for the amino acids are described in Table 2. The structural formulae of compounds (1) to (6) are shown in Table 3.

[Table 1]

| Peptide sequences of cyclic peptides | | | | | | | | | | | | |
|---|---|---|---|---|---|---|---|---|---|---|---|---|
| Compound | Sequence | | | | | | | | | | | |
| Compound (1) | Ala | Gly | MeLeu | MeIle | MeAla | MePhe | Thr | MePhe | MeLeu | Pro | Asp | pip |
| Compound (2) | | MeAla | MePhe | MeGly | MeLeu | Thr | MeAla | MeLeu | MeIle | Ser(tBu) | Asp | pip |
| Compound (3) | | D-Val | MeLeu | MePhe | MeAla | Thr | MeGly | MeLeu | Ser(tBu) | MeIle | Asp | pip |
| Compound (4) | | | MeAla | Thr | MeAla | Leu | MePhe | MePhe | Leu | MeLeu | Asp | pip |
| Compound (5) | | | D-Val | MePhe | MeLeu | Thr | MeGly | MeLeu | Ser(tBu) | MeIle | Asp | pip |
| Compound (6) | | | | g-MeAbu | MePhe | Thr | MeGly | Ser(tBu) | MeLeu | MeLeu | Asp | pip |

[Table 2]

| D escriptions of amino acid abbreviations | |
|---|---|
| Abbreviation | IUPAC Name |
| Ala | (S)-2-aminopropanoic acid |
| Gly | 2-aminoacetic acid |
| MeLeu | (S)-4-methyl-2-(methylamino)pentanoic acid |
| Melle | (2S,3S)-3-methyl-2-(methylamino)pentanoic acid |
| MeAla | (S)-2-(methylamino)propanoic acid |
| MePhe | (S)-2-(methylamino)-3-phenylpropanoic acid |
| Thr | (2S,3R)-2-amino-3-hydroxybutanoic acid |
| Pro | (S)-pyrrolidine-2-carboxylic acid |
| Asp | (S)-2-aminosuccinic acid |
| Leu | (S)-2-amino-4-methylpentanoic acid |
| MeGly | 2-(methylamino)acetic acid |
| Ser(tBu) | (2R)-2-amino-3-[(2-methyl-2-propanyl)oxy]propanoate |
| D-Val | (R)-2-amino-3-methylbutyric acid |
| g-MeAbu | 4-(methylamino)butyric acid |

[Table 3]

| Structural Formulae of Compounds (1) to (6) | |
|---|---|
| Compounds | |
| Compound (1) | 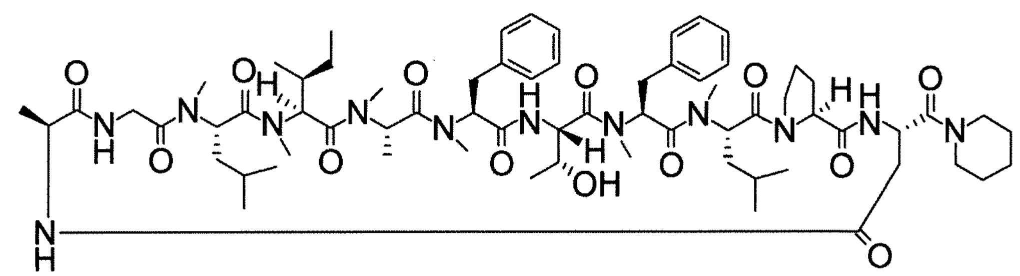 |

(continued)

| Structural Formulae of Compounds (1) to (6) | |
|---|---|
| Compounds | |
| Compound (2) | 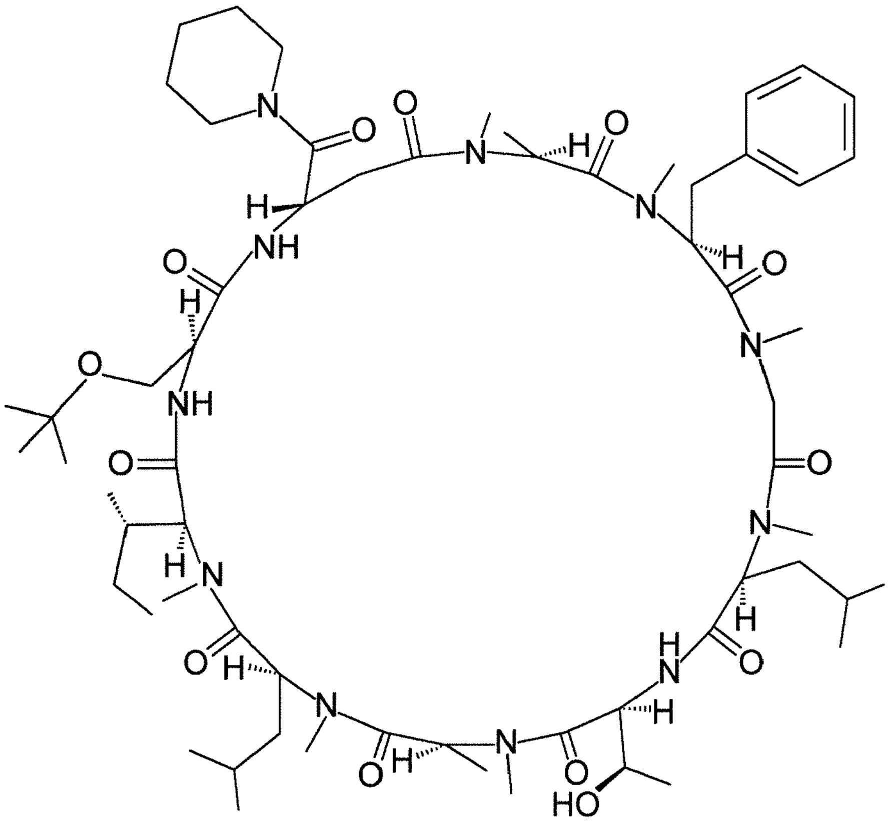 |
| Compound (3) | 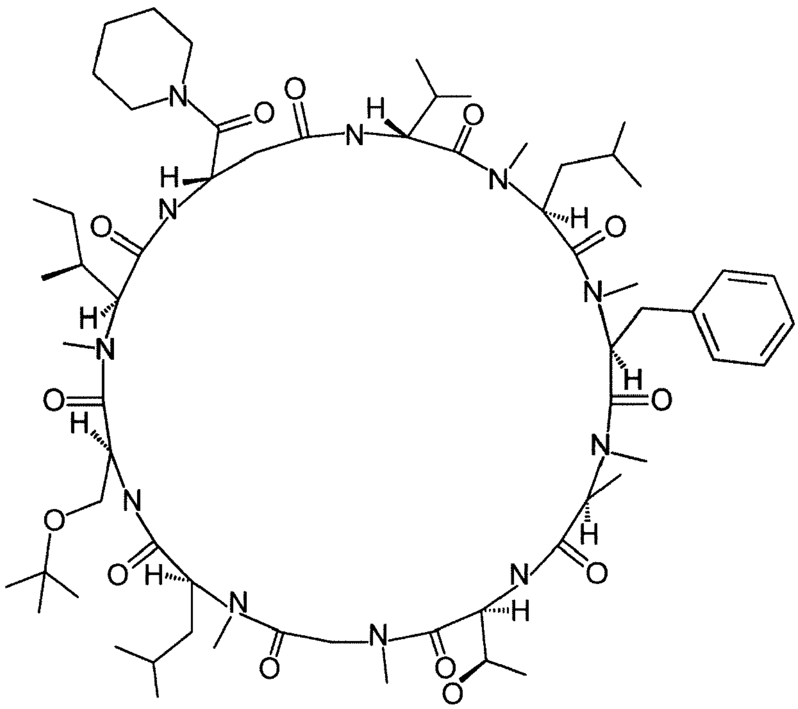 |

(continued)

| Structural Formulae of Compounds (1) to (6) | |
|---|---|
| Compounds | |
| Compound (4) | 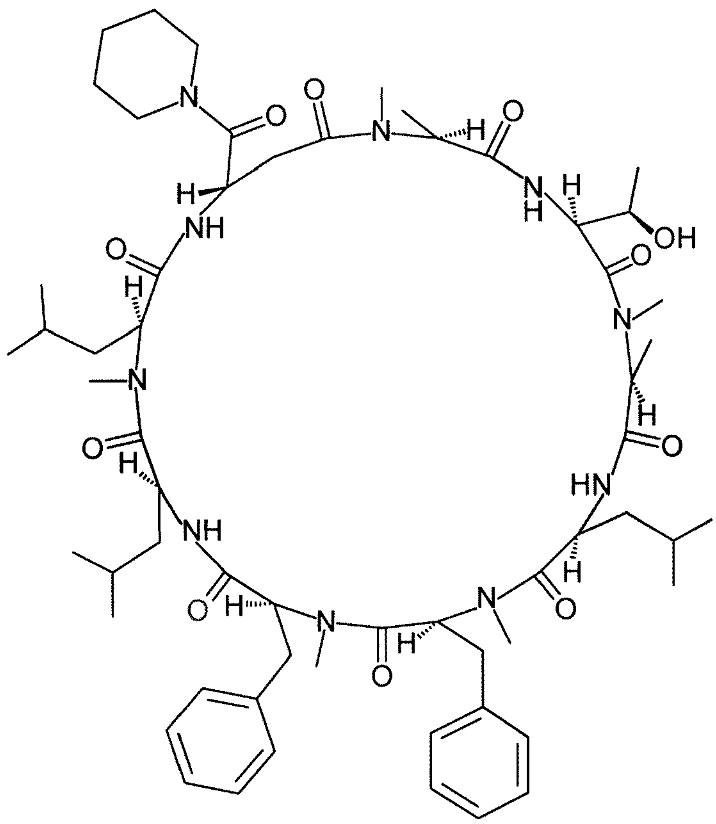 |
| Compound (5) | 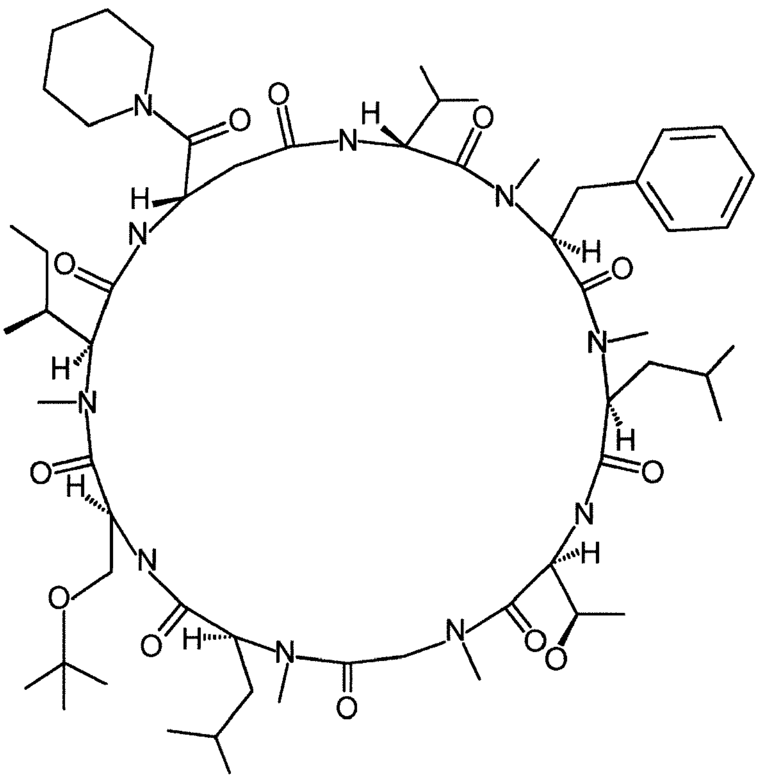 |

(continued)

| Structural Formulae of Compounds (1) to (6) | |
|---|---|
| Compounds | |
| Compound (6) | 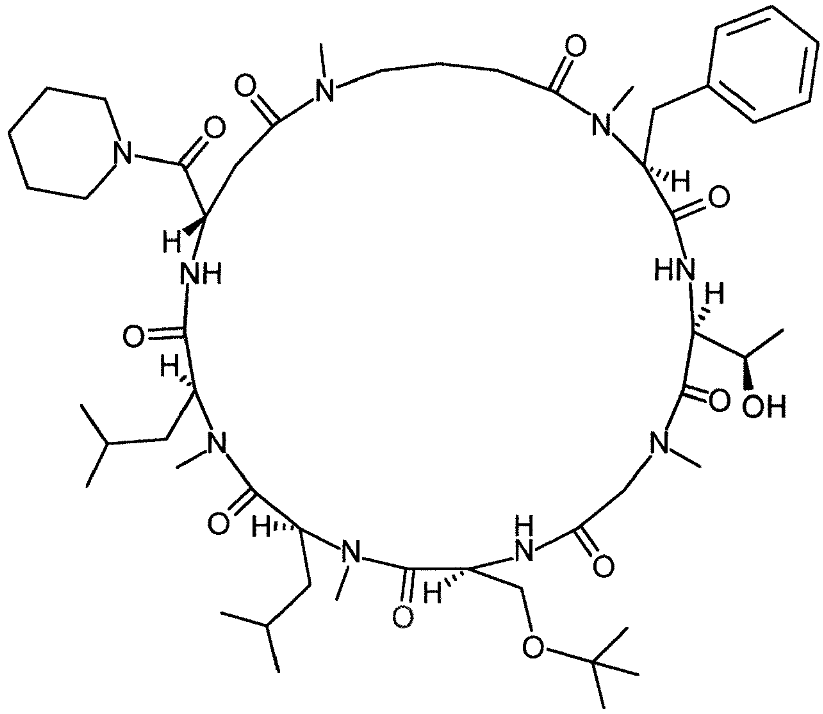 |

(Example 1-2) Evaluation of physical properties of the cyclic peptides

(Example 1-2-1) Caco-2 membrane permeability evaluation

After Caco-2 cells were cultured on a 96-well transwell for three weeks, DMEM + FaSSIF (1% DMSO) + the compound was added to the Apical side and DMEM was added to the Basal side, and this was pre-incubated under 5% $CO_2$ at 37°C and 80 rpm for 20 hours to 24 hours. After pre-incubation, the pre-incubation solutions on the Apical and Basal sides were removed by aspiration and washed, a permeability test was initiated by adding FaSSIF/HBSS buffer (pH 6.0) containing the compound to the Apical side and HBSS buffer (pH 7.4) containing 4% BSA to the Basal side. Each well was shaken under 5% $CO_2$, at 37°C and 80 rpm. 180 minutes after initiation, samples on the Basal side were collected, and the amount of permeation was determined by LC/MS. The permeability coefficient was calculated from the amount of permeation.

(Example 1-2-2) Evaluation of LogD (pH 7.4)

Lipophilicities of drugs to be targeted for the self-emulsifying formulations in the present invention were evaluated (LogD (pH 7.4) measurements). A two-phase separated solution of *n*-octanol and a pH 7.4 buffer was prepared, a DMSO solution of the drug was added to the prepared two-phase-separated solution. After shaking (room temperature, 1800 rpm, two hours), the solution was centrifuged (3000 rpm, ten minutes), only the buffer fractions were collected, and drug concentrations in the buffer fractions were measured by LC/MS. LogD (pH 7.4) was calculated from the measured drug concentrations.

[Table 4]

| Physical Property of Cyclic Peptides | | | |
|---|---|---|---|
| Compounds | Caco-2 Membrane Permeability (cm/s) | LogD (pH 7.4) | Compound MW |
| Compound (1) | $2.62\times10^{-7}$ | 5. 19 | 1297.6 |

(continued)

| Physical Property of Cyclic Peptides | | | |
|---|---|---|---|
| Compounds | Caco-2 Membrane Permeability (cm/s) | LogD (pH 7.4) | Compound MW |
| Compound (2) | $1.15\times10^{-7}$ | 4. 43 | 1210.6 |
| Compound (3) | $2.49\times10^{-7}$ | 4. 18 | 1224.6 |
| Compound (4) | $4.14\times10^{6}$ | 5. 17 | 1129.5 |
| Compound (5) | $1.85\times10^{-6}$ | 4. 31 | 1139.5 |
| Compound (6) | $3.40\times10^{-8}$ | 3. 19 | 1012.3 |

[Example 2] Preparation of SEDDS (1)

Olive oil (manufactured by Sigma-Aldrich Co.), oleic acid (EXTRA OLEIN 99, manufactured by NOF Corp.), Solutol HS-15 (manufactured by BASF; generic name: polyoxyethylene hydroxystearate), and Peceol (manufactured by Gattefosse Co.; generic name: glyceryl monooleate) were mixed at the ratio described in Table 5, and the mixture was stirred at 37°C for six hours or more to prepare Composition (1).

SEDDS (1) was prepared by adding Composition (1) to Compound (1) at 10 mg/mL, and stirring this at 37°C for six hours or more to completely dissolve Compound (1).

[Table 5]

| Constituent of Composition (1) | |
|---|---|
| Components | Volume (%) |
| Olive Oil | 22. 5 |
| Oleic Acid | 7. 5 |
| Solutol HS-15 | 56. 0 |
| Peceol | 14. 0 |

[Example 3]

(Example 3-1) Preparation of SEDDS (2)

Composition (2) was prepared by performing the same method as that of Example 2, except that the mixing proportions were changed to the proportions shown in Table 6. Thereafter, SEDDS (2) was prepared by adding Composition (2) to Compound (1) at 11.4 mg/mL, and stirring this at 37°C for six hours or more to completely dissolve Compound (1).

[Table 6]

| Constituent of Composition (2) | |
|---|---|
| Components | Volume (%) |
| Olive Oil | 15. 0 |
| Oleic Acid | 5. 0 |
| Solutol HS-15 | 64. 0 |
| Peceol | 16. 0 |

(Example 3-2) Preparation of SEDDS (3)

Composition (3) was prepared by performing the same method as that of Example 3-1, except that Peceol was changed to Nikkol SO-30V (manufactured by Nikko Chemicals Co., Ltd.; generic name: sorbitan trioleate). Thereafter,

SEDDS (3) was prepared by adding Composition (3) to Compound (1) at 10 mg/mL, and stirring this at 37°C for six hours or more to completely dissolve Compound (1).

(Example 3-3) Preparation of SEDDS (4)

Composition (4) was prepared by performing the same method as that of Example 2, except that the mixing proportions were changed to those shown in Table 7. Thereafter, SEDDS (4) was prepared by adding Composition (4) to Compound (1) at 10 mg/mL, and stirring this at 37°C for six hours or more to completely dissolve Compound (1).

[Table 7]

| Constituent of Composition (4) | |
|---|---|
| Components | Volume (%) |
| Olive Oil | 30. 0 |
| Solutol HS-15 | 56. 0 |
| Peceol | 14. 0 |

[Example 4] Preparation of SEDDS (5) to (31)

Compositions (5) to (31) were prepared by performing the same method as that of Example 2, except that olive oil was changed to the oils shown in Table 8. The following were used for the oil: almond oil / coconut oil / macadamia nut oil / avocado oil / safflower oil / soybean oil / linseed oil / castor oil / sunflower oil / cotton seed oil / corn oil / sesame oil (manufactured by Wako Pure Chemical Corp.), cacao butter (manufactured by MP biomedicals), rapeseed oil (manufactured by NACALAI TESQUE, INC.), palm oil / Tricaprylin / Triolein (manufactured by Sigma Aldrich Co.), high-oleic sunflower oil (Oleinrich: manufactured by SHOWA SANGYO Ltd.), high-oleic safflower oil (manufactured by Nisshin Oillio Group Ltd.), peanut oil (manufactured by Junsei Chemical Co., Ltd.), Tricaproin / Tricaprin / Trilinolein / Trierucin (manufactured by Tokyo Chemical Industry Co. Ltd.), Tripalmitolein / Trilinolenin / Trieicosenoin (manufactured by Larondan Inc.). Thereafter, SEDDS (5) to (31) were prepared by adding Compositions (5) to (31) to Compound (1) at 10 mg/mL, and stirring these at 37°C for six hours or more to completely dissolve Compound (1).

[Table 8]

| Oils used for SEDDS (5) to (31) | | | |
|---|---|---|---|
| Example No. | Composition No. | Formulation No. | Oil |
| 4-1 | Composition (5) | SEDDS (5) | Almond Oil |
| 4-2 | Composition (6) | SEDDS (6) | Coconut Oil |
| 4-3 | Composition (7) | SEDDS (7) | Cacao Oil |
| 4-4 | Composition (8) | SEDDS (8) | Macadamia Nut Oil |
| 4-5 | Composition (9) | SEDDS (9) | Avocado Oil |
| 4-6 | Composition (10) | SEDDS (10) | Safflower Oil |
| 4-7 | Composition (11) | SEDDS (11) | Soy Bean Oil |
| 4-8 | Composition (12) | SEDDS (12) | Flaxseed Oil |
| 4-9 | Composition (13) | SEDDS (13) | Rapeseed Oil |
| 4-10 | Composition (14) | SEDDS (14) | Castor Oil |
| 4-11 | Composition (15) | SEDDS (15) | Palm Oil |
| 4-12 | Composition (16) | SEDDS (16) | High-oleic Sunflower Oil |
| 4-13 | Composition (17) | SEDDS (17) | High-oleic Safflower Oil |
| 4-14 | Composition (18) | SEDDS (18) | Sunflower Seed Oil |
| 4-15 | Composition (19) | SEDDS (19) | Cottonseed Oil |

(continued)

| Oils used for SEDDS (5) to (31) | | | |
|---|---|---|---|
| Example No. | Composition No. | Formulation No. | Oil |
| 4-16 | Composition (20) | SEDDS (20) | Corn Oil |
| 4-17 | Composition (21) | SEDDS (21) | Sesame Oil |
| 4-18 | Composition (22) | SEDDS (22) | Peanut Oil |
| 4-19 | Composition (23) | SEDDS (23) | Tricaproin |
| 4-20 | Composition (24) | SEDDS (24) | Tricaprylin |
| 4-21 | Composition (25) | SEDDS (25) | Tricaprin |
| 4-22 | Composition (26) | SEDDS (26) | Tripalmitolein |
| 4-23 | Composition (27) | SEDDS (27) | Triolein |
| 4-24 | Composition (28) | SEDDS (28) | Trilinolein |
| 4-25 | Composition (29) | SEDDS (29) | Trilinolenin |
| 4-26 | Composition (30) | SEDDS (30) | Trieicosenoin |
| 4-27 | Composition (31) | SEDDS (31) | Trierucin |

[Comparative Example 1] Preparation of formulations for solution administration

Compounds (1) to (6) were dissolved in dimethyl sulfoxide (manufactured by Wako Pure Chemical Corp.) at 10 mg/mL. Cremophor EL (manufactured by Sigma Aldrich Co.; generic name: polyoxyethylene castor oil) was added such that the concentrations of the compounds become 5 mg/mL and the solutions were mixed by stirring. Furthermore, water for injection was added such that the concentrations of the compounds become 1 mg/mL to prepare the formulations for solution administration.

[Comparative Example 2] Preparation of SEDDS (32)

By referring to the formulation examples for a lymphatically transported self-emulsifying formulation described in WO 2002/102354, Pharm Res (2010), 27: 878-893, and such, Composition (32) was prepared as follows. Soybean oil (manufactured by Sigma-Aldrich Co.), Maisine 35-1 (manufactured by Gattefosse Corp.; generic name: glyceryl monolinolenate), and Cremophor EL (manufactured by Sigma Aldrich Co.; generic name: polyoxyethylene castor oil) were mixed at the proportion shown in Table 9. After the mixture was stirred at 37°C for one hour, 10 vol% of ethanol was added and then stirred.

SEDDS (32) was prepared by adding Composition (32) to Compound (1) at 10 mg/mL, and stirring this at 37°C for six hours or more to completely dissolve Compound (1).

[Table 9]

| Constituent of Composition (32) | |
|---|---|
| Components | Volume (%) |
| Soy Bean Oil | 34. 7 |
| Maisine 35-1 | 33. 7 |
| Cremophor EL | 31. 6 |

[Comparative Example 3]

(Comparative Example 3-1) Preparation of SEDDS (33)

Composition (33) was prepared by performing the same method as that of Example 3-2, except that the mixing proportions were changed to those shown in Table 10. Thereafter, SEDDS (33) was prepared by adding Composition

(33) to Compound (1) at 11.3 mg/mL, and stirring this at 37°C for six hours or more to completely dissolve Compound (1).

[Table 10]

| Constituent of Composition (33) | |
|---|---|
| Components | Volume (%) |
| Olive Oil | 7. 5 |
| Oleic Acid | 2. 5 |
| Solutol HS-15 | 72. 0 |
| Nikkol SO-30V | 18. 0 |

(Comparative Example 3-2) Preparation of SEDDS (34)

Composition (34) having the constituent shown in Table 11 was prepared by performing the same method as that of Example 3-2, except that olive oil and oleic acid were not used. Thereafter, SEDDS (34) was prepared by adding Composition (34) to Compound (1) at 12.5 mg/mL, and stirring this at 37°C for six hours or more to completely dissolve Compound (1).

[Table 11]

| Constituent of Composition (34) | |
|---|---|
| Components | Volume (%) |
| Solutol HS-15 | 80. 0 |
| Nikkol SO-30V | 20. 0 |

[Example 5] Preparation of SEDDS formulations

(Example 5-1) Preparation of SEDDS (35)

SEDDS (35) was prepared by performing the same method as that of Example 2, except that the dissolved compound was changed from Compound (1) to Compound (2).

(Example 5-2) Preparation of SEDDS (36)

SEDDS (36) was prepared by performing the same method as that of Example 2, except that the dissolved compound was changed from Compound (1) to Compound (3).

[Comparative Example 4] Preparation of SEDDS formulations

(Comparative Example 4-1) Preparation of SEDDS (37)

SEDDS (37) was prepared by performing the same method as that of Example 2, except that the dissolved compound was changed from Compound (1) to Compound (4).

(Comparative Example 4-2) Preparation of SEDDS (38)

SEDDS (38) was prepared by performing the same method as that of Example 2, except that the dissolved compound was changed from Compound (1) to Compound (5).

(Comparative Example 4-3) Preparation of SEDDS (39)

SEDDS (39) was prepared by performing the same method as that of Example 2, except that the dissolved compound was changed from Compound (1) to Compound (6).

[Example 6] Mouse PK study

(Example 6-1) Preparation of SEDDS administration formulation

For evaluation of SEDDS formulations prepared in Examples 2 to 5 and Comparative Examples 2 to 4, water for injection was added to SEDDS (1) to (39) at the proportions shown in Table 12. Then, the mixtures were stirred at room temperature for 15 minutes or more to prepare SEDDS administration formulations in which the concentration of the compound was 1 mg/mL.

[Table 12]

| Added ratios of water for injection | |
|---|---|
| Formulation No. | SEDDS/Water for injection (v/v%) |
| SEDDS (1), (3) to (32), (35) to (39) | 10/90 |
| SEDDS (2) | 8. 7/91. 3 |
| SEDDS (33) | 8. 9/91. 1 |
| SEDDS (34) | 8/92 |

(Example 6-2) Mouse PK study

The solution administration formulation prepared in Comparative Example 1 and SEDDS formulations prepared in Examples 2 to 5 and Comparative Examples 2 to 4 were evaluated for blood kinetics after oral administration in mice. The compounds were orally administered at a dose of 10 mg/kg to male mice (C57BL/6J, six-week-old, provided by Beijing HFK Bioscience: three mice per group). Blood until 24 hours after the administration was collected over time from the dorsal foot vein using a hematocrit tube treated with heparin as an anticoagulant. Plasma was separated from the blood by centrifugation and subjected to deproteinization treatment with acetonitrile, after which the plasma concentration was measured using an LC/MS/MS instrument (API4000, manufactured by AB SCIEX). The blood concentration over time of each formulation is shown in Figs. 1 to 13. Pharmacokinetic parameters were calculated from the resulting plasma concentration over time by non-compartment analysis using analysis software Phoenix WinNonlin 7.0 (manufactured by Certara L. P.), and the results are shown in Tables 14 and 15.

Definition of each parameter is shown below. The area under the plasma concentration-time curve (AUC; ng·h/mL), the highest plasma concentration (Cmax; ng/mL), time taken to reach the maximum plasma concentration (Tmax; h), and the relative bioavailability (rBA) after oral administration were calculated. When the plasma concentration was at or below the lower limit of quantification, the concentration was regarded as 0 ng/mL. For AUC, the value of the area from the time of administration to seven hours later was calculated, and it was converted based on the concentration of the compound in the actual administration solution so that the administered dose becomes 10 mg/kg. rBA was calculated as the ratio of AUC of a SEDDS formulation (AUCsedds) to the AUC of the solution administration formulation (AUCsol) (AUCsedds/AUCsol) when the same compound is administered. Regarding Compound (1), the AUCs of the groups to which a SEDDS formulation in the Examples was administered were all confirmed to be higher than the AUCs of the group administered with the solution administration formulation of Comparative Example 1 and of the group administered with the SEDDS formulation of Comparative Example 2, and shortening of Tmax and increase in Cmax were observed (Table 14). Accordingly, by using surfactants comprising oleic acid as a moiety such as Peceol and Nikkol SO-30V, poorly membrane permeable compounds were demonstrated to show higher absorbability in comparison to solution administration formulation, regardless of the type of oil (number of carbons in the fatty acid is C6 to C22).

Furthermore, Compounds (1) to (3) whose Caco-2 membrane permeability was $1.80 \times 10^{-6}$ or less and LogD was 3.2 or greater as indicated in Example 1-2, showed rBA of 1 or higher; whereas Compounds (4) to (6) whose Caco-2 membrane permeability or LogD were outside the above-mentioned range failed to achieve rBA of 1 or higher (Table 15). Accordingly, preferred range of physical properties (logD (pH 7.4), Caco-2 Papp (cm/sec)) of compounds was specified, for which improvement in absorbability at an oral administration is possible with the use of SEDDS formulation prescriptions in the present invention in comparison to when using solution administration formulations (Fig. 16).

[Table 14]

| Pharmacokinetics Parameters for Compound 1 Formulation | | | | | |
|---|---|---|---|---|---|
| Example No. | Formulation | AUC | Tmax | Cmax | rBA |
| Comparative Example 1 | Solution Administration Formulation | 314 | 1. 670 | 190 | - |
| Example 2 | SEDDS (1) | 1431 | 1. 000 | 1030 | 4. 56 |
| Example 3-1 | SEDDS (2) | 1852 | 0. 667 | 1070 | 5. 90 |
| Example 3-2 | SEDDS (3) | 1205 | 1. 000 | 822 | 3. 84 |
| Example 3-3 | SEDDS (4) | 1897 | 1. 000 | 1270 | 6. 04 |
| Example 4-1 | SEDDS (5) | 1377 | 0. 667 | 1410 | 4. 39 |
| Example 4-2 | SEDDS (6) | 1510 | 0. 500 | 1310 | 4. 81 |
| Example 4-3 | SEDDS (7) | 1743 | 0. 667 | 1510 | 5. 55 |
| Example 4-4 | SEDDS (8) | 1686 | 0. 500 | 1500 | 5. 37 |
| Example 4-5 | SEDDS (9) | 1580 | 0. 500 | 1160 | 5. 03 |
| Example 4-6 | SEDDS (10) | 2917 | 0. 500 | 2250 | 9. 29 |
| Example 4-7 | SEDDS (11) | 1733 | 0. 500 | 1640 | 5. 52 |
| Example 4-8 | SEDDS (12) | 2231 | 0. 667 | 1580 | 7. 10 |
| Example 4-9 | SEDDS (13) | 1703 | 0. 500 | 1530 | 5. 42 |
| Example 4-10 | SEDDS (14) | 2721 | 0. 833 | 1820 | 8. 67 |
| Example 4-11 | SEDDS (15) | 2375 | 0.667 | 1820 | 7. 56 |
| Example 4-12 | SEDDS (16) | 2108 | 0. 500 | 1570 | 6. 7 1 |
| Example 4-13 | SEDDS (17) | 1681 | 0. 500 | 1320 | 5. 35 |
| Example 4-14 | SEDDS (18) | 2196 | 0. 500 | 1720 | 6. 99 |
| Example 4-15 | SEDDS (19) | 2198 | 0. 667 | 1840 | 7. 00 |
| Example 4-16 | SEDDS (20) | 1931 | 0. 500 | 1840 | 6. 15 |
| Example 4-17 | SEDDS (21) | 2126 | 0. 667 | 1920 | 6. 77 |
| Example 4-18 | SEDDS (22) | 2378 | 0. 500 | 2020 | 7. 57 |
| Example 4-19 | SEDDS (23) | 2032 | 0. 500 | 1800 | 6. 47 |
| Example 4-20 | SEDDS (24) | 1696 | 0. 500 | 1330 | 5. 40 |
| Example 4-21 | SEDDS (25) | 1661 | 0. 500 | 1390 | 5. 29 |
| Example 4-22 | SEDDS (26) | 1796 | 0. 500 | 1960 | 5. 72 |
| Example 4-23 | SEDDS (27) | 2098 | 0. 500 | 1960 | 6. 68 |
| Example 4-24 | SEDDS (28) | 1625 | 0. 500 | 1600 | 5. 18 |
| Example 4-25 | SEDDS (29) | 2274 | 0. 667 | 1600 | 7. 24 |
| Example 4-26 | SEDDS (30) | 1535 | 0. 500 | 1860 | 4. 89 |
| Example 4-27 | SEDDS (31) | 2420 | 0. 667 | 1870 | 7. 71 |
| Comparative Example 2 | SEDDS (32) | 1015 | 1. 000 | 526 | 3. 23 |
| Comparative Example 3-1 | SEDDS (33) | 485 | 1. 000 | 391 | 1. 54 |
| Comparative Example 3-2 | SEDDS (34) | 553 | 0. 833 | 361 | 1. 76 |

[Table 15]

| Pharmacokinetics Parameters for Formulations with Compounds other than Compound 1 | | | | | | |
|---|---|---|---|---|---|---|
| Example No. | Formulation | Compound | AUC | Tmax | Cmax | rBA |
| Comparative Example 1 | Solution Administration Formulation | Compound (2) | 674 | 1.670 | 278 | - |
| Example 5-1 | SEDDS (35) | | 701 | 0.833 | 529 | 1.04 |
| Comparative Example 1 | Solution Administration Formulation | Compound (3) | 1988 | 1.670 | 659 | - |
| Example 5-2 | SEDDS (36) | | 4170 | 1.000 | 1680 | 2.10 |
| Comparative Example I | Solution Administration Formulation | Compound (4) | 816 | 0.833 | 414 | - |
| Comparative Example 4- 1 | SEDDS (37) | | 754 | 0.500 | 429 | 0.92 |
| Comparative Example 1 | Solution Administration Formulation | Compound (5) | 2628 | 0.833 | 1500 | - |
| Comparative Example 4-2 | SEDDS (38) | | 2416 | 0.250 | 2150 | 0.92 |
| Comparative Example 1 | Solution Administration Formulation | Compound (6) | 91 | 0.500 | 41 | - |
| Comparative Example 4-3 | SEDDS (39) | | 46 | 0.417 | 32 | 0.51 |

(Example 6-3) PK study in mice whose lymphatic absorption was inhibited

The solution administration formulations prepared in Comparative Example 1 and SEDDS formulation prepared in Example 2 were evaluated for blood kinetics after oral administration to mice whose lymphatic absorption was inhibited in advance. The evaluation method employed the method of inhibiting lymphatic absorption by orally administering in advance a solution of cycloheximide in saline to mice (European Journal of Pharmaceutical Sciences 24(4), 2005, 381-388). The PK study was performed by the same method as that of Example 6-2, except that one hour before oral administration of a solution administration formulation or a SEDDS formulation, 1 mg/mL solution of cycloheximide (manufactured by Sigma-Aldrich Co.) in saline was administered orally at 6 mg/kg, and pharmacokinetic parameters were calculated (Table 16). The change in blood concentration is shown in Fig. 14. rBA was 4.56 when cycloheximide was not administered (Table 14), whereas rBA was 1.71 when cycloheximide was administered in advance, showing that the SEDDS formulation of the present invention was absorbed *via* the lymph route thereby leading to increase in rBA.

[Table 16]

| Pharmacokinetics Parameters for Solution Administration Formulation and SEDDS Formulation in Mice whose Lymphatic Absorption was Inhibited | | | | |
|---|---|---|---|---|
| Formulation | AUC | Tmax | Cmax | rBA |
| Solution Administration Formulation | 302 | 1.330 | 127 | - |
| SEDDS (1) | 578 | 0.500 | 553 | 1.91 |

(Example 6-4) PK study in Mdr1a/b-Bcrp-knockout mice

The solution administration formulations prepared in Comparative Example 1 and SEDDS formulation prepared in Example 2 were evaluated for blood kinetics after oral administration to mice whose P-gp and BCRP transporters were knocked out. The PK study was performed by the same method as that of Example 6-2, except that the mice used were changed to Mdr1a/b-Bcrp-knockout mice (produced by Clea Japan), and pharmacokinetic parameters were calculated (Table 17). The change in blood concentration is shown in Fig. 15. rBA was 4.56 in normal mice (Table 14) whereas rBA was 1.51 in mice whose P-gp and BCRP transporters were knocked out, showing that the SEDDS formulation of the present invention was absorbed with avoiding the transporters, thereby leading to increase in rBA.

[Table 17]

| Pharmacokinetics Parameters for Solution Administration Formulation and SEDDS Formulation in Mdrla/b-BCRP Knockout Mice | | | | |
|---|---|---|---|---|
| Formulation | AUC | Tmax | Cmax | rBA |
| Solution Administration Formulation | 6050 | 2.000 | 1240 | - |
| SEDDS (1) | 9140 | 1.000 | 2300 | 1.51 |

(Reference Example 1)

Table 13 lists the physical property values (Caco-2, LogD) of commercially available middle molecular-weight compounds having a molecular weight in the range of approximately 700 to approximately 1200. The physical property values of the commercially available middle molecular-weight compounds are plotted in Fig. 16 along with the experimental values obtained in Example 6-2. It is apparent that as compared to the physical property values of commercially available conventional middle molecular-weight compounds, the physical property values of a group of compounds which are preferred application targets for the lymphatically transported SEDDS formulations of the present invention (BA UP in Fig. 16), which are shown in Example 6-2, indicate higher lipid solubility (logD (pH 7.4)), and have physical properties of poor membrane permeability (Caco-2 Papp (cm/sec)) (Fig. 16). This demonstrates that the self-emulsifying formulations in the present invention are formulations targeting a group of compounds which conventionally have been difficult to develop as oral pharmaceutical products.

[Table 13]

| Physical Property of Commercially-available Middle Molecular Weight Compounds | | | |
|---|---|---|---|
| Commercially-available middle molecular weight compounds | Caco-2 Membrane Permeability (cm/s) | LogD (pH 7.4) | Compound MW (MW: 721-1203) |
| AmphotericinB | 7.36E-08 | 0.11 | 924 |
| Cyclosporin | 1.76E-06 | 5.33 | 1203 |
| Erythromycin | 3.82E-07 | 0.89 | 734 |
| Paclitaxel | 1.38E-07 | 3.36 | 854 |
| Rapamycin | 1.84E-06 | 4.36 | 914 |
| Rifabutin | 1.08E-06 | 3.88 | 847 |
| Rifampicin | 1.73E-06 | 1.61 | 823 |
| Rifapentine | 5.12E-06 | 2.45 | 877 |
| Roxithromycin | 1.08E-07 | 2.04 | 837 |
| Tacrolimus | 2.48E-06 | 4.19 | 804 |
| Telithromycin | 3.19E-07 | 2.10 | 812 |
| Itraconazole | 1.06E-06 | 4.29 | 706 |
| Vinorelbine | 9.03E-08 | 2.82 | 1079 |
| Azithromycin | 5.15E-07 | 0.62 | 749 |
| Ritonavir | 1.74E-06 | 4.29 | 721 |
| Digoxin | 2.54E-07 | 1.21 | 781 |

[Example 7] Establishment of improved methods for membrane permeability assay using Caco-2 cells

(1) Cell culture

Caco-2 cells (CACO-2 Lot No. 028; Riken BRC Cell Bank) were seeded at a density of 1.0 × $10^5$ cells/well on a membrane of 24-well transwell (Corning HTS Transwell, pore size 0.4 μm, polycarbonate membrane), and were cultured in an incubator maintained at 37°C and 5% $CO_2$ with a DMEM medium containing 10% FBS, penicillin-streptomycin-glutamine (100x), L-glutamine, sodium chloride solution, and non-essential amino acids (Neaa). The medium was exchanged every two or three days, and Caco-2 cells were subjected to membrane permeability measurements on the $21^{st}$ to $23^{rd}$ day after seeding.

(2) Pre-incubation

To perform a long incubation as an improved method for membrane permeability assay, various buffers were examined for the effects of incubation time on cells through evaluation of transepithelial electric resistance (TEER).

According to conventional methods for membrane permeability assay (Artursson, P., 2001. Adv Drug Deliv Rev 46, 27-43; Mason, A.K., 2013. Drug Metab Dispos 41, 1347-1366; Polli, J.W., 2001. J Pharmacol Exp Ther 299, 620-628; and Sun, H. 2008. Expert Opin Drug Metab Toxicol 4, 395-411), fasted state simulated intestinal and stomach fluids (FaSSIF) (with 1% DMSO) was added to the Apical side and Hanks' Balanced Salt Solutions (HBSS) (with 4% BSA) was added to the Basal side, and this was incubated at 37°C. As a result, incubation for three hours was found to decrease TEER by 30% or more. This revealed that conventional methods cannot be used for incubation of over three hours, and compounds with long lag time cannot be evaluated by conventional methods (Fig. 19).

Next, for performing long incubations, effects of incubation time (0, 2, 4, 6, 8, and 24 hours) in a medium (DMEM) on cells were evaluated by measuring the TEER at each time. As a result, adding DMEM + FaSSIF (1% DMSO, 2% dimethylacetamide (DMA)) to the Apical side and adding DMEM to the Basal side showed that TEER practically does not decrease for 24 hours. This result revealed that under conditions of DMEM + FaSSIF (1% DMSO, 2% DMA) on the Apical side and DMEM on the Basal side, a 24-hour incubation can be performed (Fig. 20).

(3) Membrane permeability evaluation after pre-incubation

A cyclic peptide cyclosporine (Compound A) and other cyclic peptides (Compounds B to H) were used as test substances, and after pre-incubation established in (2) above, membrane permeability assays from the Apical side to the Basal side (AtoB tests) were performed according to conventional methods. Pre-incubation was performed after culturing Caco-2 cells for three weeks according to (1) above. More specifically, according to (2) above, a 24-hour pre-incubation was performed under the conditions of DMEM + FaSSIF (1% DMSO, 2% DMA) + test substance on the Apical side and DMEM on the Basal side. The AtoB test performed after the pre-incubation was initiated by removing the pre-incubation solutions from the Apical side and the Basal side by aspiration, and adding FaSSIF/HBSS (with 1% DMSO, 2% DMA) (pH 6.0) containing a test substance to the Apical side and adding HBSS (4% BSA) (pH 7.4) to the Basal side. Each well was shaken at 220 rpm while warming to keep it at 37°C. Samples were collected from the Basal side 30, 60, 90, and 120 minutes after initiation, and LC/MS was used to measure the amount of permeation. The membrane permeability coefficient ($P_{app}$) was calculated from the amount of permeation.

Absorption ratios (Fa) were calculated by evaluating the plasma concentration and fecal excretion ratio after intravenous and oral administration of the cyclic peptide cyclosporine and other cyclic peptides to mice. The compounds were intravenously and orally administered to male mice (C57BL/6J, six-week-old, produced by Clea Japan). Blood was collected over time from the dorsal foot vein using a hematocrit tube treated with heparin as an anticoagulant until 24 hours after the administration. Feces were collected up to 72 hours. Measurements were performed using a LC/MS/MS instrument (API3200, manufactured by AB SCIEX). Absorption ratios (Fa) were calculated from the obtained plasma concentrations and fecal concentrations of the drugs (Qingqing X., et al., 2016, Xenobiotica. 46, 913-921).

For most of the test substances, the membrane permeability coefficients calculated by the improved method (the method in which pre-incubation is performed for a long time) were greater than those calculated by conventional methods; and, this revealed that the membrane permeability coefficients were underestimated in conventional methods. In particular, Compound H which could not be evaluated since its membrane permeability coefficient was smaller than 1.0 × $10^{-8}$ cm/sec in conventional methods had membrane permeability coefficient of 3 × $10^{-7}$ cm/sec in the improved method, and large shifts were observed for compounds with small membrane permeability coefficient. Furthermore, Fa and membrane permeability coefficient of a compound tended to show better correlation in the improved method (Pre-incubation (+)) when compared to the conventional method (Pre-incubation (-)) (Table 18, and Figs. 21 and 22). Accordingly, it was shown that performing membrane permeability measurements by the improved method enables prediction of absorption ratios of the test substances. Since Fa was 0.3 or higher in compounds whose membrane permeability

coefficients measured by the improved method were 1.0 × $10^{-6}$ cm/sec or greater, it was considered that "membrane permeability coefficient ($P_{app}$) ≥ 1.0 × $10^{-6}$ cm/sec" can be used as one of criterion for selecting peptide compounds with high membrane permeability (for example, peptide compounds having membrane permeability at a level that enables their development as oral agents).

[Table 18]

| | $P_{app}$ (cm/sec) | | Fa |
|---|---|---|---|
| | Preincubation (-) | Preincubation (+) | |
| Compound A | 8.75E-06 | 1.10E-05 | 0.898 |
| Compound B | 1.75E-06 | 4.17E-06 | 0.628 |
| Compound C | 6.62E-06 | 9.67E-06 | 0.935 |
| Compound D | 8.31E-07 | 2.91E-06 | 0.350 |
| Compound E | 5.33E-07 | 9.50E-07 | 0.592 |
| Compound F | 5.38E-08 | 1.08E-06 | 0.492 |
| Compound G | 1.88E-07 | 1.71E-06 | 0.335 |
| Compound H | N.D. | 3.23E-07 | 0.169 |
| N.D. = Not detected | | | |

Measurement of membrane permeability was carried out according to the improved method established above. More specifically, after Caco-2 cells were cultured on a 96-well transwell for three weeks, DMEM + FaSSIF (1% DMSO) + the compound was added to the Apical side and DMEM was added to the Basal side, and this was pre-incubated at 5% $CO_2$, 37°C, and 80 rpm for 20 hours to 24 hours. After pre-incubation, the pre-incubation solutions on the Apical side and on the Basal side were removed by aspiration and washed, FaSSIF/HBSS buffer (1% DMSO) (pH 6.0) containing the compound was added to the Apical side, and HBSS buffer (4% BSA) (pH 7.4) was added to the Basal side, and membrane permeability assays were initiated. Each well was shaken under conditions of 5% $CO_2$, 37°C, and 80 rpm, and 180 minutes after initiation, samples on the Basal side were collected, and the amount of permeation was determined by LC/MS. From the amount of permeation, the membrane permeability coefficient ($P_{app}$) was calculated. Note that "Caco-2 (cm/sec)" written in the columns of the Tables herein and "$P_{app}$ (cm/sec)" are used synonymously.

## Industrial Applicability

The present invention has provided lymphatically transported self-emulsifying formulations for improving membrane permeability of poorly membrane-permeable compounds. Using formulations of the present invention can improve the membrane permeability of middle molecular-weight compounds (for example, molecular weight of 500 to 6000) in which (i) logD (pH 7.4) value of the drug is 3.2 or greater, and/or (ii) Caco-2 Papp (cm/sec) value of the drug is 1.8E-6 or less. Applying middle molecular-weight compounds to the formulations of the present invention enables use of such middle molecular-weight compounds as pharmaceuticals for oral administration.

## ITEMS

The present invention relates to the following items:

1. A self-emulsifying formulation, which comprises a surfactant comprising oleate ester or oleylether as a moiety, an oily component, and a poorly membrane-permeable drug.

2. The self-emulsifying formulation of item 1, which is for enhancing membrane permeability of the poorly membrane-permeable drug.

3. The self-emulsifying formulation of item 1 or 2, which is lymphatically transported.

4. The self-emulsifying formulation of any one of items 1 to 3, wherein the drug has features (i) and (ii) below:

(i) logD (pH 7.4) value of the drug is 3.2 or greater; and
(ii) Caco-2 Papp (cm/sec) value of the drug is 1.8E-6 or less.

5. The self-emulsifying formulation of any one of items 1 to 4, wherein the drug is a peptide compound comprising a cyclic portion, wherein the compound has features (i) and/or (ii) below:

(i) the compound comprises the cyclic portion whose total number of natural amino acid and amino acid analog residues is 5 to 12, and the compound's total number of natural amino acid and amino acid analog residues is 9 to 13;
(ii) the compound comprises at least two *N*-substituted amino acids and comprises at least one amino acid that is not *N*-substituted.

6. The self-emulsifying formulation of any one of items 1 to 5, which further comprises one or more types of hydrophilic surfactants.

7. The self-emulsifying formulation of any one of items 1 to 6, which further comprises oleic acid.

8. The self-emulsifying formulation of any one of items 1 to 7, wherein the surfactant comprising oleate ester or oleylether as a moiety is one or more types of surfactants selected from the group consisting of glyceryl monooleate, decaglyceryl monooleate, polyglyceryl-3 oleate, polyglyceryl-3 dioleate, polyethylene glycol (10) monooleate, polyethylene glycol (15) monooleate, polyethylene glycol (20) monooleate, polyethylene glycol (30) monooleate, polyethylene glycol (35) monooleate, apricot kernel oil polyoxyethylene-6 ester, sorbitan monooleate, sorbitan trioleate, polyethylene glycol (10) oleylether, polyethylene glycol (15) oleylether, polyethylene glycol (20) oleylether, and polyethylene glycol (50) oleylether.

9. The self-emulsifying formulation of any one of items 1 to 8, wherein the oily component is one or more types of oily components selected from the group consisting of olive oil, almond oil, coconut oil, cacao butter, macadamia nut oil, avocado oil, safflower oil, soybean oil, linseed oil, rapeseed oil, castor oil, palm oil, high-oleic sunflower oil, high-oleic safflower oil, sunflower oil, cotton seed oil, corn oil, sesame oil, peanut oil, apricot kernel oil, candlenut oil, grapeseed oil, pistachio seed oil, sunflower oil, hazelnut oil, jojoba oil, meadowfoam oil, rosehip oil, Tricaproin, Tricaprylin, Tricaprin, Tripalmitolein, Triolein, Trilinolein, Trilinolenin, Trieicosenoin, and Trierucin.

10. The self-emulsifying formulation of any one of items 1 to 8, wherein the oily component is an oily component comprising oleic acid as the main oil type component.

11. The self-emulsifying formulation of item 10, wherein the oily component comprising oleic acid as the main oil type component is one or more types of oily component selected from the group consisting of camellia oil, sunflower oil, avocado oil, avocado oil, safflower oil, almond oil, olive oil, rapeseed oil, and cashew oil.

12. The self-emulsifying formulation of any one of items 6 to 11, wherein the hydrophilic surfactant is polyoxyethylene hydroxystearate, polyoxyethylene hydroxyoleate, polyoxyl 40 hardened castor oil, or polyoxyl 35 castor oil.

13. The self-emulsifying formulation of any one of items 1 to 12, wherein the oily component is 8 vol% to 40 vol% based on the whole formulation (excluding the volume of the drug).

14. A self-emulsifying formulation, which comprises glyceryl monooleate at 9 vol% to 19 vol%, polyoxyethylene hydroxystearate at 51 vol% to 61 vol%, and olive oil at 17.5 vol% to 27.5 vol% based on the whole formulation (excluding the volume of a drug), and a poorly membrane-permeable drug.

15. A self-emulsifying formulation, which comprises a surfactant comprising oleate ester or oleylether as a moiety, an oily component, and a poorly membrane-permeable drug having features (i) to (iii) below:

(i) logD (pH 7.4) value of the drug is 3.2 or greater;
(ii) Caco-2 Papp (cm/sec) value of the drug is 1.8E-6 or less; and
(iii) molecular weight of the drug is 500 or greater.

16. The self-emulsifying formulation of item 15, wherein the drug is a peptide compound comprising a cyclic portion, wherein the compound has features (i) and/or (ii) below:

(i) the compound comprises the cyclic portion whose total number of natural amino acid and amino acid analog residues is 5 to 12, and the compound's total number of natural amino acid and amino acid analog residues is 9 to 13; and
(ii) the compound comprises at least two *N*-substituted amino acids, and comprises at least one amino acid that is not *N*-substituted.

**Claims**

**1.** A method of measuring membrane permeability of a test substance using Caco-2 cells, which comprises the step of pre-incubating Caco-2 cells in the mixed presence with the test substance.

**2.** The method of claim 1, which comprises the step of pre-incubating Caco-2 cells in the mixed presence with the test substance for two hours or more.

**3.** The method of any one of claim 1 or 2, wherein pre-incubation time in the pre-incubating step is 48 hours or less.

**4.** A pre-incubation method for measuring membrane permeability, which comprises the step of incubating Caco-2 cells in the mixed presence with a test substance for four hours or more.

**5.** The method of claim 4, which comprises the step of incubating Caco-2 cells in the mixed presence with the test substance for 24 hours or more.

**6.** The method of any one of claims 1 to 5, wherein the step of pre-incubating Caco-2 cells or the step of incubating Caco-2 cells is performed by contacting the Caco-2 cells with a medium.

**7.** The method of claim 6, wherein the medium is a cell culture medium.

**8.** The method of claim 6, wherein the medium is DMEM.

**9.** The method of any one of claim 1 to 8, wherein the test substance is a substance with ClogP of 3 or greater.

**10.** The method of any one of claims 1 to 9, wherein the test substance is a peptide compound.

**11.** The method of any one of claims 1 to 10, which further comprises the step of measuring membrane permeability of the test substance by placing a solution containing the test substance so that it contacts with one side of a Caco-2 cell layer, placing a solution not containing the test substance so that it contacts with the other side of the Caco-2 cell layer, and after a predetermined period of time, measuring the amount of the test substance in the solution on said one side and/or the amount of the test substance in the solution on said other side.

**12.** A method of screening for a test substance, which comprises the steps of:

(a) measuring membrane permeability of a plurality of test substances by the method of any one of claims 1 to 11; and
(b) selecting a desired test substance from the plurality of test substances based on the membrane permeability data obtained in (a).

**13.** The method of claim 12, wherein the step (b) includes selecting a test substance having a membrane permeability coefficient ($P_{app}$) of $1.0 \times 10^{-6}$ cm/second or greater.

**14.** A method of producing a peptide compound, which comprises the steps of:

(i) measuring membrane permeability of a plurality of peptide compounds by the method of any one of claims 1 to 11;
(ii) selecting a desired peptide compound from the plurality of peptide compounds based on the membrane permeability data obtained in (i); and
(iii) producing a peptide compound based on the amino acid sequence of the peptide compound selected in (ii).

**15.** A peptide compound produced by the method of claim 14.

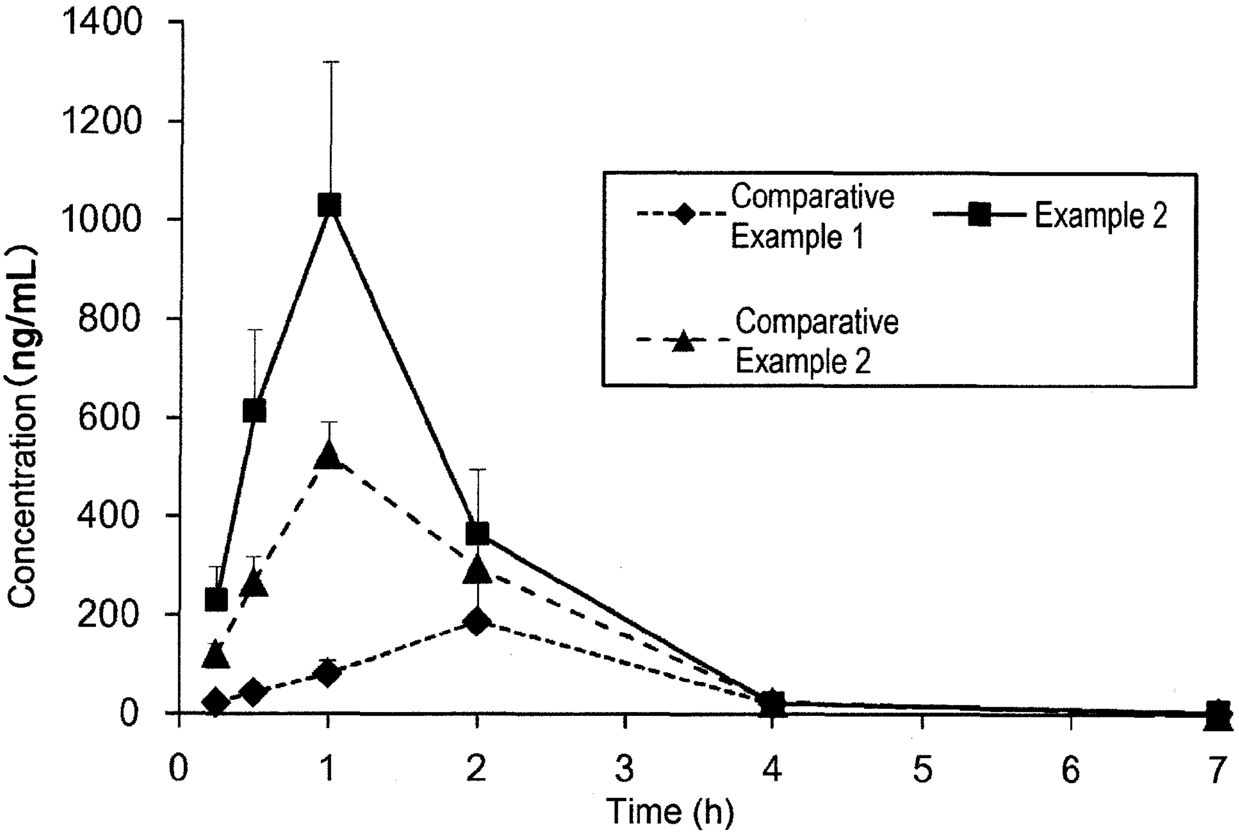
1400
1200
1000
800
600
400
200
0
Concentration (ng/mL)
0
1
2
3
4
5
6
7
Time (h)
Comparative Example 1
Example 2
Comparative Example 2

FIG. 1

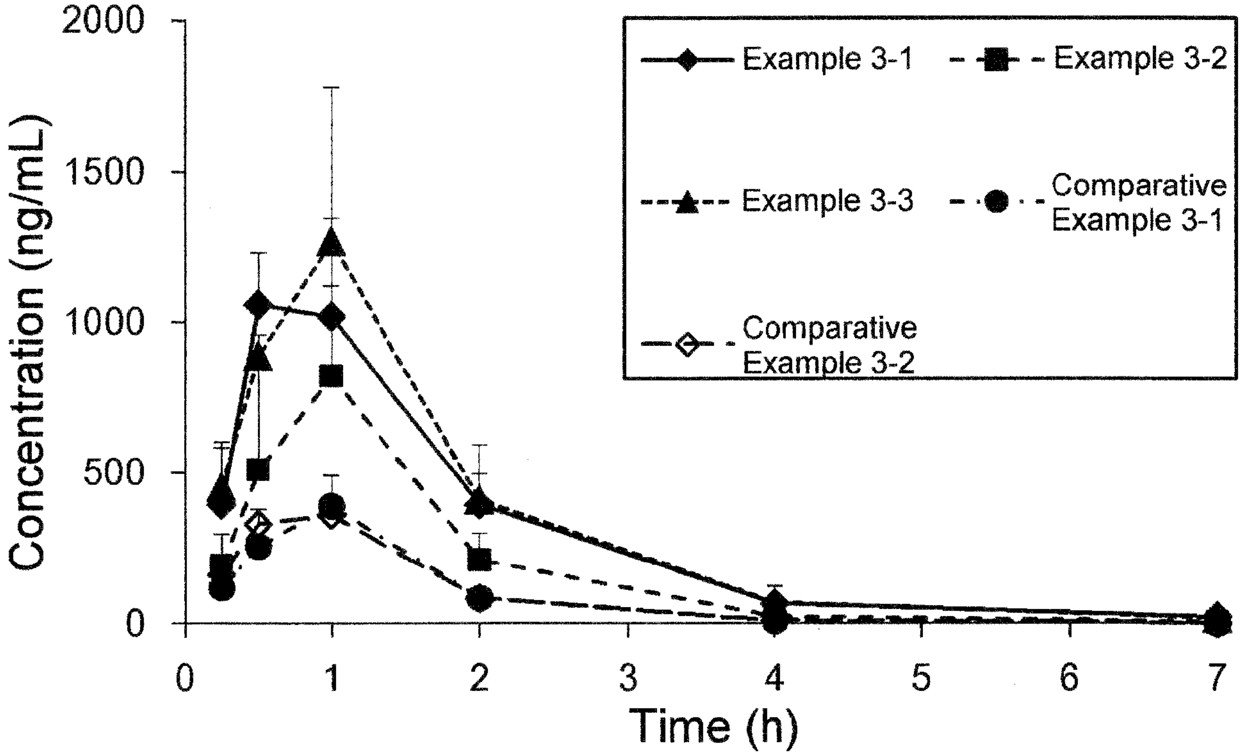
2000
1500
1000
500
0
Concentration (ng/mL)
0
1
2
3
4
5
6
7
Time (h)
Example 3-1
Example 3-2
Example 3-3
Comparative Example 3-1
Comparative Example 3-2

FIG. 2

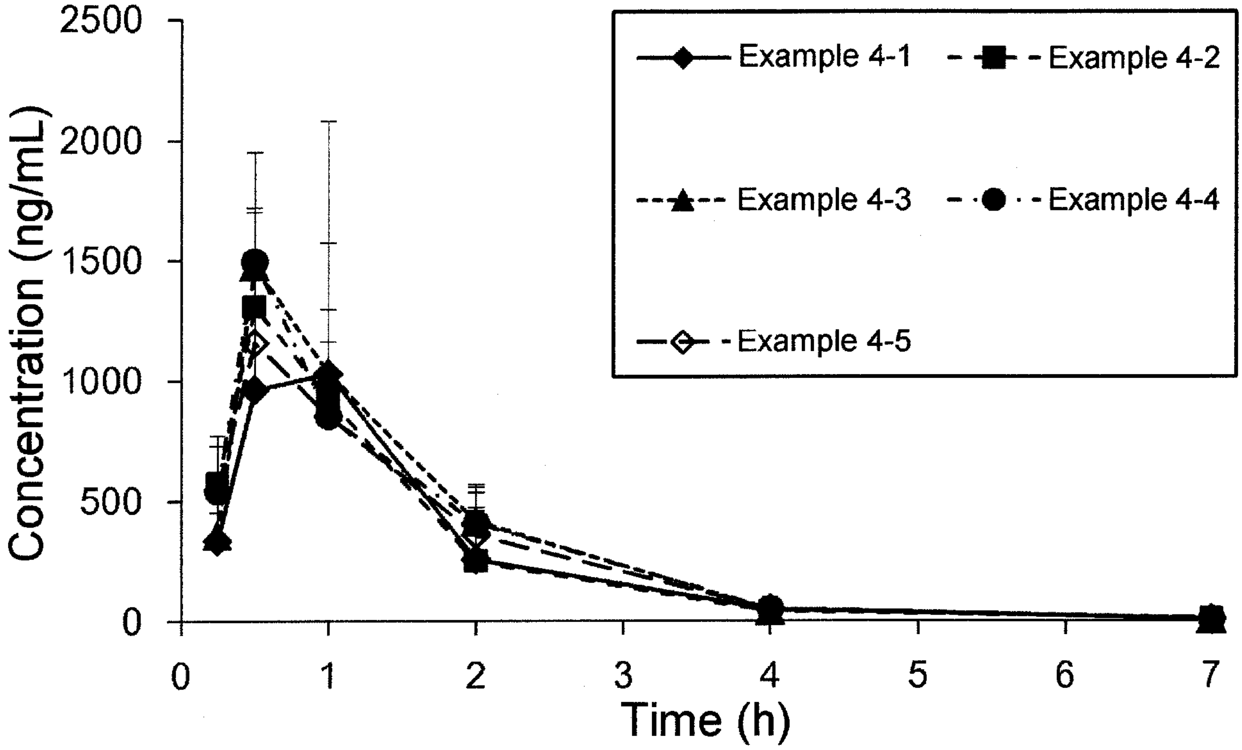
2500
2000
1500
1000
500
0
Concentration (ng/mL)
0
1
2
3
4
5
6
7
Time (h)
Example 4-1
Example 4-2
Example 4-3
Example 4-4
Example 4-5

FIG. 3

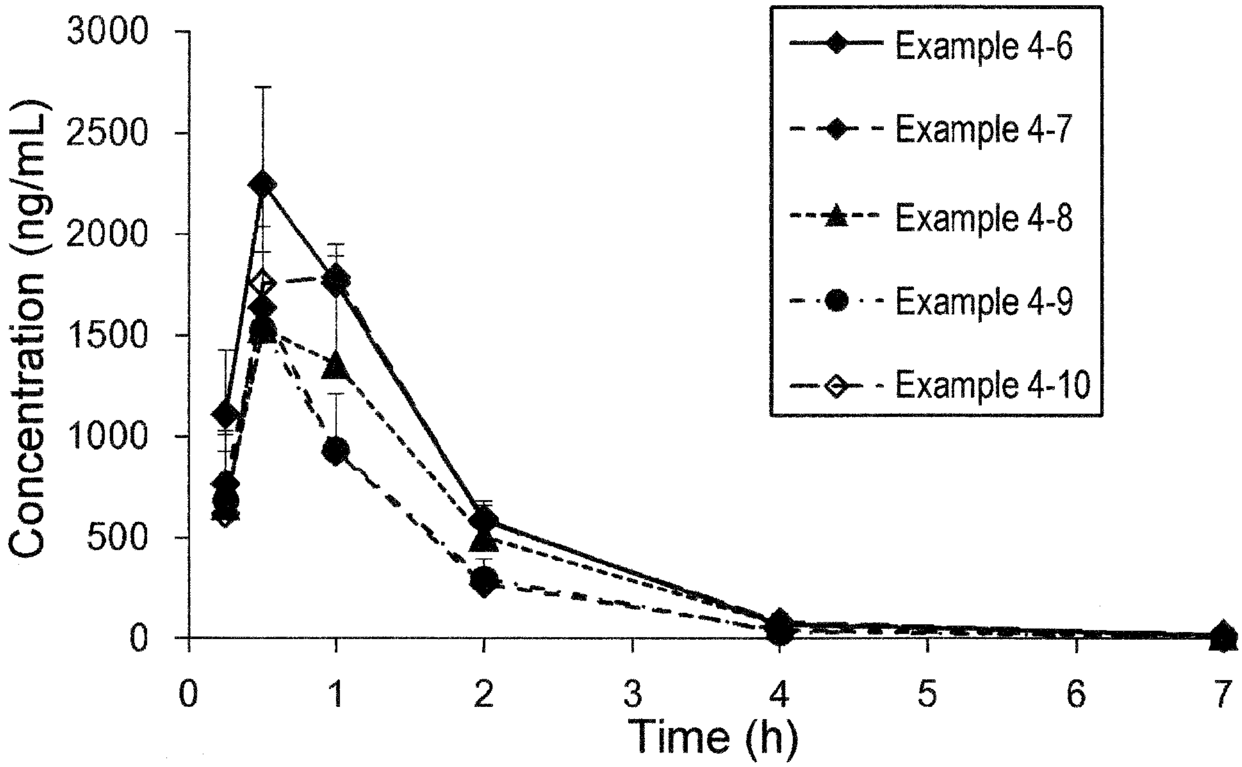
3000
2500
2000
1500
1000
500
0
Concentration (ng/mL)
0
1
2
3
4
5
6
7
Time (h)
Example 4-6
Example 4-7
Example 4-8
Example 4-9
Example 4-10

FIG. 4

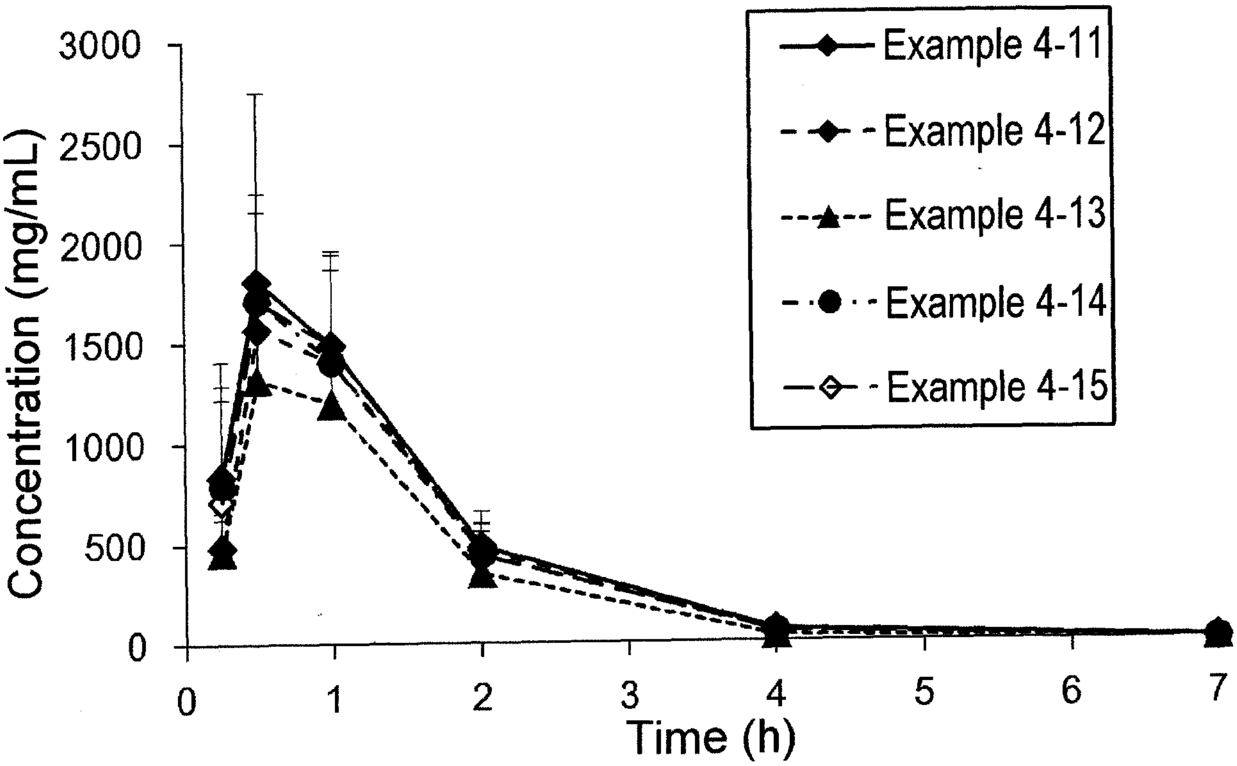
Example 4-11
Example 4-12
Example 4-13
Example 4-14
Example 4-15
Concentration (mg/mL)
3000
2500
2000
1500
1000
500
0
0
1
2
3
4
5
6
7
Time (h)

FIG. 5

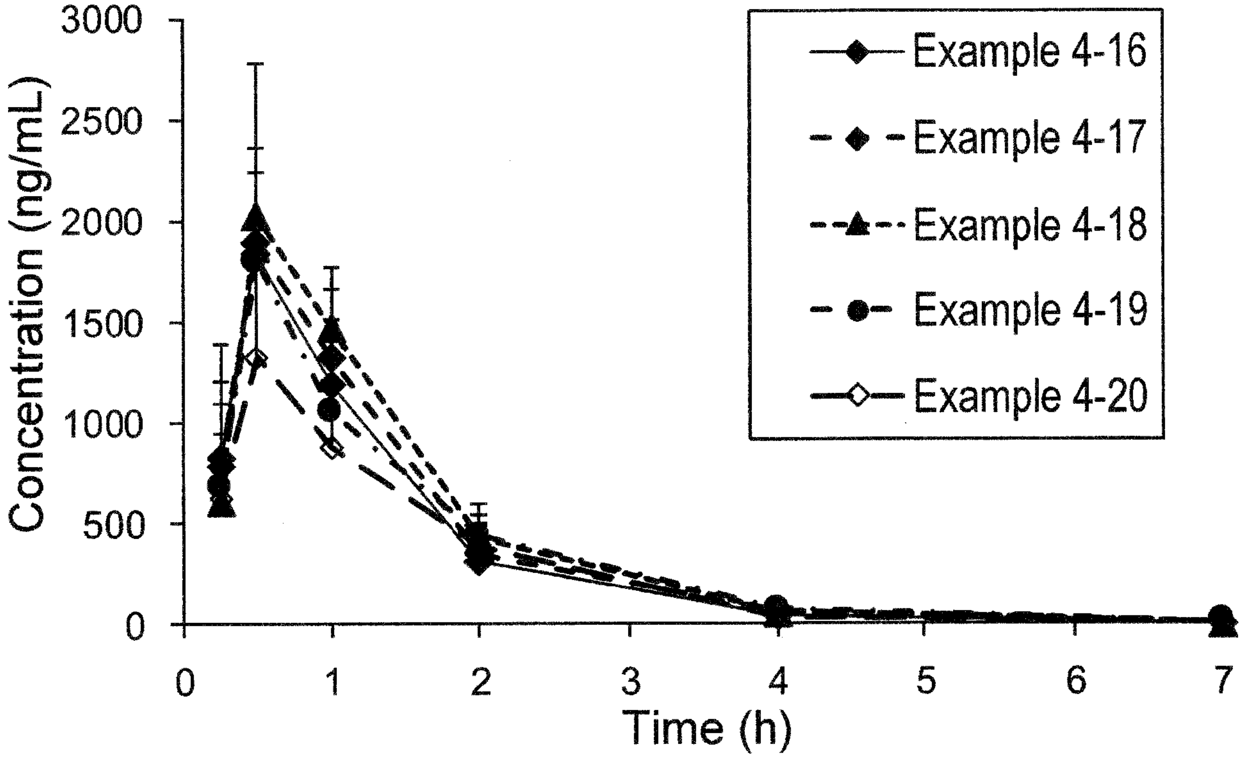
3000
2500
2000
1500
1000
500
0
Concentration (ng/mL)
0
1
2
3
4
5
6
7
Time (h)
Example 4-16
Example 4-17
Example 4-18
Example 4-19
Example 4-20

FIG. 6

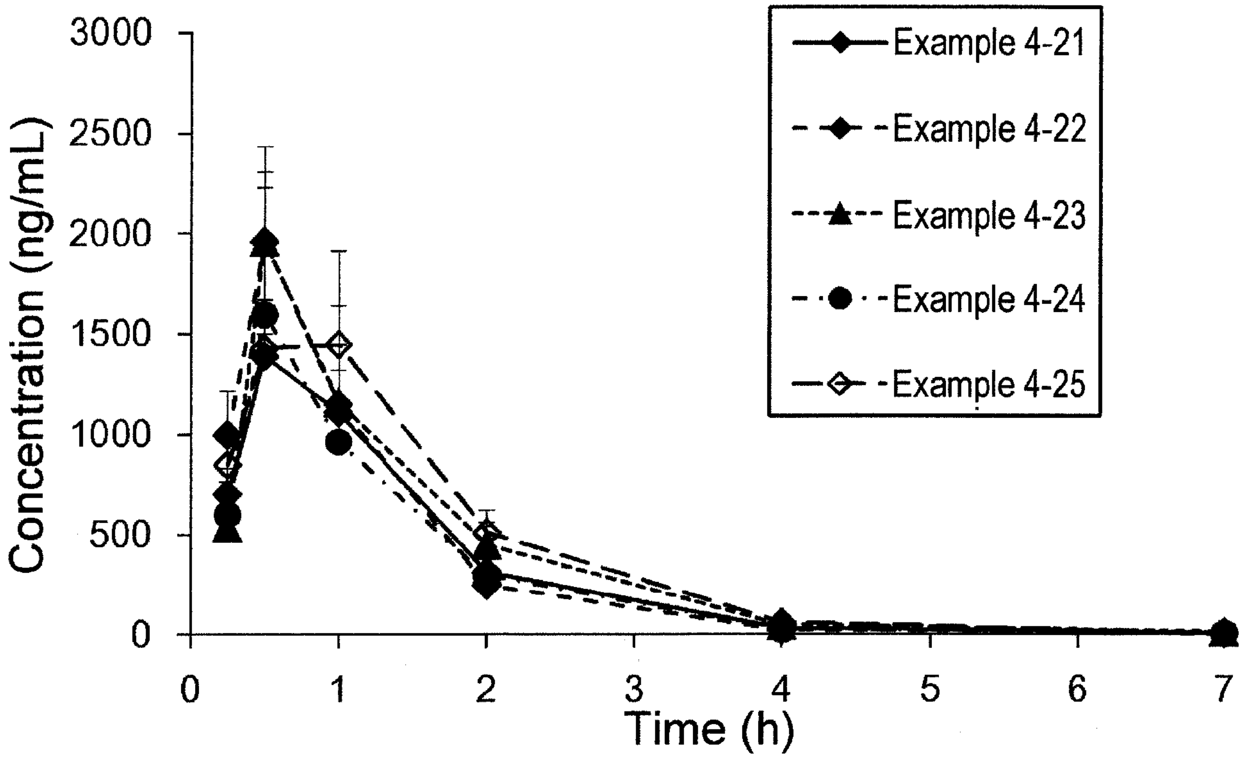
3000
2500
2000
1500
1000
500
0
Concentration (ng/mL)
0
1
2
3
4
5
6
7
Time (h)
Example 4-21
Example 4-22
Example 4-23
Example 4-24
Example 4-25

FIG. 7

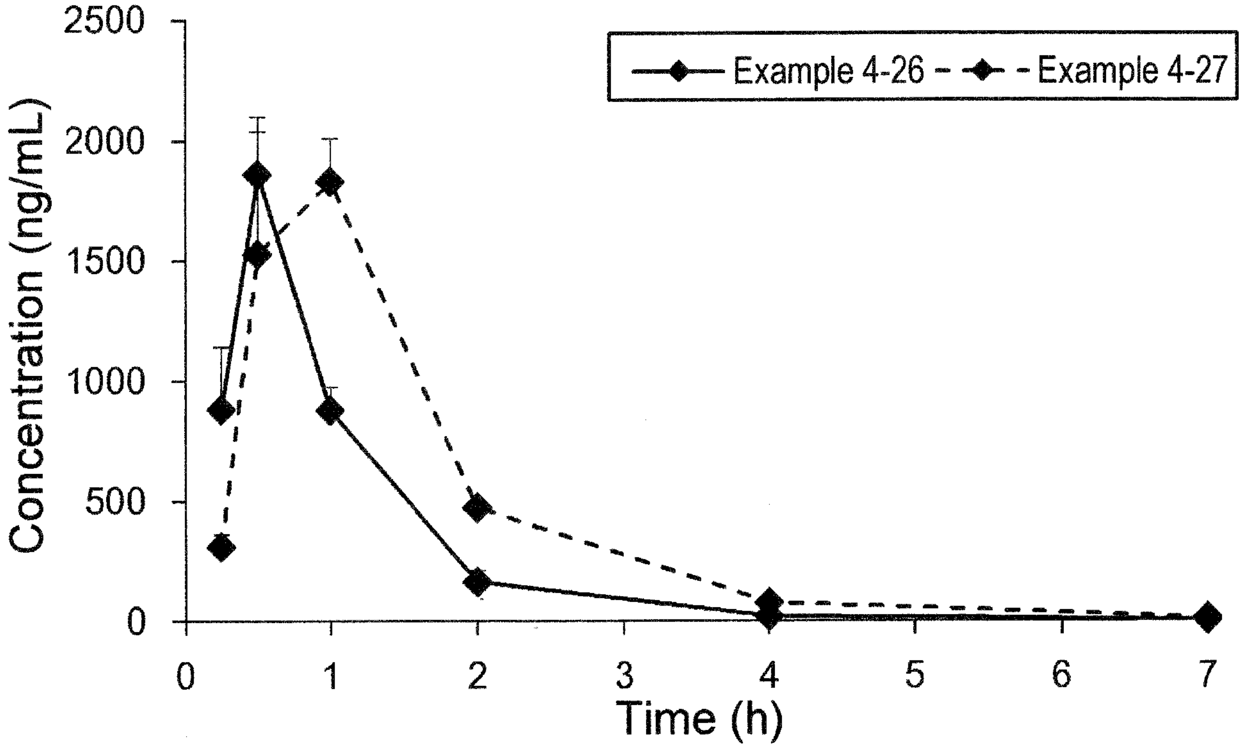
Example 4-26
Example 4-27
Concentration (ng/mL)
2500
2000
1500
1000
500
0
0
1
2
3
4
5
6
7
Time (h)

FIG. 8

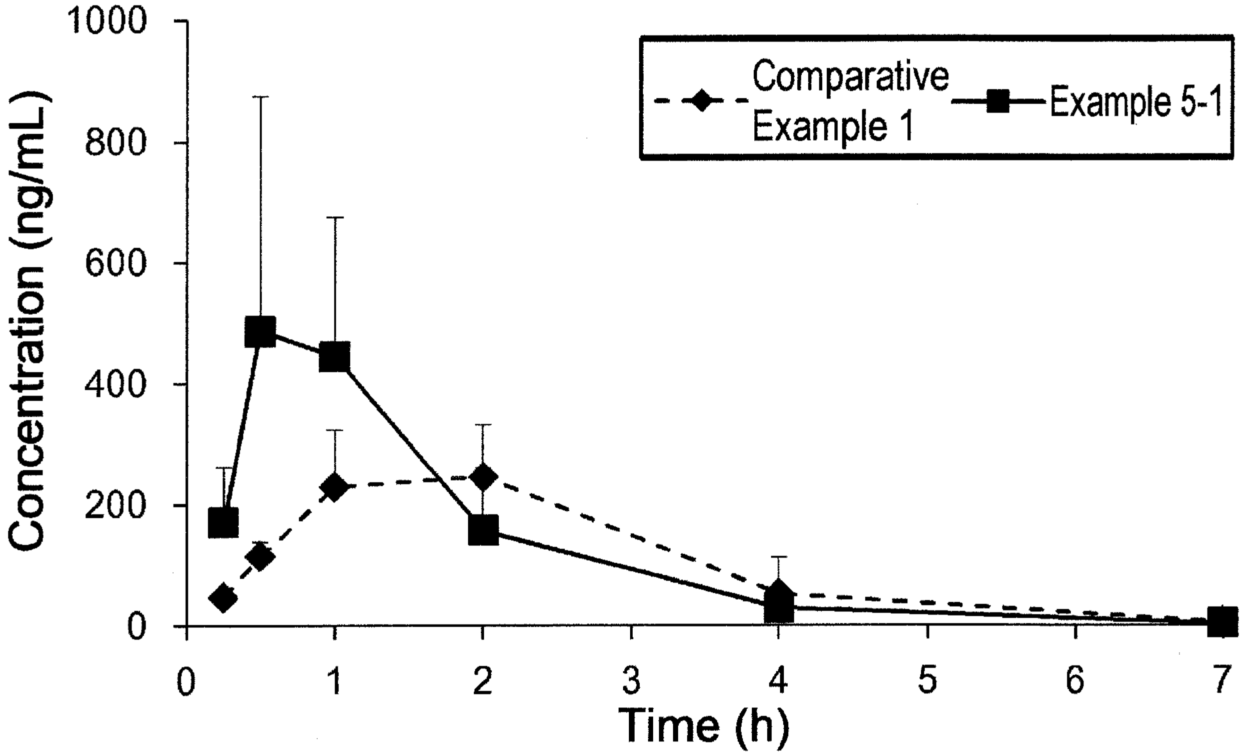
Concentration (ng/mL)
1000
800
600
400
200
0
Comparative Example 1
Example 5-1
0
1
2
3
4
5
6
7
Time (h)

FIG. 9

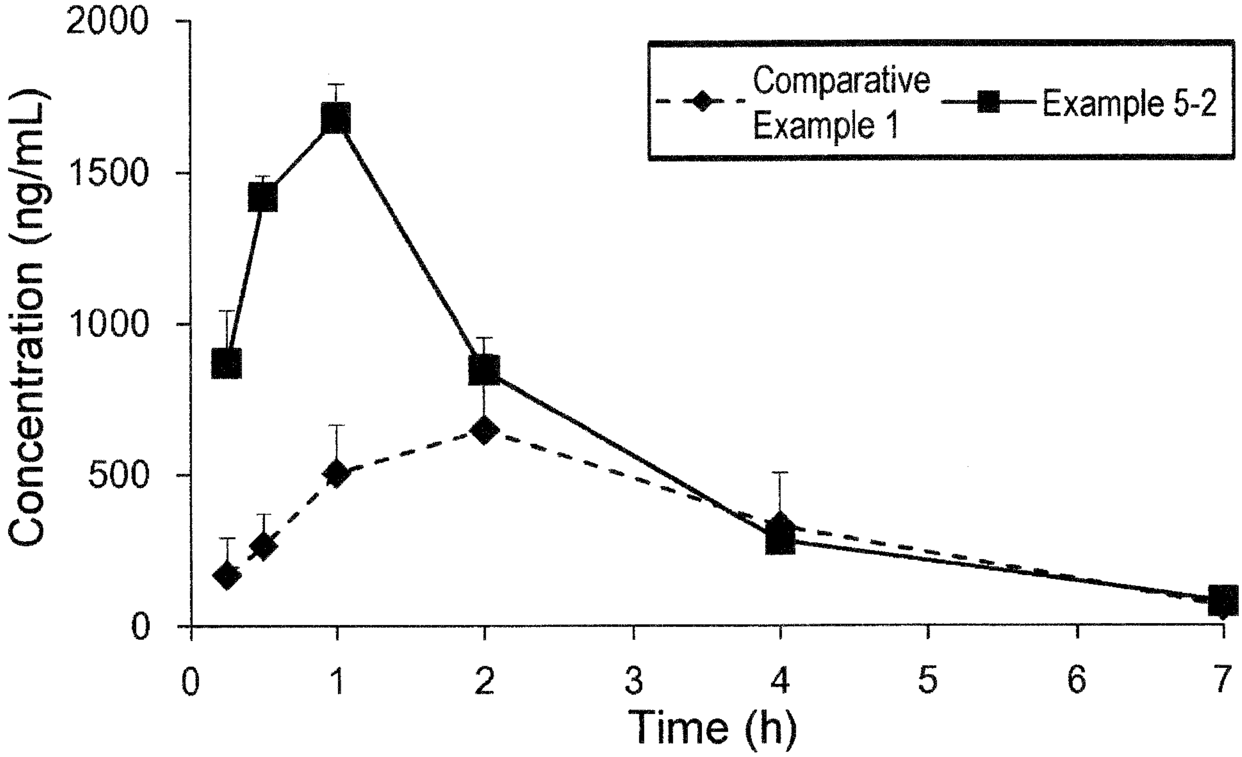
2000
1500
1000
500
0
Concentration (ng/mL)
0
1
2
3
4
5
6
7
Time (h)
Comparative Example 1
Example 5-2

FIG. 10

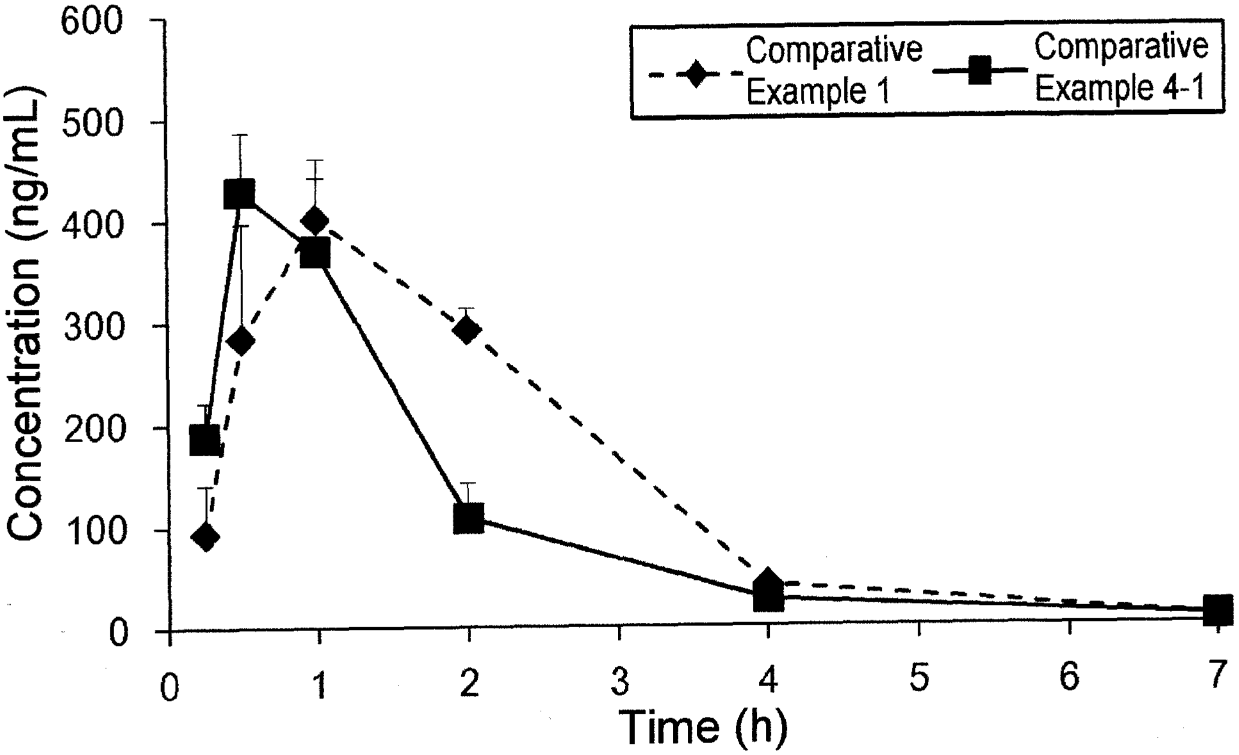
600
500
400
300
200
100
0
Concentration (ng/mL)
Comparative Example 1
Comparative Example 4-1
0
1
2
3
4
5
6
7
Time (h)

FIG. 11

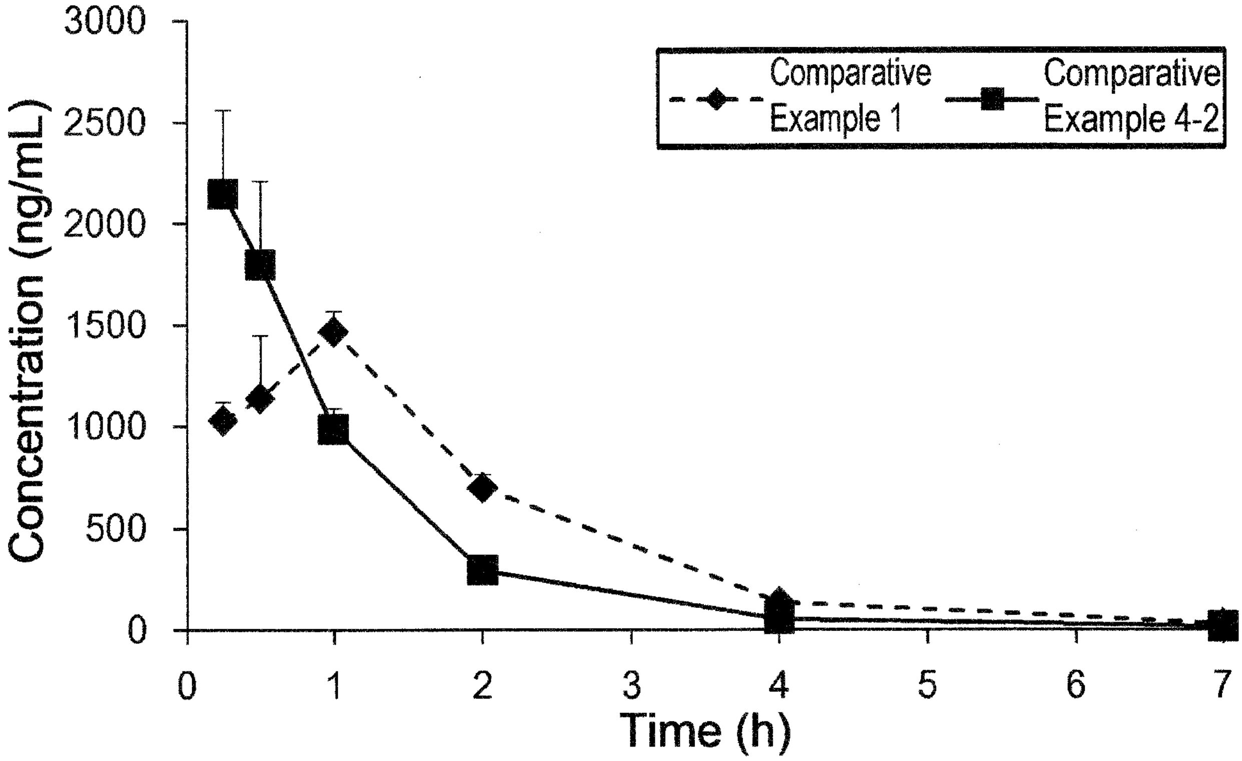
3000
2500
2000
1500
1000
500
0
Concentration (ng/mL)
Comparative Example 1
Comparative Example 4-2
0
1
2
3
4
5
6
7
Time (h)

FIG. 12

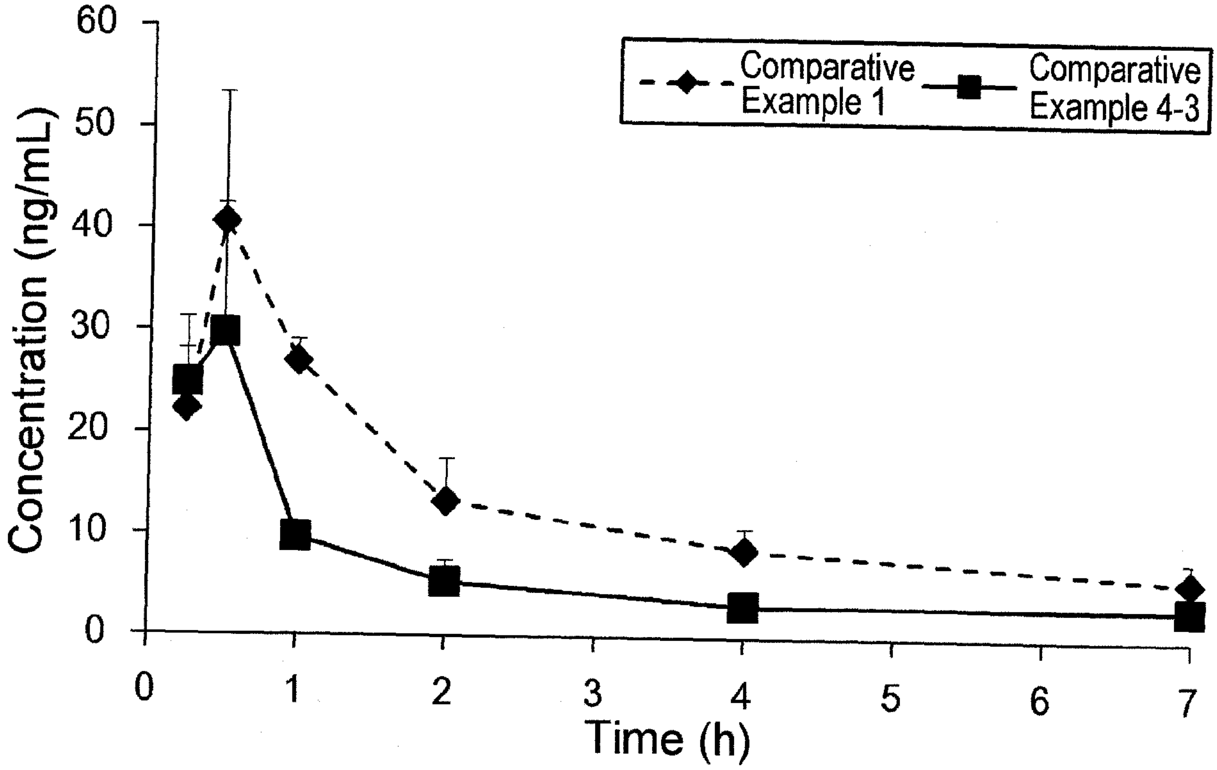
60
50
40
30
20
10
0
Concentration (ng/mL)
0
1
2
3
4
5
6
7
Time (h)
Comparative Example 1
Comparative Example 4-3

FIG. 13

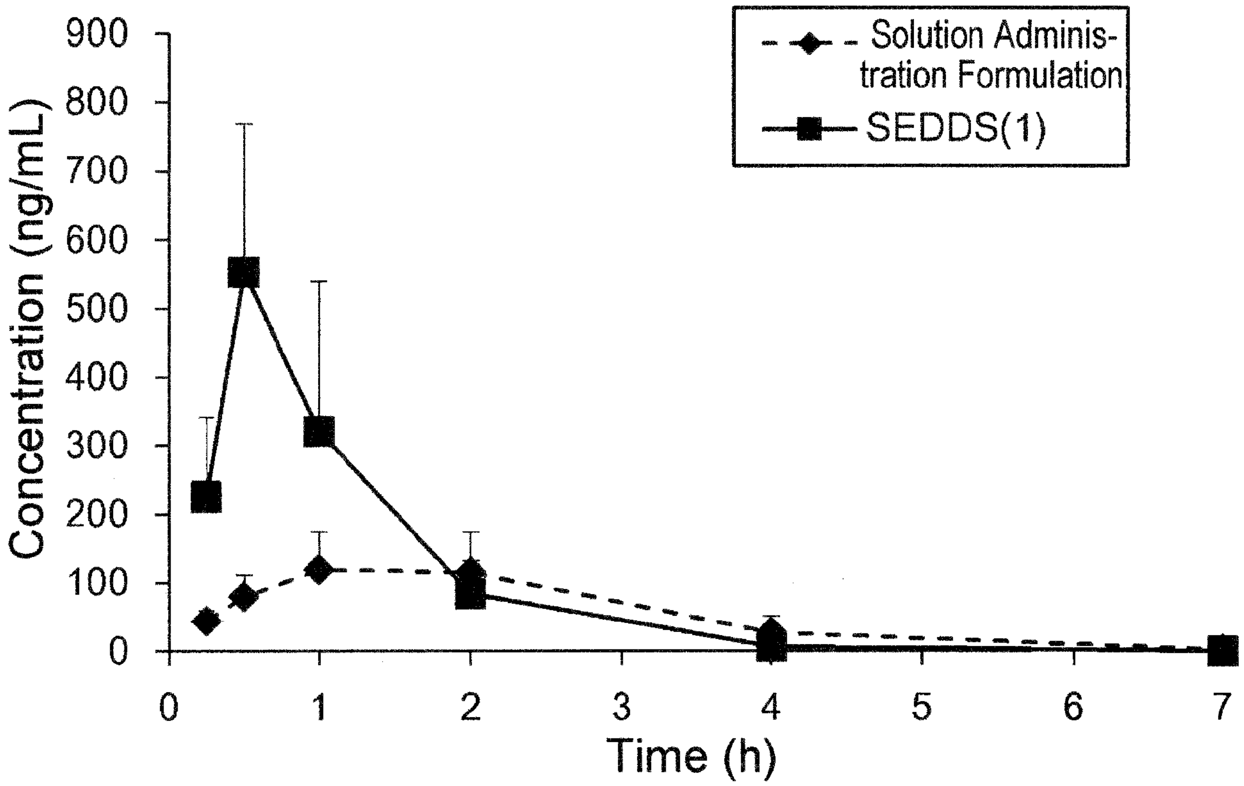
Solution Administration Formulation
SEDDS(1)
Concentration (ng/mL)
900
800
700
600
500
400
300
200
100
0
0
1
2
3
4
5
6
7
Time (h)

FIG. 14

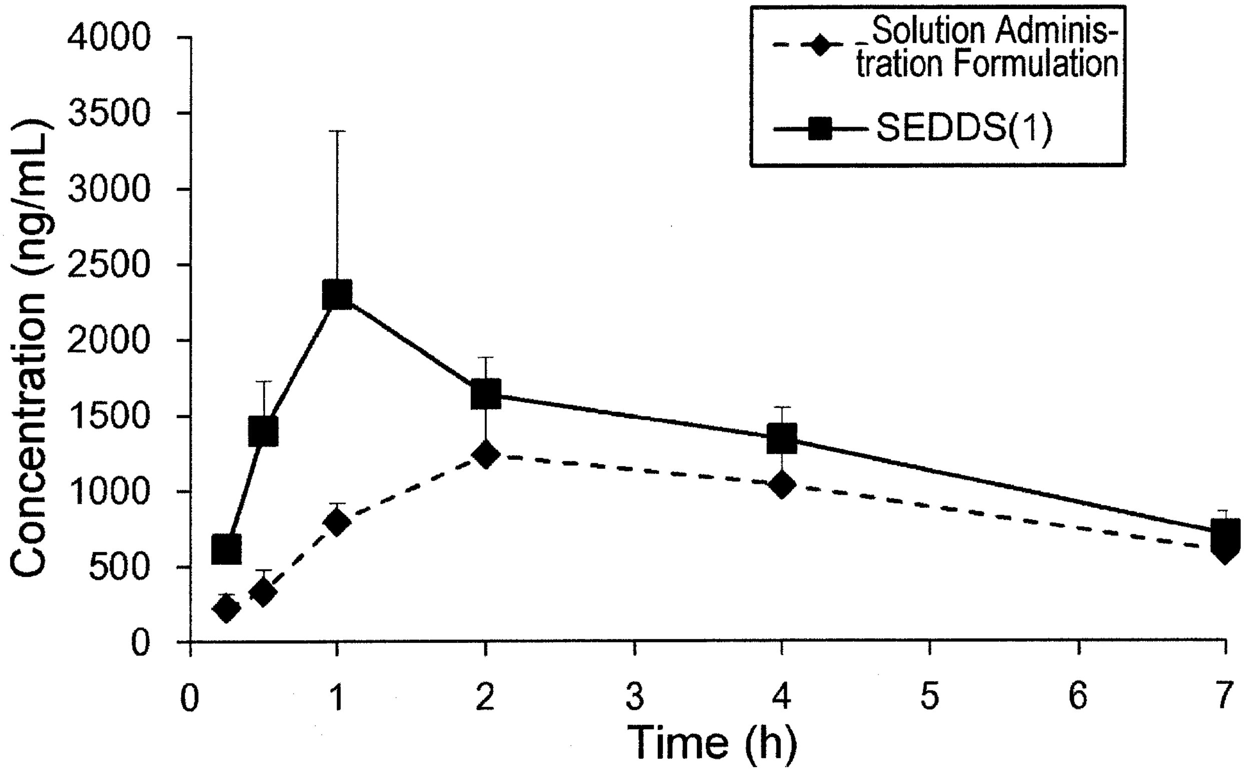
Solution Administration Formulation
SEDDS(1)
Concentration (ng/mL)
4000
3500
3000
2500
2000
1500
1000
500
0
0
1
2
3
4
5
6
7
Time (h)

FIG. 15

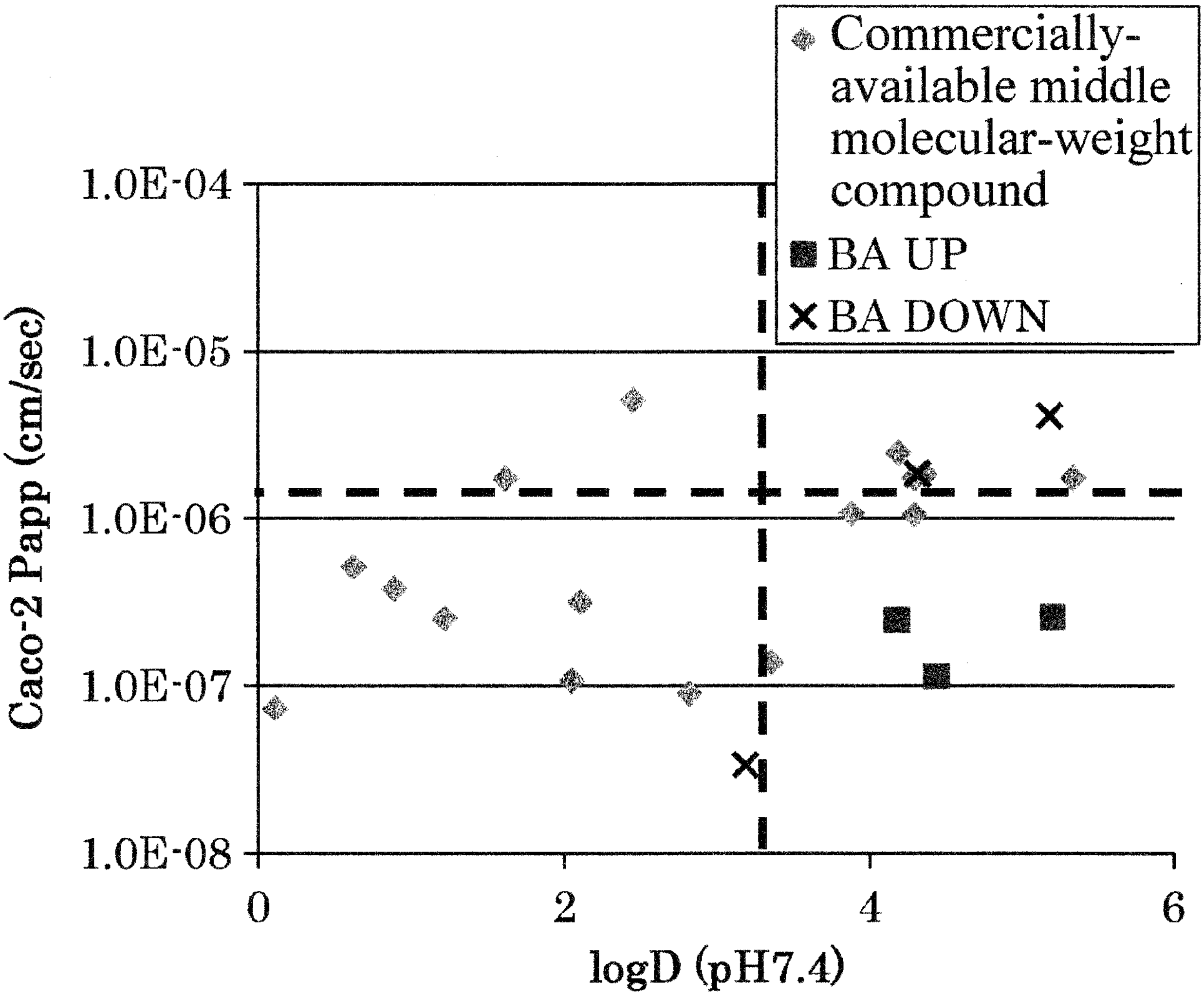
Commercially-available middle molecular-weight compound
BA UP
BA DOWN
1.0E-04
1.0E-05
1.0E-06
1.0E-07
1.0E-08
Caco-2 Papp (cm/sec)
0
2
4
6
logD (pH7.4)

FIG. 16

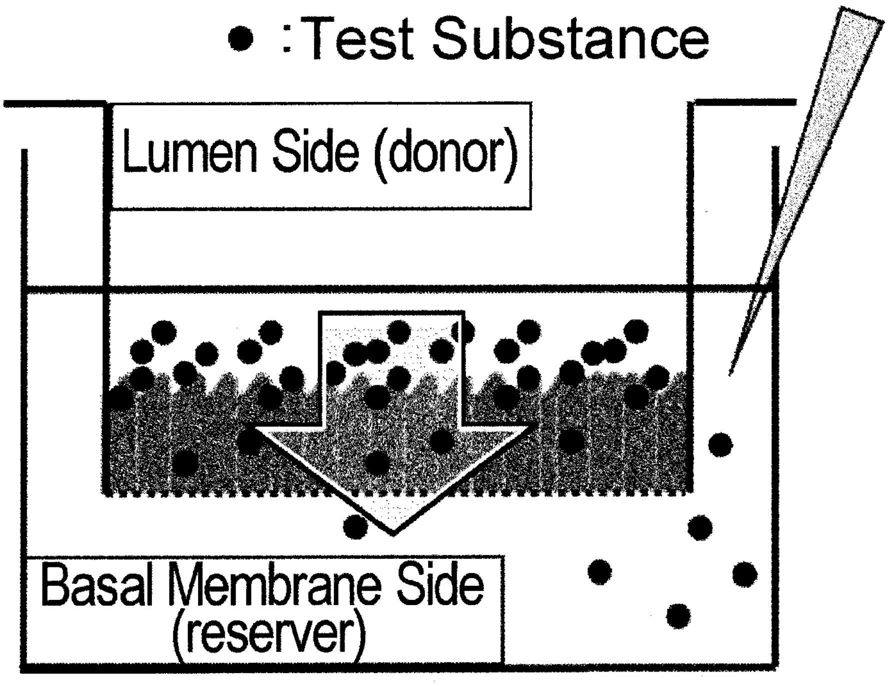
● : Test Substance
Lumen Side (donor)
Basal Membrane Side (reserver)

FIG. 17

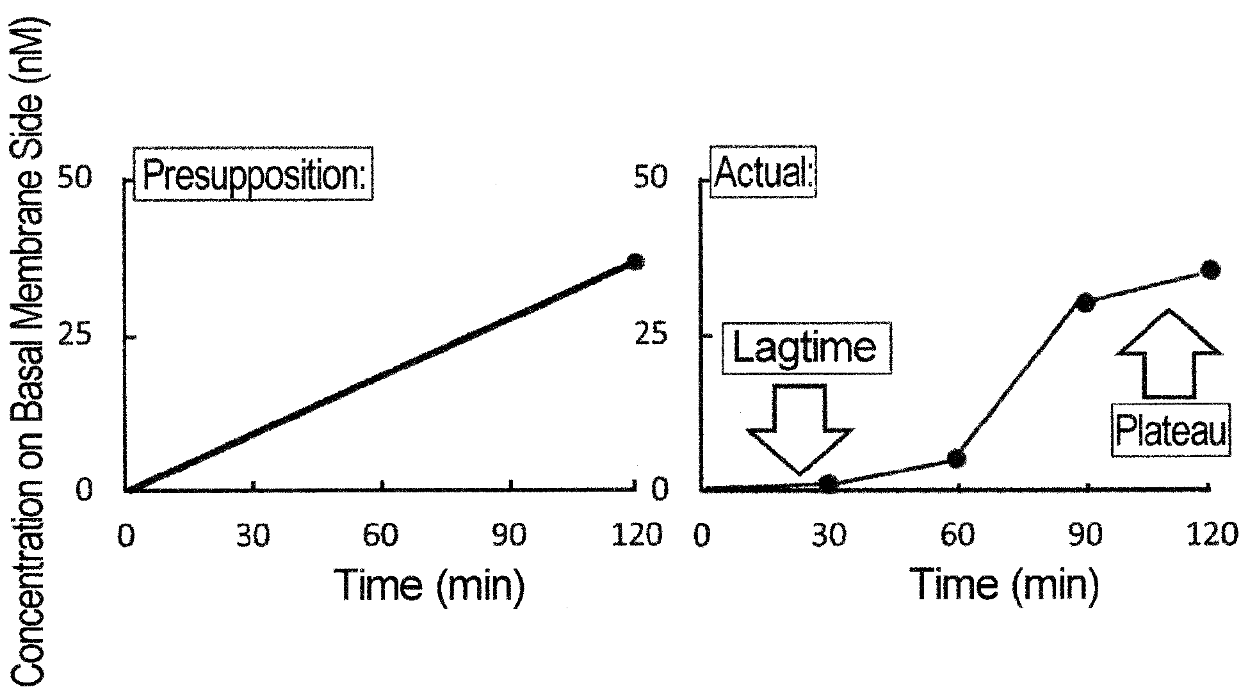
Concentration on Basal Membrane Side (nM)
Presupposition:
50
25
0
0
30
60
90
120
Time (min)
Actual:
50
25
0
Lagtime
Plateau
0
30
60
90
120
Time (min)

FIG. 18

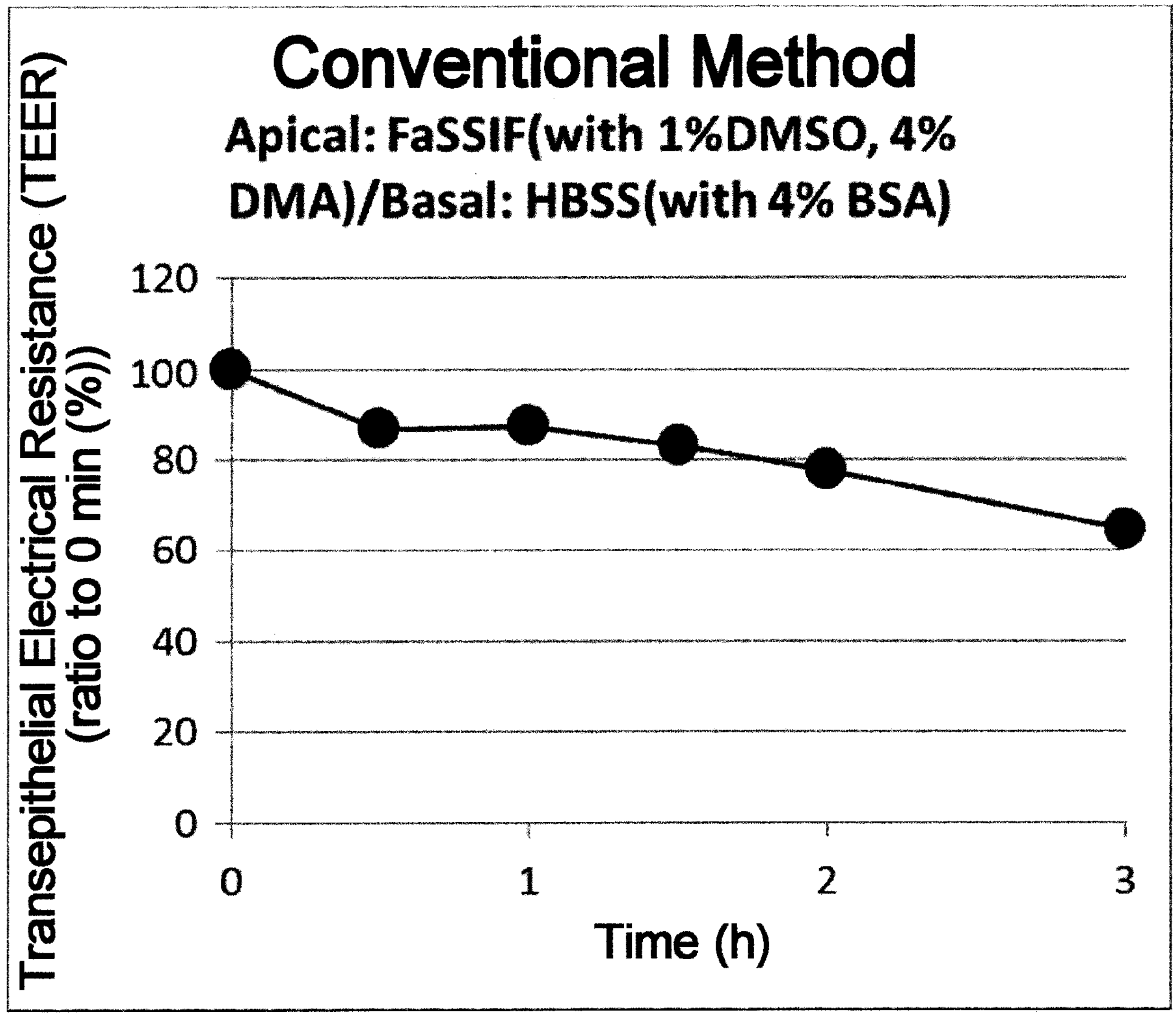
Conventional Method
Apical: FaSSIF(with 1%DMSO, 4% DMA)/Basal: HBSS(with 4% BSA)
Transepithelial Electrical Resistance (TEER) (ratio to 0 min (%))
120
100
80
60
40
20
0
0
1
2
3
Time (h)

FIG. 19

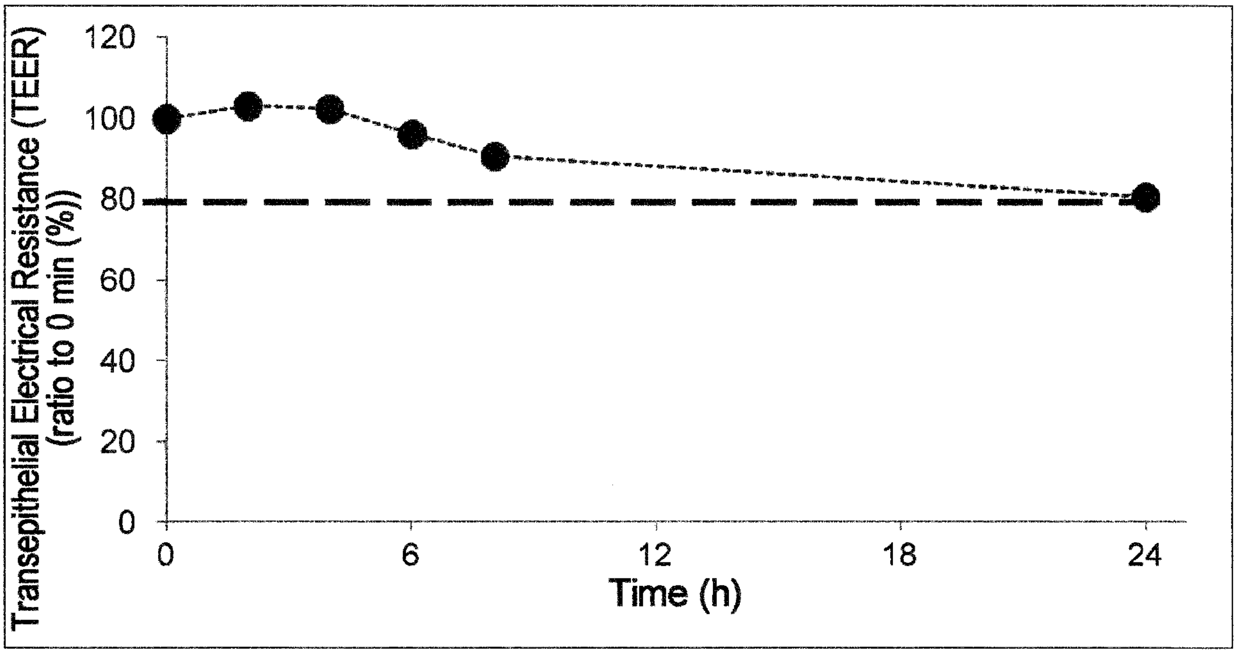
Transepithelial Electrical Resistance (TEER)
(ratio to 0 min (%))
120
100
80
60
40
20
0
0
6
12
18
24
Time (h)

FIG. 20

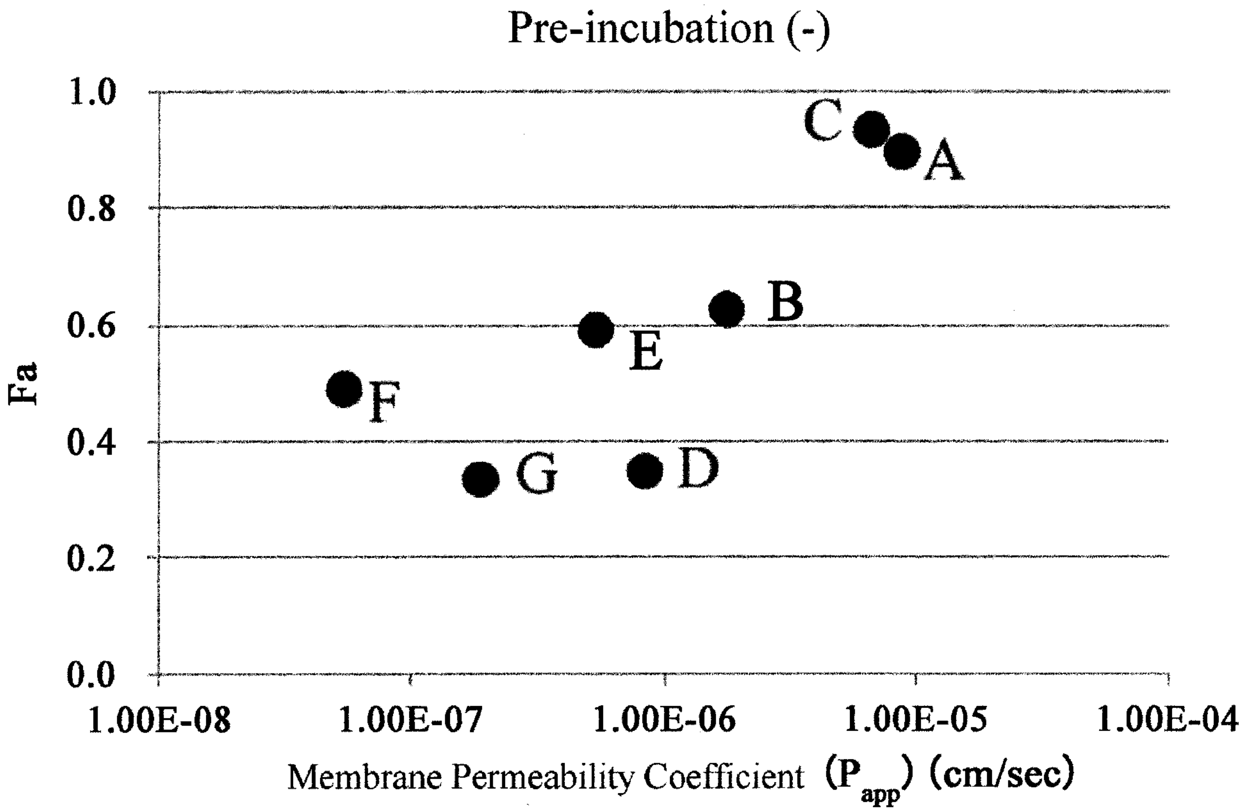
Pre-incubation (-)
1.0
0.8
0.6
0.4
0.2
0.0
Fa
C
A
B
E
F
G
D
1.00E-08
1.00E-07
1.00E-06
1.00E-05
1.00E-04
Membrane Permeability Coefficient (Papp) (cm/sec)

FIG. 21

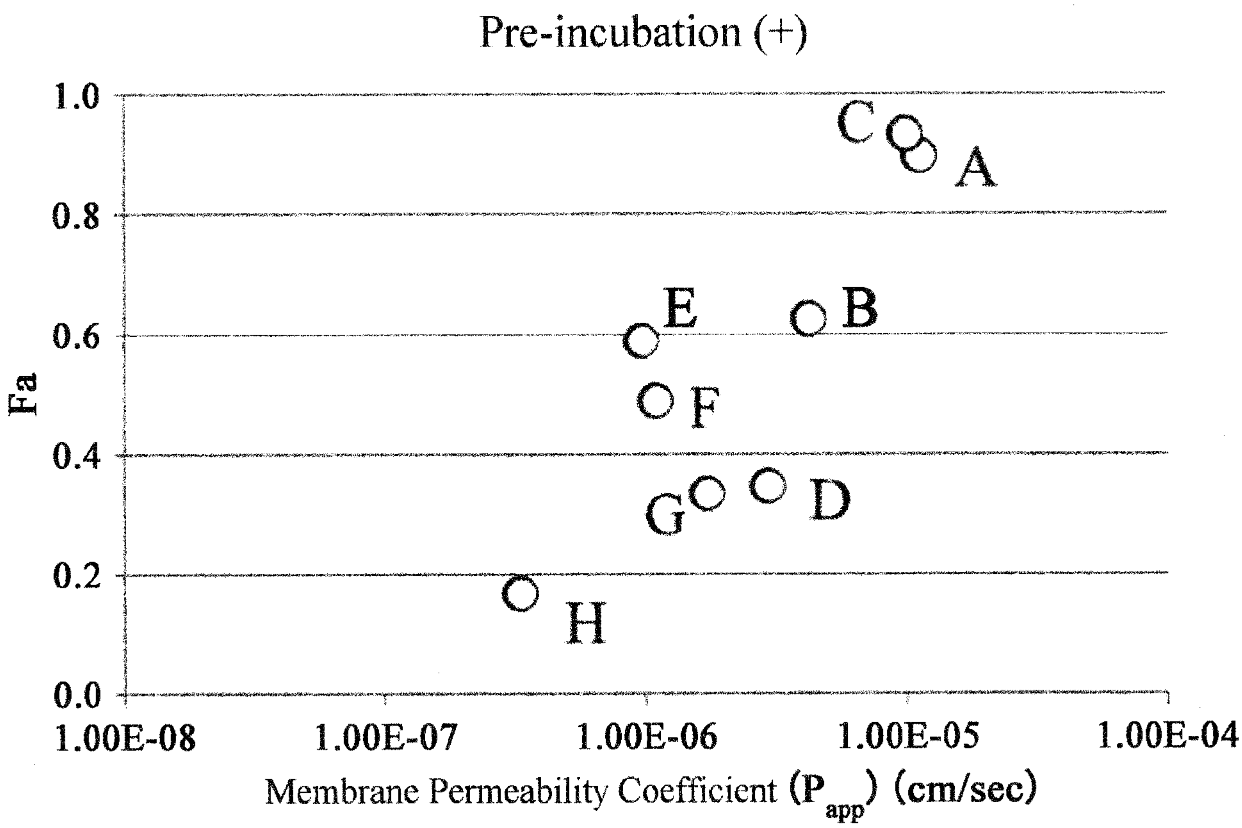
Pre-incubation (+)
1.0
0.8
0.6
0.4
0.2
0.0
Fa
C
A
E
B
F
G
D
H
1.00E-08
1.00E-07
1.00E-06
1.00E-05
1.00E-04
Membrane Permeability Coefficient (Papp) (cm/sec)

FIG. 22

Europäisches Patentamt
European Patent Office
Office européen des brevets

# EUROPEAN SEARCH REPORT

Application Number
EP 21 20 3289

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WESSLING S T ET AL: "Caco-2 cell permeability and stability of two d-glucopyranuronamide conjugates of thyrotropin-releasing hormone", BIOORGANIC, ELSEVIER, AMSTERDAM, NL, vol. 15, no. 14, 15 July 2007 (2007-07-15), pages 4946-4950, XP025803997, ISSN: 0968-0896, DOI: 10.1016/J.BMC.2007.04.042 [retrieved on 2007-07-15] * the whole document * | 1-15 | INV. A61K9/107 A61K47/10 A61K47/14 A61K47/44 |
| X | ANO RIEKO ET AL: "Relationships between structure and high-throughput screening permeability of peptide derivatives and related compounds with artificial membranes: application to prediction of Caco-2 cell permeability", BIOORGANIC, vol. 12, no. 1, 1 January 2004 (2004-01-01), pages 257-264, XP085628556, ISSN: 0968-0896, DOI: 10.1016/J.BMC.2003.10.002 * the whole document * | 1-15 | TECHNICAL FIELDS SEARCHED (IPC) A61K C07K |
| X | WANG CONAN K ET AL: "Exploring experimental and computational markers of cyclic peptides: Charting islands of permeability", EUROPEAN JOURNAL OF MEDICINAL CHEMISTRY, ELSEVIER, AMSTERDAM, NL, vol. 97, 25 April 2015 (2015-04-25), pages 202-213, XP029171149, ISSN: 0223-5234, DOI: 10.1016/J.EJMECH.2015.04.049 * 2.4; page 204 * | 1-15 | |

-/--

The present search report has been drawn up for all claims

1

| Place of search | Date of completion of the search | Examiner |
|---|---|---|
| Munich | 1 July 2022 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 20 3289

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NIELSEN HANNE MRCK ET AL: "Impact of Lipid-Based Drug Delivery Systems on the Transport and Uptake of Insulin Across Caco-2 Cell Monolayers", JOURNAL OF PHARMACEUTICAL SCIENCES, AMERICAN CHEMICAL SOCIETY AND AMERICAN PHARMACEUTICAL ASSOCIATION, US, vol. 105, no. 9, 1 September 2016 (2016-09-01), pages 2743-2751, XP009512699, ISSN: 0022-3549, DOI: 10.1016/J.XPHS.2016.01.006 * the whole document * ----- | 1-15 | |
| X | SARAH MAUD FISCHER ET AL: "Effect of the non-ionic surfactant Poloxamer 188 on passive permeability of poorly soluble drugs across Caco-2 cell monolayers", EUROPEAN JOURNAL OF PHARMACEUTICS AND BIOPHARMACEUTICS, ELSEVIER SCIENCE PUBLISHERS B.V., AMSTERDAM, NL, vol. 79, no. 2, 19 April 2011 (2011-04-19), pages 416-422, XP028391561, ISSN: 0939-6411, DOI: 10.1016/J.EJPB.2011.04.010 [retrieved on 2011-04-28] * the whole document * ----- | 1-9, 11-13 | TECHNICAL FIELDS SEARCHED (IPC) |
| T | DOUGHERTY PATRICK G. ET AL: "Understanding Cell Penetration of Cyclic Peptides", CHEMICAL REVIEWS, vol. 119, no. 17, 11 September 2019 (2019-09-11), pages 10241-10287, XP055881368, US ISSN: 0009-2665, DOI: 10.1021/acs.chemrev.9b00008 * the whole document * ----- | | |

The present search report has been drawn up for all claims

1

| Place of search | Date of completion of the search | Examiner |
|---|---|---|
| Munich | 1 July 2022 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2006112541 A
- WO 2002102354 A
- WO 2012033478 A
- WO 2012026566 A
- WO 2012033154 A
- WO 2012074130 A
- WO 2015030014 A


### Non-patent literature cited in the description


- **DONOVAN, M.D. et al.** Absorption of polyethylene glycols 600 through 2000: The molecular weight dependence of gastrointestinal and nasal absorption. *Pharm. Res.,* 1990, vol. 7, 863-868
- **CA LIPINSKI.** *Adv. Drug Del. Rev.,* 1997, vol. 23, 3
- **SATYANARAYANAJOIS, S. D. ; HILL, R. A.** Medicinal chemistry for 2020. *Future Med. Chem.,* 2011, vol. 3, 1765
- **GANESAN, A.** The impact of natural products upon modern drug discovery. *Curr. Opin. Chem. Bio.,* 2008, vol. 12, 306
- **GRACIA, S. R. ; GAUS, K. ; SEWALD, N.** Synthesis of chemically modified bioactive peptides: recent advances, challenges and developments for medicinal chemistry. *Future Med. Chem.,* 2009, vol. 1, 1289
- **R. S. LOKEY et al.** *Nat. Chem. Biol.,* 2011, vol. 7 (11), 810-817
- **J. W. SZOSTAK et al.** *J. Am. Chem. Soc.,* 2008, vol. 130, 6131-6136
- **T. KAWAKAMI et al.** *Chemistry & Biology,* 2008, vol. 15, 32-42
- **H. KESSLER et al.** *J. Am. Chem. Soc.,* 2012, vol. 134, 12125-12133
- *Drug Metab Dispos,* May 2006, vol. 34 (5), 729-33
- *Pharm Res,* June 2009, vol. 26 (6), 1486-95
- *Journal of Pharmaceutical Sciences,* 2000, vol. 89, 1073-1084
- **H. FISCHER et al.** Permeation of permanently positive charged molecules through artificial membranes-influence of physic-chemical properties. *Eur J. Pharm. Sci.,* 2007, vol. 31, 32-42
- **MOLTKE et al.** Midazolam hydroxylation by human liver microsomes in vitro: inhibition by fluoxetine, norfluoxetine, and by azole antifungal agents. *J Clin Pharmacol,* 1996, vol. 36 (9), 783-791
- **M. KATO et al.** The intestinal first-pass metabolism of substances of CYP3A4 and P-glycoprotein-quantitative analysis based on information from the literature. *Drug Metab. Pharmacokinet.,* 2003, vol. 18 (6), 365-372
- *Comb Chem High Throughput Screen.,* 2010, vol. 13, 75-87
- *Nature Chem. Bio.,* 2009, vol. 5, 502
- *Nat Chem Biol.,* 2009, vol. 5, 888-90
- *Bioconjugate Chem.,* 2007, vol. 18, 469-476
- *ChemBioChem,* 2009, vol. 10, 787-798
- *Chemical Communications (Cambridge, United Kingdom),* 2011, vol. 47 (36), 9946-9958
- **GURSOY, N.R. et al.** *Biomedicine & pharmacotherapy,* 2004, vol. 58, 173-182
- Japanese Pharmacopoeia
- Japanese Pharmaceutical Codex. 1997
- *Japanese Pharmaceutical Excipients,* 1998
- Japanese Specifications and Standards for Food Additives
- Japanese Standards of Cosmetic Ingredients
- *Japanese Cosmetic Ingredients Codex*
- *Japanese Standards of Quasi-drug Ingredients*
- *United States Pharmacopeia,* vol. 24
- *British Pharmacopeia,* 2000
- *European Pharmacopeia,* 2000
- *National Formulary,* vol. 19
- *European Journal of Pharmaceutical Sciences,* 2005, vol. 24 (4), 381-388
- **ARTURSSON, P.** *Adv Drug Deliv Rev,* 2001, vol. 46, 27-43
- **MASON, A.K.** *Drug Metab Dispos,* 2013, vol. 41, 1347-1366
- **POLLI, J.W.** *J Pharmacol Exp Ther,* 2001, vol. 299, 620-628
- **SUN, H.** *Expert Opin Drug Metab Toxicol,* 2008, vol. 4, 395-411
- **BHOOPATHY, S. et al.** *Methods Mol Biol,* 2014, vol. 1113, 229-252

- **KNIPP, G.T. et al.** *J Pharm Sci,* 1997, vol. 86, 1105-1110
- **KORZEKWA, K.R. et al.** *Drug Metab Dispos,* 2012, vol. 40, 865-876
- **SUN, H. et al.** *Drug Metab Dispos,* 2008, vol. 36, 102-123
- **OZEKI K. et al.** *Int J Pharm,* 2015, vol. 495, 963-971
- **P. ARTURSSONAND J. et al.** *Biochem Biophys Res Commun,* 1991, vol. 175, 880-885
- *Pharm Res,* 2010, vol. 27, 878-893
- **QINGQING X. et al.** *Xenobiotica,* 2016, vol. 46, 913-921